# EUROPEAN PATENT APPLICATION

(11) **EP 4 450 489 A1**
(43) Date of publication of application: **23.10.2024**
(21) Application number: 23168357.4
(22) Date of filing: 18.04.2023
(51) Int. Cl.: C07D 261/20, A61P 35/00, A61K 47/64

(54) **STABLE 4-ISOXAZOLINE CONJUGATES**

(71) Applicant: Synaffix B.V., 5349 AB Oss (NL)
(72) Inventor: The designation of the inventor has not yet been filed
(74) Representative: Nederlandsch Octrooibureau

(57) **Abstract**

The invention concerns nitrones that are capable of forming 4-isoxazoline compounds by strain-promoted alkyne-nitrone cycloaddition (SPANC), which have improved stability. The 4-isoxazoline compounds do not disintegrate by rearrangement into two molecules. As such, the nitrones according to the present invention are suitable for preparing bioconjugates by SPANC reaction. The invention further concerns the thus obtained bioconjugates, as well as the application thereof in targeting cells and treating cancer.

## Description

### Field of the invention

The present invention is in the field of organic chemistry. More specifically, the present invention relates to formation of isoxazoline compounds by strain-promoted alkyne-nitrone cycloaddition using improved nitrones. This reaction is especially suitable as conjugation reaction in the preparation of antibody-conjugates. Such antibody-conjugates can be applied for the more treatment of diseases such as cancer.

### Background

Antibody-drug conjugates (ADC), considered as one of the major classes of targeted therapy, are comprised of an antibody to which is attached a pharmaceutical agent. The antibodies (also known as binding agents or ligands) can be small protein formats (scFv's, Fab fragments, DARPins, Affibodies, etc.) but are generally monoclonal antibodies (mAbs) of IgG type which have been selected based on their high selectivity and affinity for a given antigen, their long circulating half-lives, and little to no immunogenicity. Thus, mAbs as ligands for a carefully selected biological receptor provide an ideal targeting platform for selective delivery of pharmaceutical drugs. For example, a monoclonal antibody known to bind selectively with a specific cancer-associated antigen can be used for delivery of a chemically conjugated cytotoxic agent to the tumour, via binding, internalization, intracellular processing and finally release of active catabolite. The cytotoxic agent may be small molecule toxin, a protein toxin or other formats, like oligonucleotides. As a result, the tumour cells can be selectively eradicated, while sparing normal cells which have not been targeted by the antibody. Similarly, chemical conjugation of an antibacterial drug (antibiotic) to an antibody can be applied for treatment of bacterial infections, while conjugates of anti-inflammatory drugs are under investigation for the treatment of autoimmune diseases and for example attachment of an oligonucleotide to an antibody is a potential promising approach for the treatment of neuromuscular diseases. Hence, the concept of targeted delivery of an active pharmaceutical drug to a specific cellular location of choice is a powerful approach for the treatment of a wide range of diseases, with many beneficial aspects versus systemic delivery of the same drug.

Currently, cytotoxic payloads include for example microtubule-disrupting agents [e.g. auristatins such as monomethyl auristatin E (MMAE) and monomethyl auristatin F (MMAF), maytansinoids such as DM1 and DM4, tubulysins], DNA-damaging agents [e.g., calicheamicin, pyrrolobenzodiazepine (PBD) dimers, indolinobenzodiapine dimers, duocarmycins, anthracyclines such as PNU-159,682, topoisomerase inhibitors [e.g. DXd, exatecan, SN-38] or RNA polymerase II inhibitors [e.g. amanitin]. ADCs that have reached market approval include for example payloads MMAE, MMAF, DM1, calicheamicin, SN-38, DXd and a PBD dimer, while a BLA has been filed for an ADC based on DM4 and a pivotal trials is running for an ADC based on duocarmycin. A larger variety of payloads is still under clinical evaluation or has been in clinical trials in the past, e.g. eribulin, indolinobenzodiazepine dimer, PNU-159,682, amanitin, hemi-asterlin, doxorubicin, vinca alkaloids and others. Finally, various ADCs in early clinical or late-stage preclinical stage are conjugated to novel payloads for example, KSP inhibitors, MMAD, cryptophycins, and others.

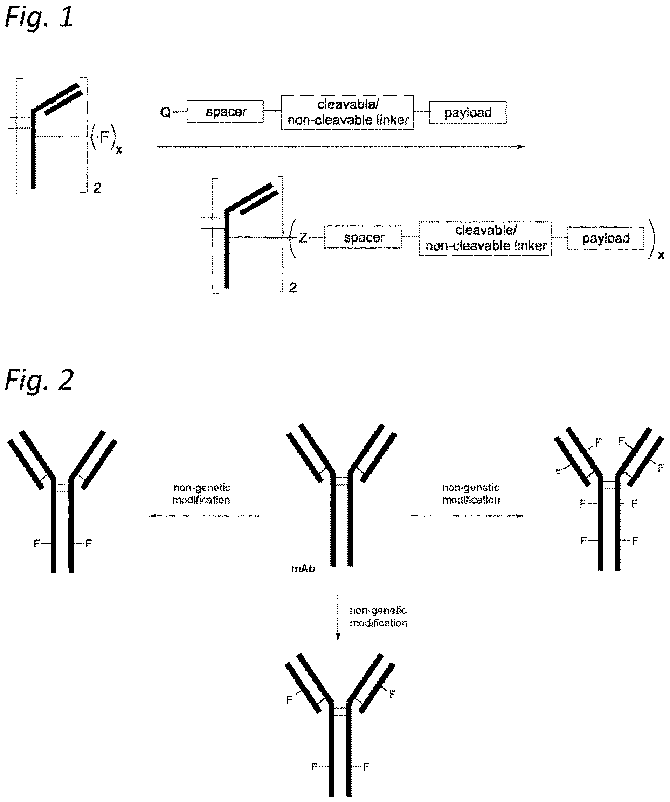

ADCs are prepared by conjugation of a linker-drug to a protein, a process known as bioconjugation. Many technologies are known for bioconjugation, as summarized in G.T. Hermanson, "Bioconjugate Techniques", Elsevier, 3rd Ed. 2013**,** incorporated by reference. Conceptually, the method the preparation of an ADC by bioconjugation entails the reaction of x number of reactive moieties F present on the antibody with a complementary reactive moiety Q present on the pharmaceutical drug (the payload), see Figure 1.

Typically, a chemical linker is present between Q and the payload. This linker needs to possess a number of key attributes, including the requirement to be stable in plasma after drug administration for an extended period of time. A stable linker enables localization of the ADC to the projected site or cells in the body and prevents premature release of the payload in circulation, which would indiscriminately induce undesired biological response of all kinds, thereby lowering the therapeutic index of the ADC. Upon internalization, the ADC should be processed such that the payload is effectively released so it can exert its mode-of-action inside the cell. The linker can also contain a spacer element. There are two families of linkers, non-cleavable and cleavable. Non-cleavable linkers consist of a chain of atoms between the antibody and the payload, which is fully stable under physiological conditions, irrespective of which organ or biological compartment the antibody-drug conjugate resides in. As a consequence, liberation of the payload from an ADC with a non-cleavable linker relies on the complete (lysosomal) degradation of the antibody after internalization of the ADC into a cell. As a result of this degradation, the payload will be released, still carrying the linker, as well as a peptide fragment and/or the amino acid from the antibody the linker was originally attached to. Cleavable linkers utilize an inherent property of a cell or a cellular compartment for selective release of the payload from the ADC, which generally leaves no trace of linker after processing. For cleavable linkers, there are three commonly used mechanisms: (1) susceptibility to specific enzymes, (2) pH-sensitivity, and (3) sensitivity to redox state of a cell (or its microenvironment). The cleavable linker may also contain a self-immolative unit, for example based on a para-aminobenzyl alcohol group and derivatives thereof. A linker may also contain an additional element, often referred to as spacer or stretcher unit, to connect the linker with a reactive group for attachment to the antibody via a reactive moiety F present on the antibody.

The reactive moiety F can be naturally present in the antibody, for example the reactive moiety can be the side chain of lysine or cysteine, which can be employed for acylation (lysine side chain) or alkylation (cysteine side chain). Acylation of the e-amino group in a lysine side-chain is typically achieved by subjecting the protein to a reagent based on an activated ester or activated carbonate derivative, for example SMCC is applied for the manufacturing of Kadcyla^{®}. Besides conjugation to the side chains of the naturally present amino acids lysine or cysteine, a range of other conjugation technologies has been explored based on a two-stage strategy involving (a) introduction on a novel reactive group F, followed by (b) reaction with another complementary reactive group Q. For example, a method can be used to introduce a given number of reactive moieties F onto an antibody, which can be two, four or eight (Figure 2).

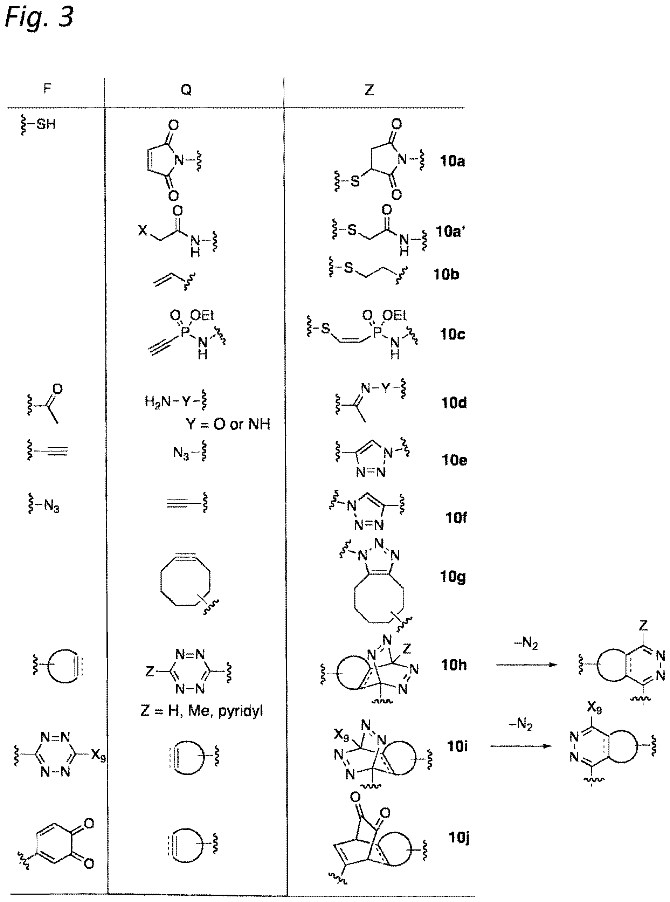

An example of an unnatural reactive functionality F that can be employed for bioconjugation of linker-drugs is the oxime group, suitable for oxime ligation or the azido group, suitable for click chemistry conjugation. The oxime can be installed in the antibody by genetic encoding of a non-natural amino acid, e.g. *p*-acetophenylalanine, as for example demonstrated by Axup et al. Proc. Nat. Acad. Sci. 2012, 109, 16101-16106, incorporated by reference, or by enzymatic alkylation of a cysteine present in a CAAX sequence with a prenyl group containing a remote keto group, as for example disclosed in WO2012153193. The azide can be installed in the antibody by genetic encoding of *p*-azidomethylphenylalanine or *p*-azidophenylalanine, as for example demonstrated by Axup et al. Proc. Nat. Acad. Sci. 2012, 109, 16101-16106, incorporated by reference. Similarly, Zimmerman et al., Bioconj. Chem. 2014, 25, 351-361, incorporated by reference have employed a cell-free protein synthesis method to introduce *p*-azidomethylphenylalanine (AzPhe) into monoclonal antibodies for conversion into ADCs by means of metal-free click chemistry. Also, it has also be shown by Nairn et al., Bioconj. Chem. 2012, 23, 2087-2097, incorporated by reference, that a methionine analogue like azidohomoalanine (Aha) can be introduced into protein by means of auxotrophic bacteria and further converted into protein conjugates by means of click chemistry. Finally, genetic encoding of aliphatic azides in recombinant proteins using a pyrrolysyl-tRNA synthetase/tRNA_{CUA} pair was shown by Nguyen et al., J. Am. Chem. Soc. 2009, 131, 8720-8721, incorporated by reference, and labelling was achieved by click chemistry, either by copper-catalyzed alkyne-azide cycloaddition (CuAAC) or strain-promoted alkyne-azide cycloaddition (SPAAC). Besides, CuAAC and SPAAC, bioconjugation of linker-drugs to antibodies (and other biomolecules such as glycans, nucleic acids) can be achieved by a range of other metal-free click chemistries, see e.g. Nguyen and Prescher, Nature Rev. Chem. 2020, 4, 476-489, incorporated by reference. For example, nitrones are capable of reacting with strained alkynes in a strain-promoted alkyne-nitrone cycloaddition (SPANC) reaction, giving an isoxazoline connecting group. Further, oxidation of a specific tyrosine in a protein can give an ortho-quinone, which readily undergoes cycloaddition with strained alkenes (e.g. TCO) or strained alkynes, see e.g. Bruins et al., Chem. Eur. J. 2017, 24, 4749-4756, incorporated by reference. Besides cyclooctyne, certain cycloheptynes are also suitable for metal-free click chemistry, as reported by Wetering et al. Chem. Sci. 2020, 11, 9011-9016, incorporated by reference. A tetrazine moiety can also be introduced into a protein or a glycan by various means, for example by genetic encoding or chemical acylation, and may also undergo cycloaddition with cyclic alkenes and alkynes. A list of pairs of functional groups F and Q for metal-free click chemistry is provided in Figure 3.

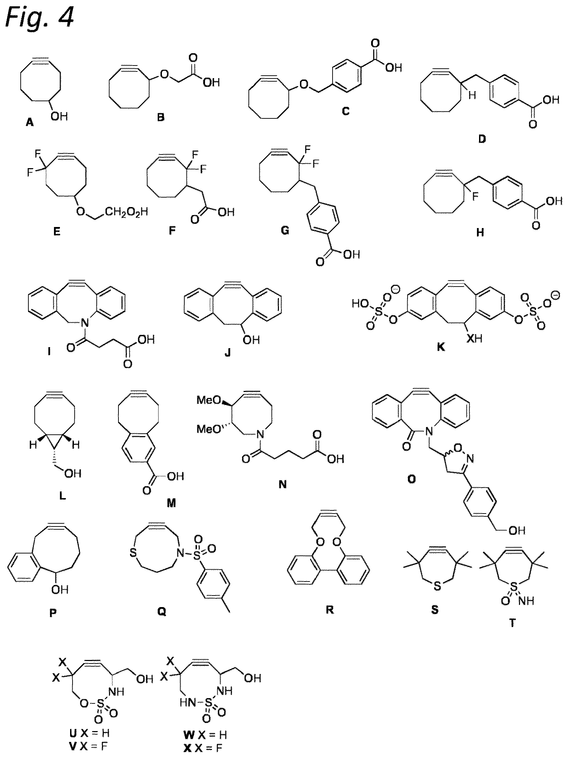

In SPAAC and SPANC bioconjugation, the linker-drug is functionalized with a cyclic alkyne and the cycloaddition with the nitrone- or azido-modified antibody is driven by relief of ring-strain. Conversely, the linker-drug can be functionalized with an azide or nitrone moiety and the antibody with cyclic alkyne. Various strained alkynes suitable for metal-free click chemistry are indicated in Figure 4.

A method of increasing popularity in the field of ADCs is based on enzymatic installation of a non-natural functionality F. For example, Lhospice etal., Mol. Pharmaceut. 2015, 12, 1863-1871, incorporated by reference, employ the bacterial enzyme transglutaminase (BTG or TGase) for installation of an azide moiety onto an antibody. A genetic method based on C-terminal TGase-mediated azide introduction followed by conversion in ADC with metal-free click chemistry was reported by Cheng et al., Mol. Cancer Therap. 2018, 17, 2665-2675, incorporated by reference. It has been shown in WO2014065661, by van Geel et al., Bioconj. Chem. 2015, 26, 2233-2242, Verkade et al., Antibodies 2018, 7, 12, and Wijdeven at al. MAbs 2022, 14, 2078466, all incorporated by reference, that enzymatic remodelling of the native antibody glycan at N297 enables introduction of an azido-modified sugar, suitable for attachment of cytotoxic payload using metal-free click chemistry. Similarly, the enzymatic glycan remodelling protocol can also be employed to install a free thiol group on an antibody for conjugation based on any of the methods described above for cysteine conjugation.

SPANC has emerged as a viable alternative to SPAAC. The reaction between a nitrone and a stained alkyne gives a 4-isoxazoline ring (see e.g. McKay et al., Org. Biomol. Chem. 2012, 10, 3066-3070, incorporated by reference), which provides a covalent connection between the substituent on the nitrone (e.g. a cytotoxic payload) and on the strained alkyne (e.g. an antibody). A drawback that hampers widespread application of SPANC for the preparation of ADCs is the instability of the 4-isoxazoline ring, which may break-down by rearrangement (see MacKenzie et al., Curr. Opin. Chem. Biol. 2014, 21, 81-88 (in particular Fig 2b), incorporated by reference). The present invention provides a solution for the instability of the isoxazoline products of the SPANC reaction.

### Summary of the invention

The inventors have developed nitrones that are capable of 4-isoxazoline compounds by strain-promoted alkyne-nitrone cycloaddition (SPANC), where the 4-isoxazoline compounds do not disintegrate by rearrangement into two molecules, but any rearrangement that may occur leaves the molecular structure intact to such an extent that all substituents of the 4-isoxazoline remain covalently connected.

The nitrone according to the invention has structure (1): 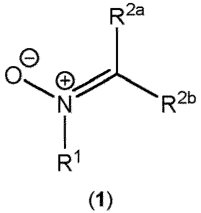 wherein
- R¹ is L¹⁰XR⁴, wherein:
   - L¹⁰ is a linker of structure (C(R³)₂)_{z}, wherein z is 2 or 3, and each R³ is individually selected from H and C₁₋₄ alkyl, or two occurrences of R³ may be joined together to form an oxo group, a C₃₋₆ (hetero)cycloalkyl group;
   - R⁴ is selected from H and C₁₋₄ alkyl;
   - XisS, OorNH,
- R^{2a} is selected from H and C₁₋₆ (cyclo)alkyl;
- R^{2b} is selected from the group consisting of C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups, or wherein R^{2b} is L(D)ᵣ, wherein D is a payload, r is an integer in the range of 1 - 10, and L is a linker covalently connecting D with the nitrone group, or R^{2b} is L⁶B, wherein B is a biomolecule and L⁶ is a linker covalently connecting B with the nitrone group;
- wherein when R^{2a} and R^{2b} are different, the configuration of the C=N double bond in the nitrone group may be *E* or *Z*.

Compounds, especially bioconjugates, that are prepared via SPANC with the nitrones according to the invention have improved stability, especially regarding reduced non-induced release of a payload from the bioconjugate. This leads to an improved shelf-life of the bioconjugate according to the invention. Furthermore, when the bioconjugate according to the invention is used in treatment, the improved stability leads to an improved safety (higher tolerability), since less payloads (typically cytotoxins) are released at locations where the payload should not be released and causes an adverse reaction, and an improved efficacy, since more payloads are released at the location where the payload should effectuate a beneficial medical effect. This in turn leads to an improved therapeutic index for the conjugates according to the invention.

### Detailed description

### Definitions

The verb "to comprise", and its conjugations, as used in this description and in the claims is used in its non-limiting sense to mean that items following the word are included, but items not specifically mentioned are not excluded. In addition, reference to an element by the indefinite article "a" or "an" does not exclude the possibility that more than one of the elements is present, unless the context clearly requires that there is one and only one of the elements. The indefinite article "a" or "an" thus usually means "at least one".

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer.

The compounds disclosed in this description and in the claims may further exist as exo and *endo* diastereoisomers. Unless stated otherwise, the description of any compound in the description and in the claims is meant to include both the individual exo and the individual *endo* diastereoisomers of a compound, as well as mixtures thereof. When the structure of a compound is depicted as a specific *endo* or exo diastereomer, it is to be understood that the invention of the present application is not limited to that specific *endo* or exo diastereomer.

Furthermore, the compounds disclosed in this description and in the claims may exist as *cis* and *trans* isomers. Unless stated otherwise, the description of any compound in the description and in the claims is meant to include both the individual *cis* and the individual *trans* isomer of a compound, as well as mixtures thereof. As an example, when the structure of a compound is depicted as a *cis* isomer, it is to be understood that the corresponding *trans* isomer or mixtures of the *cis* and *trans* isomer are not excluded from the invention of the present application. When the structure of a compound is depicted as a specific *cis* or *trans* isomer, it is to be understood that the invention of the present application is not limited to that specific *cis* or *trans* isomer.

The compounds according to the invention may exist in salt form, which are also covered by the present invention. The salt is typically a pharmaceutically acceptable salt, containing a pharmaceutically acceptable anion. The term "salt thereof' means a compound formed when an acidic proton, typically a proton of an acid, is replaced by a cation, such as a metal cation or an organic cation and the like. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt. In a preferred embodiment, the compounds according to the invention are not in salt form, except for where explicitly indicated in the structure.

The term "pharmaceutically accepted" salt means a salt that is acceptable for administration to a patient, such as a mammal (salts with counterions having acceptable mammalian safety for a given dosage regime). Such salts may be derived from pharmaceutically acceptable inorganic or organic bases and from pharmaceutically acceptable inorganic or organic acids. "Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

A linker is herein defined as a moiety that connects (covalently links) two or more elements of a compound. A linker may comprise one or more spacer moieties. A spacer-moiety is herein defined as a moiety that spaces (i.e. provides distance between) and covalently links together two (or more) parts of a linker. The linker may be part of e.g. a linker-construct, a linker-conjugate, a linker-payload (e.g. linker-drug) or an antibody-conjugate, as defined below.

A "hydrophilic group" or "polar linker" is herein defined as any molecular structure containing one or more polar functional groups that imparts improved polarity, and therefore improved aqueous solubility, to the molecule it is attached to. Preferred hydrophilic groups are selected from a carboxylic acid group, an alcohol group, an ether group, a polyethylene glycol group, an amino group, an ammonium group, a sulfonate group, a phosphate group, an acyl sulfamide group or a carbamoyl sulfamide group. In addition to higher solubility other effects of the hydrophilic group include improved click conjugation efficiency, and, once incorporated into an antibody-drug conjugate: less aggregation, improved pharmacokinetics resulting in higher efficacy and in vivo tolerability.

A "self-immolative group" is herein defined as a part of a linker in an antibody-drug conjugate with a function is to conditionally release free drug at the site targeted by the ligand unit. The activatable self-immolative moiety comprises an activatable group (AG) and a self-immolative spacer unit. Upon activation of the activatable group, for example by enzymatic conversion of an amide group to an amino group or by reduction of a disulfide to a free thiol group, a self-immolative reaction sequence is initiated that leads to release of free drug by one or more of various mechanisms, which may involve (temporary) 1,6-elimination of a *p*-aminobenzyl group to a *p-*quinone methide, optionally with release of carbon dioxide and/or followed by a second cyclization release mechanism. The self-immolative assembly unit can part of the chemical spacer connecting the antibody and the payload (via the functional group). Alternatively, the self-immolative group is not an inherent part of the chemical spacer, but branches off from the chemical spacer connecting the antibody and the payload.

The term "click probe" refers to a functional moiety that is capable of undergoing a click reaction, i.e. two compatible click probes mutually undergo a click reaction such that they are covalently linked in the product. Compatible probes for click reactions are known in the art, and preferably include (cyclic) alkynes and nitrones.

The term "(hetero)alkyl" refers to alkyl groups and heteroalkyl groups. Heteroalkyl groups are alkyl groups wherein one or more carbon units in the alkyl chain (e.g. CH₂, CH or C) are replaced by heteroatoms, such as O, S, S(O), S(O)₂ or NR⁴. In other words, the alkyl chain is interrupted with one ore more elements selected from O, S, S(O), S(O)₂ and NR⁴. Such interruptions are distinct from substituents, as they occur within the chain of an alkyl group, whereas substituents are pendant groups, monovalently attached to e.g. a carbon atom of an alkyl chain. In a preferred embodiment, the (hetero)alkyl group is an alkyl group, e.g. ethyl (Et), isopropyl (i-Pr), n-propyl (n-Pr), tert-butyl (t-Bu), isobutyl (i-Bu), n-butyl (n-Bu) or n-pentyl.

Likewise, the term "(hetero)aryl" refers to aryl groups and heteroaryl groups. Heteroaryl groups are aryl groups wherein one or more carbon units in the ring (e.g. CH) are replaced by heteroatoms, such as O, S, N or NR⁴.

A "bioconjugate" is herein defined as a compound wherein a biomolecule is covalently connected to a target molecule via a linker. A bioconjugate may also be referred to as "conjugate". A bioconjugate comprises one or more biomolecules B and one or more payloads D. The linker may comprise one or more spacer moieties.

A "biomolecule" is herein defined as any molecule that can be isolated from nature or any molecule composed of smaller molecular building blocks that are the constituents of macromolecular structures derived from nature, in particular nucleic acids, proteins, glycans and lipids. Examples of a biomolecule include an enzyme, a (non-catalytic) protein, a polypeptide, a peptide, an amino acid, an oligonucleotide, a monosaccharide, an oligosaccharide, a polysaccharide, a glycan, a lipid and a hormone.

A "domain" may be any region of a protein, generally defined on the basis of sequence homologies and often related to a specific structural or functional entity. The term domain is used in this document to designate either individual Ig-like domains, such as "N-domain" or for groups of consecutive domains, such as "A3-B3 domain".

The term "glycoprotein" is herein used in its normal scientific meaning and refers to a protein comprising one or more monosaccharide or oligosaccharide chains ("glycans") covalently bonded to the protein. A glycan may be attached to a hydroxyl group on the protein (O-linked-glycan), *e.g*. to the hydroxyl group of serine, threonine, tyrosine, hydroxylysine or hydroxyproline, or to an amide function on the protein (*N*-glycoprotein), *e.g*. asparagine or arginine, or to a carbon on the protein (*C*-glycoprotein), *e.g.* tryptophan. A glycoprotein may comprise more than one glycan, may comprise a combination of one or more monosaccharide and one or more oligosaccharide glycans, and may comprise a combination of N-linked, O-linked and C-linked glycans. It is estimated that more than 50% of all proteins have some form of glycosylation and therefore qualify as glycoprotein. Examples of glycoproteins include PSMA (prostate-specific membrane antigen), CAL (candida antartica lipase), gp41, gp120, EPO (erythropoietin), antifreeze protein and antibodies.

The term "glycan" is herein used in its normal scientific meaning and refers to a monosaccharide or oligosaccharide chain that is linked to a protein. The term glycan thus refers to the carbohydrate-part of a glycoprotein. The glycan is attached to a protein via the C-1 carbon of one sugar, which may be without further substitution (monosaccharide) or may be further substituted at one or more of its hydroxyl groups (oligosaccharide). A naturally occurring glycan typically comprises 1 to about 10 saccharide moieties. However, when a longer saccharide chain is linked to a protein, said saccharide chain is herein also considered a glycan. A glycan of a glycoprotein may be a monosaccharide. Typically, a monosaccharide glycan of a glycoprotein consists of a single N-acetylglucosamine (GIcNAc), glucose (Glc), mannose (Man) or fucose (Fuc) covalently attached to the protein. A glycan may also be an oligosaccharide. An oligosaccharide chain of a glycoprotein may be linear or branched. In an oligosaccharide, the sugar that is directly attached to the protein is called the core sugar. In an oligosaccharide, a sugar that is not directly attached to the protein and is attached to at least two other sugars is called an internal sugar. In an oligosaccharide, a sugar that is not directly attached to the protein but to a single other sugar, i.e. carrying no further sugar substituents at one or more of its other hydroxyl groups, is called the terminal sugar. For the avoidance of doubt, there may exist multiple terminal sugars in an oligosaccharide of a glycoprotein, but only one core sugar. A glycan may be an O-linked glycan, an N-linked glycan or a C-linked glycan. In an O-linked glycan a monosaccharide or oligosaccharide glycan is bonded to an O-atom in an amino acid of the protein, typically via a hydroxyl group of serine (Ser) or threonine (Thr). In an N-linked glycan a monosaccharide or oligosaccharide glycan is bonded to the protein via an N-atom in an amino acid of the protein, typically via an amide nitrogen in the side chain of asparagine (Asn) or arginine (Arg). In a C-linked glycan a monosaccharide or oligosaccharide glycan is bonded to a C-atom in an amino acid of the protein, typically to a C-atom of tryptophan (Trp).

The term "antibody" (AB) is herein used in its normal scientific meaning. An antibody is a protein generated by the immune system that is capable of recognizing and binding to a specific antigen. An antibody is an example of a glycoprotein. The term antibody herein is used in its broadest sense and specifically includes monoclonal antibodies, polyclonal antibodies, dimers, multimers, multispecific antibodies (e.g. bispecific antibodies), antibody fragments, and double and single chain antibodies. The term "antibody" is herein also meant to include human antibodies, humanized antibodies, chimeric antibodies and antibodies specifically binding cancer antigen. The term "antibody" is meant to include whole antibodies, but also fragments of an antibody, for example an antibody Fab fragment, F(ab')₂, Fv fragment or Fc fragment from a cleaved antibody, a scFv-Fc fragment, a minibody, a diabody or a scFv. Furthermore, the term includes genetically engineered antibodies and derivatives of an antibody. Antibodies, fragments of antibodies and genetically engineered antibodies may be obtained by methods that are known in the art. The antibody may be monoclonal, chimeric and/or humanized.

An antibody may be a natural or conventional antibody in which two heavy chains are linked to each other by disulfide bonds and each heavy chain is linked to a light chain by a disulfide bond. There are two types of light chain, lambda (I) and kappa (k). The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties, such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The immunoglobulin can be of any type (e.g. IgG, IgE, IgM, IgD, and IgA), class (e.g. IgG1 , IgG2, IgG3, IgG4, IgA1 and IgA2) or subclass, or allotype (e.g. human G1m1 , G1m2, G m3, non-G1m1 [that, is any allotype other than G1m1], G1m17, G2m23, G3m21 , G3m28, G3m1.1, G3m5, G3m13, G3m14, G3m10, G3m15, G3m16, G3m6, G3m24, G3m26, G3m27, A2m1, A2m2, Km1, Km2 and Km3) of immunoglobulin molecule. Preferred allotypes for administration include a non-G1m1 allotype (nG1m1), such as G1m17,1, G1m3, G1m3.1, G1m3.2 or G1m3.1.2. More preferably, the allotype is selected from the group consisting of the G1m17, 1 or G1m3 allotype. The antibody may be engineered in the Fc-domain to enhance or nihilate binding to Fc-gamma receptors, as summarized by Saunders et al. Front. Immunol. 2019, 10, doi: 10.3389/fimmu.2019.01296 and Ward et al., Mol. Immunol. 2015, 67, 131-141. For example, the combination of Leu234Ala and Leu235Ala (commonly called LALA mutations) eliminate FcyRlla binding. Elimination of binding to Fc-gamma receptors can also be achieved by mutation of the N297 amino acid to any other amino acid except asparagine, by mutation of the T299 amino acid to any other amino acid except threonine or serine, or by enzymatic Deglycosylation or trimming of the fully glycosylated antibody with for example PNGase F or an endoglycosidase. The immunoglobulins can be derived from any species, including human, murine, or rabbit origin. Each chain contains distinct sequence domains.

The term "monoclonal antibody" or "mAb" as used herein refers to an antibody molecule of a single amino acid sequence, which is directed against a specific antigen, and is not to be construed as requiring production of the antibody by any particular method. A monoclonal antibody may be produced by a single clone of B cells or hybridoma, but may also be recombinant, i.e. produced by protein engineering.

The term "chimeric antibody" refers to an engineered antibody which, in its broadest sense, contains one or more regions from one antibody and one or more regions from one or more other antibodies. In an embodiment, a chimeric antibody comprises a VH domain and a VL domain of an antibody derived from a non-human animal, in association with a CH domain and a CL domain of another antibody, in an embodiment, a human antibody. As the non-human animal, any animal such as mouse, rat, hamster, rabbit or the like can be used. A chimeric antibody may also denote a multispecific antibody having specificity for at least two different antigens.

The term "humanised antibody" refers to an antibody which is wholly or partially of non-human origin and which has been modified to replace certain amino acids, for instance in the framework regions of the VH and VL domains, in order to avoid or minimize an immune response in humans. The constant domains of a humanized antibody are most of the time human CH and CL domains. "Fragments" of (conventional) antibodies comprise a portion of an intact antibody, in particular the antigen binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of a conventional antibody may also be a single domain antibody, such as a heavy chain antibody or VHH.

The term "payload" refers to the moiety that is covalently attached to a biomolecule such as an antibody, but also to the molecule that is released from the conjugate upon cleavage of the linker. Payload thus refers to the monovalent moiety having one open end which is covalently attached to the targeting moiety via a linker, which is in the context of the present invention referred to as D, and also to the molecule that is released therefrom.

Herein, the term "therapeutic index" (TI) has the conventional meaning well known to a person skilled in the art, and refers to the ratio of the dose of drug that is toxic (i.e. causes adverse effects at an incidence or severity not compatible with the targeted indication) for 50% of the population (TD50) divided by the dose that leads to the desired pharmacological effect in 50% of the population (effective dose or ED50). Hence, TI = TD50 / ED50. The therapeutic index may be determined by clinical trials or for example by plasma exposure tests. See also Muller, et al. Nature Reviews Drug Discovery 2012, 11, 751-761. At an early development stage, the clinical TI of a drug candidate is often not yet known. However, understanding the preliminary TI of a drug candidate is of utmost importance as early as possible, since TI is an important indicator of the probability of the successful development of a drug. Recognizing drug candidates with potentially suboptimal TI at earliest possible stage helps to initiate mitigation or potentially re-deploy resources. At this early stage, TI is typically defined as the quantitative ratio between safety (maximum tolerated dose in mouse or rat) and efficacy (minimal effective dose in a mouse xenograft).

Herein, the term "therapeutic efficacy" denotes the capacity of a substance to achieve a certain therapeutic effect, e.g. reduction in tumour volume. Therapeutic effects can be measured determining the extent in which a substance can achieve the desired effect, typically in comparison with another substance under the same circumstances. A suitable measure for the therapeutic efficacy is the ED50 value, which may for example be determined during clinical trials or by plasma exposure tests. In case of preclinical therapeutic efficacy determination, the therapeutic effect of a bioconjugate (e.g. an ADC), can be validated by patient-derived tumour xenografts in mice in which case the efficacy refers to the ability of the ADC to provide a beneficial effect. Alternatively the tolerability of said ADC in a rodent safety study can also be a measure of the therapeutic effect.

Herein, the term "tolerability" refers to the maximum dose of a specific substance that does not cause adverse effects at an incidence or severity not compatible with the targeted indication. A suitable measure for the tolerability for a specific substance is the TD50 value, which may for example be determined during clinical trials or by plasma exposure tests.

### The invention

Nitrones are reactive towards strained alkynes according to the reaction in Scheme 1. In case the alkyne suffers from sufficient ring strain, induced by groups R*, it will react with the nitrone in a strain-promoted alkyne-nitrone cycloaddition (SPANC) reaction. As such, a 4-isoxazoline ring forms, wherein a covalent connection is formed between the group on the nitrone (R**) and the groups on the alkyne (R*). The SPANC reaction is well-known in the art, and is especially suitable in the synthesis of bioconjugates, wherein a covalent connection is formed between a biomolecule (e.g. via R** on the nitrone) and a payload molecule (e.g. via R* on the alkyne).

The a 4-isoxazoline ring may suffer from disintegration via the rearrangement depicted in Scheme 2. Isoxazoline ring (a) is rearranged into the aziridine ring (b), from which ring strain is released in zwitterionic species (c). Addition of water gives hydroxylamine (e), from which aldehyde (f) is split off to give ketonamine (g). This will not only destroy the isoxazoline ring, but also break the covalent linkage between the substituents at R* and the one at R**. Thus, in case the isoxazoline ring is part of a bioconjugate, the payload is removed from the biomolecule, therefore effectively eliminating its use and possibly creating severe side-effects.

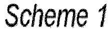

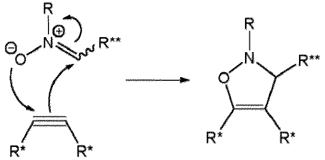

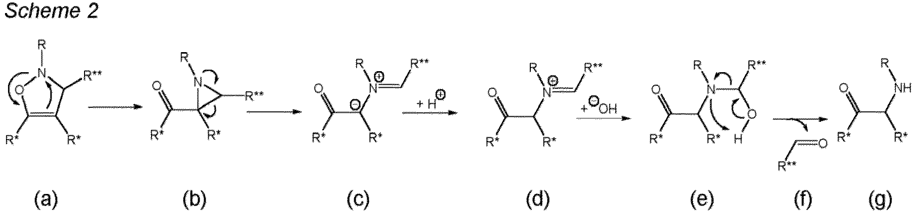

The solution for the above problem developed by the present inventors involves a specific substituent R on the nitrogen atom of the nitrone. In the context of the invention, the substituent on nitrogen, hereafter referred to as R¹, is L¹⁰XR⁴, wherein:
- L¹⁰ is a linker of structure (C(R³)₂)_{z}, wherein z is 2 or 3, and each R³ is individually selected from H and C₁₋₄ alkyl, wherein two occurrences of R³ may be joined together to form a C₃₋₆ (hetero)cycloalkyl group;
- R⁴ is selected from H and C₁₋₄ alkyl;
- X is S, O or NH,

Heteroatom X is able to capture the imine intermediate (d) of the rearrangement by reacting with the imine carbon atom. The inventors found that this leads not to splitting off of aldehyde (f), but instead leads to the rearrangement of Scheme 3 (exemplary scheme with R¹ = (CH₂)₂OH), wherein oxazolidine ring (h) is formed. This structure is stable and does not disintegrate. As such, the covalent connecting between the substituents at R* and at R** remains intact. The bioconjugate thus remains intact and effective, with the payload connected to the biomolecule. Thus, even though rearrangement of the 4-isoxazoline ring formed by the SPANC reaction is destroyed, the covalent connection is not, and therefore bioconjugates according to the present invention are a great improvement in terms of stability of the attachment of the payload to the biomolecule.

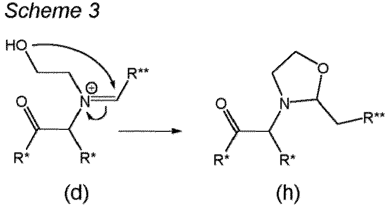

In a first aspect, the invention concerns a process for preparing an isoxazoline compound by strain-promoted alkyne nitrone cycloaddition (SPANC), comprising reacting a nitrone compound with a (hetero)cycloalkyne compound to obtain the isoxazoline compound, wherein the nitrone compound is according to structure (1): 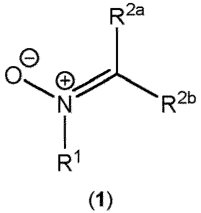 wherein:
- R¹ is L¹⁰XR⁴, wherein:
   - L¹⁰ is a linker of structure (C(R³)₂)_{z}, wherein z is 2 or 3, and each R³ is individually selected from H and C₁₋₄ alkyl, wherein two occurrences of R³ may be joined together to form a C₃₋₆ (hetero)cycloalkyl group;
   - R⁴ is selected from H and C₁₋₄ alkyl;
   - X is S, O or NH,
- R^{2a} is selected from H and C₁₋₅ (cyclo)alkyl;
- R^{2b} is selected from R¹³ is selected from the group consisting of C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups, or wherein R^{2b} is L(D)ᵣ, wherein D is a payload, r is an integer in the range of 1 - 10, and L is a linker covalently connecting D with the nitrone group, or R^{2b} is L⁶B, wherein B is a biomolecule and L⁶ is a linker covalently connecting B with the nitrone group.

In a second aspect, the invention concerns bioconjugates obtainable by the process according to the first aspect of the invention. Alternatively, the bioconjugates according to the second aspect can also be defined as having structure (3), (4), (5) or (6): 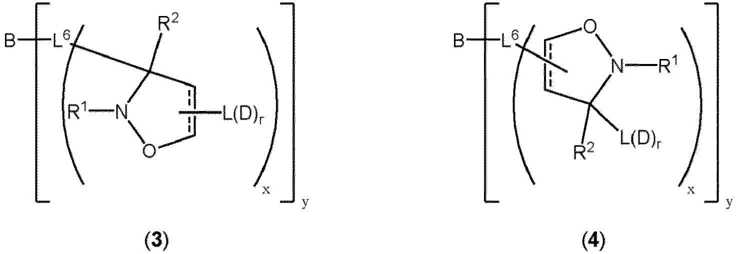 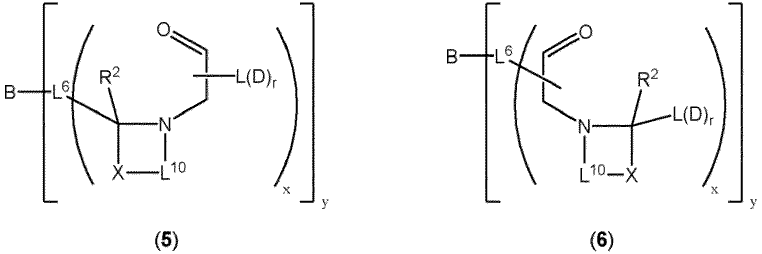 wherein:
- B is a biomolecule;
- D is a payload;
- L⁶ is a linker covalently connecting B with the isoxazoline group;
- L is a linker covalently connecting the isoxazoline group with (D)ᵣ;
- r is an integer in the range of 1 - 10;
- x is an integer in the range of 1 - 4;
- y is an integer in the range of 1 - 10;
- R¹ is L¹⁰XR⁴;
- L¹⁰ is a linker of structure (C(R³)₂)_{z}, wherein z is 2 or 3, and each R³ is individually selected from H and C₁₋₄ alkyl, wherein two occurrences of R³ may be joined together to form a C₃₋₆ (hetero)cycloalkyl group;
- R⁴ is selected from H and C₁₋₄ alkyl;
- XisS, OorNH,
- R² is selected from H and C₁₋₄ alkyl.

Herein, bioconjugates having structure (3) or (4) correspond to the isoxazoline compounds obtained by the strain-promoted alkyne nitrone cycloaddition of the first aspect, whereas bioconjugates having structure (5) or (6) correspond to the rearrangement products thereof.

The bioconjugates according to the second aspect of the invention are ideally suited for the targeting of tumour cells, in particular for the treatment of cancer. Therefore, the invention also concerns a method for targeting a tumour cell expressing a specific extracellular receptor, comprising contacting the bioconjugate according to the second aspect of the invention with cells that may possibly express the extracellular receptor, wherein the antibody specifically targets the extracellular receptor. Likewise, the invention also concerns a method for the treatment of cancer, comprising administering to a subject in need thereof the bioconjugate according to the second aspect of the invention, wherein the cancer cells specifically express an extracellular receptor.

The invention according to the present aspect further concerns a pharmaceutical composition comprising the bioconjugate according to the second aspect of the invention and a pharmaceutically acceptable carrier. The invention according to the present aspect further concerns the medical use of the bioconjugates according to the invention.

The bioconjugates according to the invention have improved stability, especially regarding reduced non-induced release of a payload from the bioconjugate. This leads to an improved shelf-life of the bioconjugate according to the invention. Furthermore, when the bioconjugate according to the invention is used in treatment, the improved stability leads to an improved safety (higher tolerability), since less payloads (typically cytotoxins) are released at locations where the payload should not be released and causes an adverse reaction, and an improved efficacy, since more payloads are released at the location where the payload should effectuate a beneficial medical effect. This in turn leads to an improved therapeutic index for the conjugates according to the invention.

In a third aspect, the invention concerns a nitrone compound having structure (1). 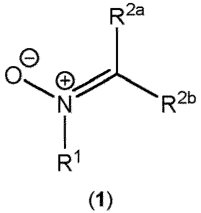 wherein:
- R¹ is L¹⁰XR⁴, wherein:
   - L¹⁰ is a linker of structure (C(R³)₂)_{z}, wherein z is 2 or 3, and each R³ is individually selected from H and C₁₋₄ alkyl, wherein two occurrences of R³ may be joined together to form a C₃₋₆ (hetero)cycloalkyl group;
   - R⁴ is selected from H and C₁₋₄ alkyl;
   - X is S, O or NH,
- R^{2a} is selected from H and C₁₋₄ alkyl;
- R^{2b} is selected from R¹³ is selected from the group consisting of C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups, or wherein R^{2b} is L(D)ᵣ, wherein D is a payload, r is an integer in the range of 1 - 10, and L is a linker covalently connecting D with the nitrone group, or R² is L⁶B, wherein B is a biomolecule and L⁶ is a linker covalently connecting B with the nitrone group.

The nitrone compound according to the third aspect of the invention are ideally suited as intermediates in the synthesis of the bioconjugates according to the invention. Hence, the invention further concerns the use of the nitrone compound according to the invention for preparing an isoxazoline compound by strain-promoted alkyne nitrone cycloaddition (SPANC). Preferably, the isoxazoline compound is a bioconjugate according to the invention. The use according to the present aspect is typically for improving the stability of the bioconjugate, for reducing non-induced release of a payload D from the bioconjugate, and/or for improving the therapeutic index of the bioconjugate. Such improvements are obtained over bioconjugates prepared by a SPANC reaction using a nitrone compound not according to the invention, in particular having a different substituents R1.

Also contemplated within the present invention are salts, preferably pharmaceutically acceptable salts, of all compounds according to the invention, including the bioconjugates.

Here below, the nitrone compound according to the third aspect is first defined. The structural features of the nitrone compound according to the invention also apply to the bioconjugates according to the invention and the process for preparing the isoxazoline compounds, in particular the bioconjugates according to the invention. The skilled person understands that any structural feature that is unchanged in the conjugation reaction is defined equally for each of the compounds according to the invention. In the conjugation reactions, only reactive moieties F and Q are transformed into connecting groups Z. As will be understood by the skilled person, the definition of the chemical moieties, as well as their preferred embodiments, apply to all aspects of the invention.

### Nitrone compound of structure (1)

Nitrone compounds are well-known in the art, and the skilled person is able to obtain the nitrone compound according to the invention using established organic chemistry. Suitable methods to prepare the nitrone compounds according to the invention are provided in the examples. As shown, the nitrones can efficiently be prepared from the corresponding hydroxylamines. Nitrones have two substituents on the carbon atom (herein referred to as R^{2a} and R^{2b}), and one on nitrogen (herein referred to as R¹). The benefits of the present invention are obtained with the specific nature of R¹.

The nitrone compound according to the invention has structure (1):

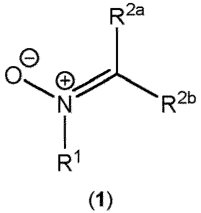

R¹ is L¹⁰XR⁴. Herein, X is a heteroatom having a lone pair which is capable of capturing the imine intermediate (d) by reacting with the imine carbon atom (see Scheme 3). In case this reaction forms a 5- or 6-membered ring, this capture of imine intermediate (d) is efficient and will stop the rearrangement reaction of Scheme 2. Hence, L¹⁰ should be a linker of two or three carbon atoms.

More specifically, L¹⁰ is a linker of structure (C(R³)₂)_{z}, wherein z is 2 or 3. Each R³ is individually selected from H and C₁₋₄ alkyl. Alternatively, two occurrences of R³ may be joined together to form an oxo group or a C₃₋₆ (hetero)cycloalkyl group. Preferably, L¹⁰ is a linker of structure CH₂-C(R³)₂ or CH₂-CH₂-C(R³)₂, more preferably L¹⁰ = CH₂-C(R³)₂. In case two occurrences of R³ are joined together to form a spiro-connected ring, it is preferred that such a ring is a C₃ - C₆ ring, preferably a C₄ or C₅ ring.

Preferred embodiments of L¹⁰ are (L¹⁰A) - (L¹⁰P):

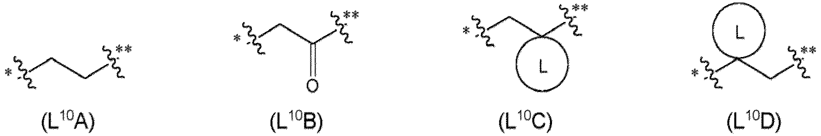

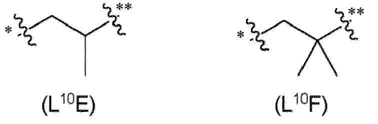

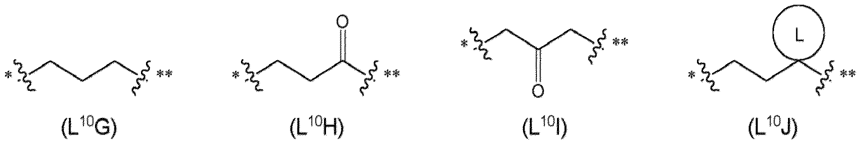

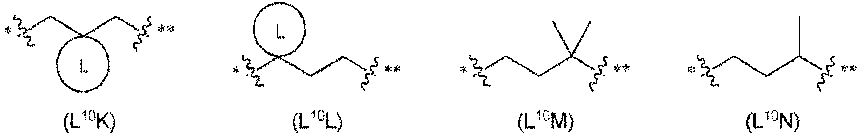

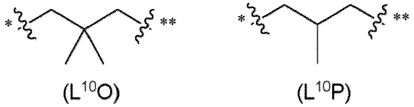

Herein, the wavy bond labelled with * is connected to the nitrogen atom of the nitrone group and the wavy bond labelled with * is connected to XR⁴. Ring (L) is spiro connected to the backbone atoms of L¹⁰. Ring (L) is preferably a cyclobutyl ring or a cyclopentyl ring, most preferably a cyclobutyl ring. Especially preferred are (L¹⁰A), (L¹⁰B), (L¹⁰C) and (L¹⁰G), wherein ring L is a cyclobutyl ring.

X is S, O or NH. Most preferably, X is O. R⁴ is selected from H and C₁₋₄ alkyl. Typically, R⁴ is H when X is O or NH, and R⁴ is H or C₁₋₄ alkyl when X is S. Thus, XR⁴ is typically selected from OH, NH₂, SH and S-C₁₋₄ alkyl. In a preferred embodiment, XR⁴ is SH, OH, NH₂, most preferably XR⁴ is OH.

The nature of R^{2a} and R^{2b} is not crucial for the present invention, and any suitable substituent for a nitrone compound can be used. R^{2a} and R^{2b} may be the same or different, typically they are different. In case R^{2a} and R^{2b} are different, the configuration of the double bound between the nitrogen atom and carbon atom of the nitrone group may either be in E-configuration of in Z-configuration. The exact configuration has no influence on the working of the present invention.

Typically, R^{2a} is selected from H and C₁ - C₆ (cyclo)alkyl. In a preferred embodiment, R^{2a} is selected from H and C₁ - C₅ (cyclo)alkyl, more preferably R^{2a} is H, Me or Et, most preferably R^{2a} is H.

Typically, R^{2b} is selected from the group consisting of C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, which may optionally be substituted and which may optionally be interrupted by one or more heteroatoms selected from O, S and NR¹⁴, wherein R¹⁴ is independently selected from the group consisting of hydrogen and C₁ - C₄ alkyl groups. Alternatively, R^{2a} and R^{2b} are joined to form a (hetero)cyclic moiety. In an alternative embodiment, R^{2b} is L(D)ᵣ, wherein D is a payload, r is an integer in the range of 1 - 10, and L is a linker covalently connecting D with the nitrone group. In a further alternative embodiment, R^{2b} is L⁶B, wherein B is a biomolecule and L⁶ is a linker covalently connecting B with the nitrone group. Preferred embodiments of biomolecule B, payload D, linkers L and L⁶ and integer r are defined further below.

In a preferred embodiment, R^{2b} is hydrogen, a C₁ - C₂₀ alkyl group, **preferably** a C₁-- C₁₆ alkyl group, more preferably a C₁- C₁₀ alkyl group, L(D)ᵣ or L⁶B. Herein, the alkyl group is optionally substituted and optionally interrupted by one or more heteroatoms selected from O, S and NR¹⁴, preferably O, wherein R¹⁴ is independently selected from the group consisting of hydrogen and C₁ - C₄ alkyl groups. In another preferred embodiment, R^{2b} is selected from the group consisting of hydrogen, methyl, ethyl, n-propyl, i-propyl, n-butyl, s-butyl, t-butyl, L(D)ᵣ or L⁶B, more preferably from the group consisting of hydrogen, methyl, ethyl, n-propyl, i-propyl, L(D)ᵣ or L⁶B, and even more preferably from the group consisting of hydrogen, methyl, ethyl, L(D)ᵣ or L⁶B.

It is especially preferred that the nitrone compound according to the invention is used in the preparation of a bioconjugate, wherein a biomolecule B is covalently connected to a payload D. Hence, it is especially preferred that R^{2b} is L(D)ᵣ or L⁶B. In one embodiment, R^{2b} is L(D)ᵣ and the nitrone compound is to be coupled with a (hetero)cycloalkyne compound comprising a biomolecule B. In one embodiment, R^{2b} is L⁶B and the nitrone compound is to be coupled with a (hetero)cycloalkyne compound comprising a payload D. It is especially preferred that the nitrone is coupled to the payload and thus that R^{2b} is L(D)ᵣ.

In an especially preferred embodiment, the nitrone is introduced at the terminal amino acid of a polypeptide. Here, the polypeptide can be biomolecule B (e.g. an antibody) or payload D. Preferably, payload D is a polypeptide, wherein the terminal amino acid is converted into a nitrone group, in which case R^{2b} = L(D)ᵣ, wherein r is typically 1.

Thus, it is preferred that the nitrone with R^{2b} = L(D)ᵣ is obtained by converting a terminal serine or threonine into a nitrone. The conversion into a nitrone can be realised by oxidizing the serine or threonine side chain and then converting into a nitrone as shown below:

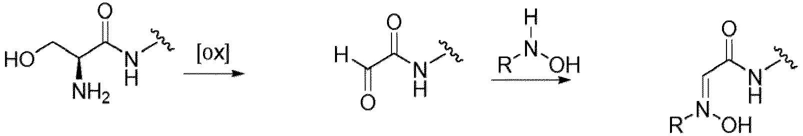

Such conversion of serine or threonine into a nitrone can be performed by conventional chemical conversions. Herein, [ox] is an oxidizing agent, such as NaIO₄. R corresponds to R¹ as defined above. It is especially preferred that the oxidation of the terminal amino acid, conversion into a nitrone and the conjugation to the (hetero)cycloalkyne are performed in one pot synthesis.

### Process for preparing an isoxazoline compound

The invention in a further aspect concerns a process for preparing an isoxazoline compound by strain-promoted alkyne nitrone cycloaddition (SPANC). In the process according to the invention, the nitrone compound according to structure (1) is reacted with a (hetero)cycloalkyne compound to obtain the isoxazoline compound. The nitrone compound according to structure (1) is defined above. The SPANC reaction is a click reaction, wherein a first click probe (the nitrone) reacts with a second click probes (the (hetero)cycloalkyne) to form a connecting group Z (the 4-isoxazoline ring). This conjugation technique is known to the skilled person. The present reactions occur under conditions such that the nitrone is reacted with the (hetero)cycloalkyne to form the 4-isoxazoline ring. The skilled person is capable of performing this reaction using the appropriate reaction conditions.

The reaction of the nitrone according to structure (1) can occur with any (hetero)cycloalkyne compound that is suitable for a SPANC reaction. The (hetero)cycloalkyne compound comprises a (hetero)cycloalkyne group, herein also referred to as Q, which may be substituted in any suitable way, depending on the desired structure of the final isoxazoline compound.

### (Hetero)cycloalkyne group Q

(Hetero)cycloalkyne group Q may be a heterocycloalkyne group or a cycloalkyne group. Preferably, the (hetero)cycloalkynyl group is a (hetero)cycloheptynyl group, a (hetero)cyclooctynyl group, a (hetero)cyclononynyl group or a (hetero)cyclodecynyl group. Herein, the (hetero)cycloalkynes may optionally be substituted. Preferably, the (hetero)cycloalkynyl group is an optionally substituted (hetero)cycloheptynyl group or an optionally substituted (hetero)cyclooctynyl group. Most preferably, the (hetero)cycloalkynyl group is a (hetero)cyclooctynyl group, wherein the (hetero)cyclooctynyl group is optionally substituted.

In an especially preferred embodiment, Q comprises a (hetero)cycloalkynyl according to structure (Q1): 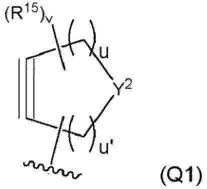 Herein:
- the wavy bond represents the connection to the remainder of the (hetero)cycloalkynyl compound;
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y² is C(R³¹)₂, O, S, S⁽⁺⁾R³¹, S(O)R³¹, S(O)=NR³¹ or NR³¹, wherein S⁽⁺⁾ is a cationic sulphur atom counterbalanced by B⁽⁻⁾, wherein B⁽⁻⁾ is an anion, and wherein each R³¹ individually is R¹⁵ or a connection with D, connected via L;
- u is 0, 1, 2, 3, 4 or 5;
- u' is 0, 1, 2, 3, 4 or 5, wherein u + u' = 4, 5, 6, 7 or 8;
- v is an integer in the range 8 - 16;

Typically, v = (u + u') x 2 (when the connection to L, depicted by the wavy bond, is via Y²) or [(u + u') x 2] - 1 (when the connection to L, depicted by the wavy bond, is via one of the carbon atoms of u and u'). In a preferred embodiment, u + u' = 4, 5 or 6, more preferably u + u' = 5. In a preferred embodiment, v = 8, 9 or 10, more preferably v = 9 or 10, most preferably v = 10.

In a preferred embodiment, Q is a (hetero)cycloalkynyl group selected from the group consisting of (Q2) - (Q20c) depicted here below.

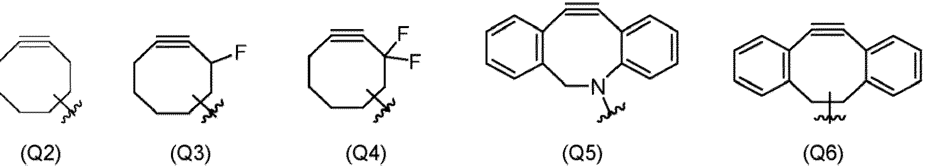

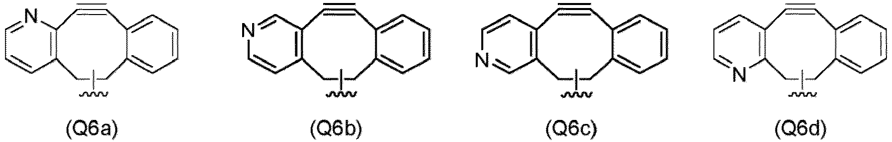

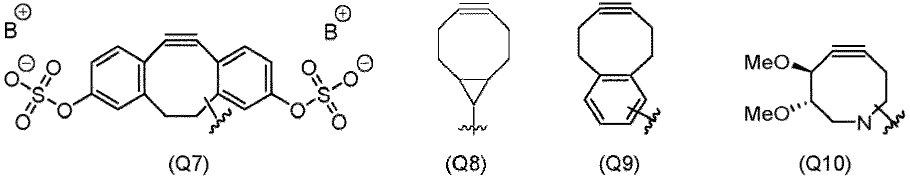

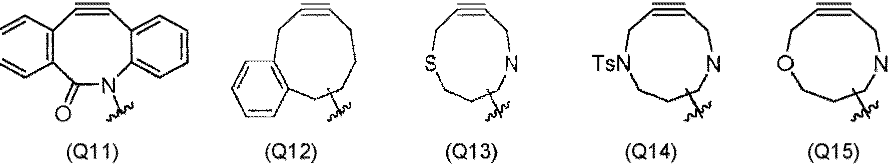

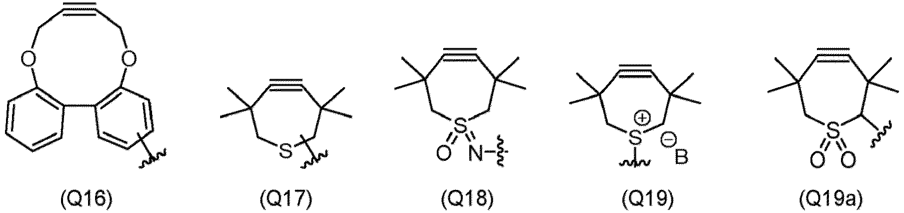

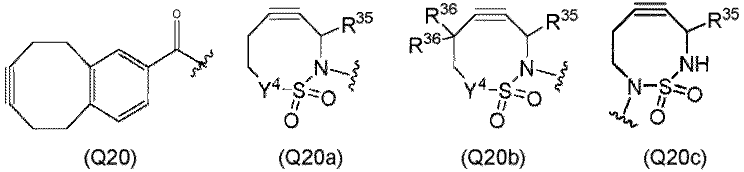

Herein, the connection to L, depicted with the wavy bond, may be to any available carbon or nitrogen atom of Q. The nitrogen atom of (Q10), (Q13), (Q14) and (Q15) may bear the connection to L, or may contain a hydrogen atom or be optionally functionalized. B⁽⁻⁾ is an anion, which is preferably selected from ⁽⁻⁾OTf, Cl⁽⁻⁾, Br⁽⁻⁾ or I⁽⁻⁾, most preferably B⁽⁻⁾ is ⁽⁻⁾OTf. B⁽⁺⁾ is a cation, preferably a pharmaceutically acceptable cation. In the conjugation reaction, B⁽⁻⁾ does not need to be a pharmaceutically acceptable anion, since B⁽⁻⁾ will exchange with the anions present in the reaction mixture anyway. In case (Q19) is used for Q, the negatively charged counter-ion is preferably pharmaceutically acceptable upon isolation of the conjugate according to the invention, such that the conjugate is readily useable as medicament. R³⁶ is an halogen selected from fluor, chlorine, bromine and iodine, preferably R³⁶ is fluor. Y⁴ is a heteroatom, preferably Y⁴ is O or NH. R³⁵ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl (hetero) aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, Si, S and NR¹⁴ wherein R¹⁴ is independently selected from the group consisting of hydrogen and C₁ - C₄ alkyl groups, preferably R³⁵ is selected from H, C₅H₁₁, CH₃, CH₂CH₃, CH₂OH or CH₂OTBS.

In a further preferred embodiment, Q is a (hetero)cycloalkynyl group selected from the group consisting of (Q21) - (Q38d) depicted here below.

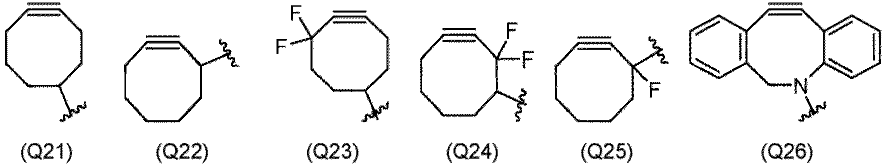

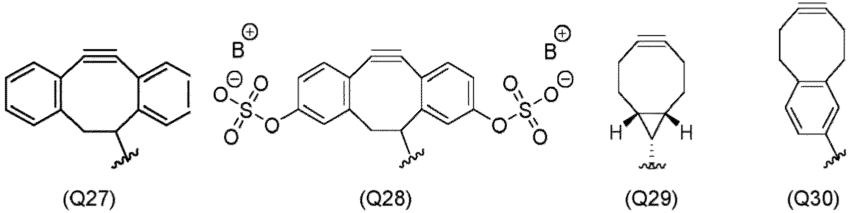

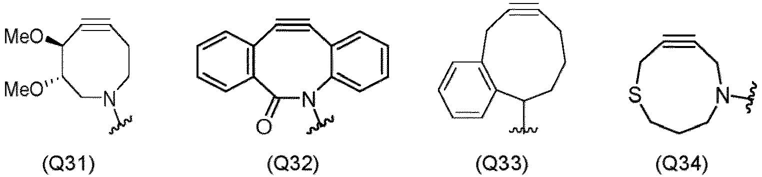

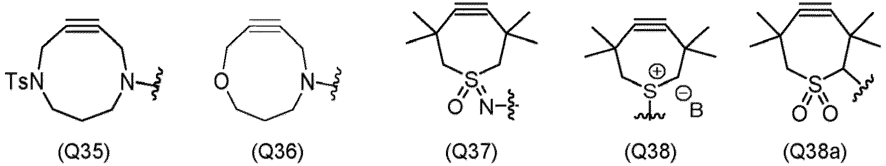

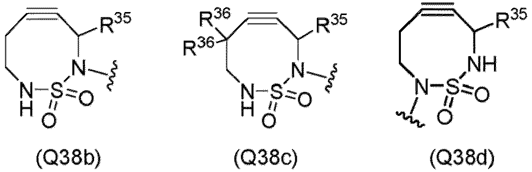

In structure (Q38), B⁽⁻⁾ is an anion, which is preferably selected from ⁽⁻⁾OTf, Cl⁽⁻⁾, Br⁽⁻⁾ or I⁽⁻⁾, most preferably B⁽⁻⁾ is ⁽⁻⁾OTf. In structure (Q28), B⁽⁺⁾ is a cation, preferably a pharmaceutically acceptable cation. Groups R³⁵ and R³⁶ on (Q38b), (Q38c) and (Q38d) are defined elsewhere and equally apply to the present embodiment.

In a preferred embodiment, Q comprises a (hetero)cyclooctyne moiety or a (hetero)cycloheptyne moiety, preferably according to structure (Q8), (Q26), (Q27), (Q28), (Q37) or (Q38a), which are optionally substituted. Each of these preferred options for Q are further defined here below.

Thus, in a preferred embodiment, Q comprises a heterocycloheptyne moiety according to structure (Q37), also referred to as a TMTHSI, which is optionally substituted. Preferably, the heterocycloheptyne moiety according to structure (Q37) is not substituted.

In an alternative preferred embodiment, Q comprises a cyclooctyne moiety according to structure (Q8), more preferably according to (Q29), also referred to as a bicyclo[6.1.0]non-4-yn-9-yl] group (BCN group), which is optionally substituted. Preferably, the cyclooctyne moiety according to structure (Q8) or (Q29) is not substituted. In the context of the present embodiment, Q preferably is a (hetero)cyclooctyne moiety according to structure (Q39) as shown below, wherein V is (CH₂)_{I} and I is an integer in the range of 0 to 10, preferably in the range of 0 to 6. More preferably, I is 0, 1, 2, 3 or 4, more preferably I is 0, 1 or 2 and most preferably I is 0 or 1. In the context of group (Q39), I is most preferably 1. Most preferably, Q is according to structure (Q42), defined further below.

In an alternative preferred embodiment, Q comprises a (hetero)cyclooctyne moiety according to structure (Q26), (Q27) or (Q28), also referred to as a DIBO, DIBAC, DBCO or ADIBO group, which are optionally substituted. In the context of the present embodiment, Q preferably is a (hetero)cyclooctyne moiety according to structure (Q40) or (Q41) as shown below, wherein Y¹ is O or NR¹¹, wherein R¹¹ is independently selected from the group consisting of hydrogen, a linear or branched C₁ - C₁₂ alkyl group or a C₄ - C₁₂ (hetero)aryl group. The aromatic rings in (Q40) are optionally O-sulfonylated at one or more positions, whereas the rings of (Q41) may be halogenated at one or more positions. Preferably, the (hetero)cyclooctyne moiety according to structure (Q40) or (Q41) is not further substituted. Most preferably, Q is according to structure (Q43), defined further below.

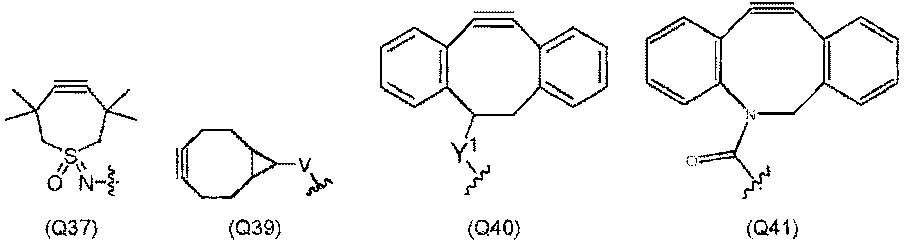

In an especially preferred embodiment, Q comprises a cyclooctynyl group and is according to structure (Q42): 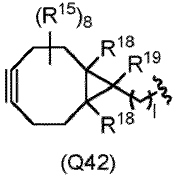 Herein:
- R¹⁵ is independently selected from the group consisting of hydrogen, halogen, -OR¹⁶, -NO₂, -CN, -S(O)₂R¹⁶, -S(O)₃⁽⁻⁾,C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of D connected via a spacer moiety; and
- I is an integer in the range 0 to 10.

In a preferred embodiment of the reactive group according to structure (Q42), R¹⁵ is independently selected from the group consisting of hydrogen, halogen, -OR¹⁶, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and C₁ - C₆ alkyl, most preferably all R¹⁵ are H. In a preferred embodiment of the reactive group according to structure (Q42), R¹⁸ is independently selected from the group consisting of hydrogen, C₁ - C₆ alkyl groups, most preferably both R¹⁸ are H. In a preferred embodiment of the reactive group according to structure (Q42), R¹⁹ is H. In a preferred embodiment of the reactive group according to structure (Q42), I is 0 or 1, more preferably I is 1.

In an especially preferred embodiment, Q comprises a (hetero)cyclooctynyl group and is according to structure (Q43): 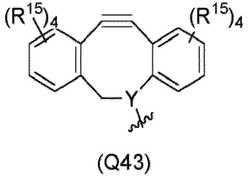 Herein:
- R¹⁵ is independently selected from the group consisting of hydrogen, halogen, -OR¹⁶, -NO₂, - CN, -S(O)₂R¹⁶, -S(O)₃⁽⁻⁾, C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y is N or CR¹⁵;
- a carbon atom in the fused aromatic rings may be replaced by a nitrogen atom, as in (Q6a) - (Q6d), preferably wherein Y is CR¹⁵.

In a preferred embodiment of the reactive group according to structure (Q43), R¹⁵ is independently selected from the group consisting of hydrogen, halogen, -OR¹⁶, -S(O)₃⁽⁻⁾, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and -S(O)₃⁽⁻⁾. In a preferred embodiment of the reactive group according to structure (Q43), Y is N or CH, more preferably Y = N.

In an especially preferred embodiment, Q comprises a (hetero)cycloheptynyl group and is according to structure (Q37) or (Q38a).

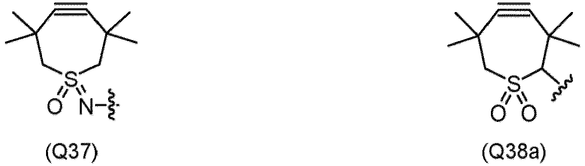

In the preferred embodiments for (hetero)cycloalkynyl group Q defined above, the wavy bond at all times refers to the connection to the remainder of the (hetero)cycloalkynyl compound. Even though the exact nature of this substituent of the (hetero)cycloalkynyl group is not relevant in the context of the present invention, it is preferred that the wavy bond represents the connection to L⁶B or L(D)ᵣ. Thus, in a preferred embodiment of the present invention:
- R^{2b} is L(D)ᵣ and the (hetero)cycloalkyne compound is according to structure Q-L⁶-B, or
- R^{2b} is L⁶B and the alkyne compound is according to structure Q-L(D)ᵣ.

Herein, B, L⁶, L, D and r are defined further below in the context of the bioconjugates according to the invention. As such, the process according to the present invention is for preparing a bioconjugate, and the SPANC reaction is a bioconjugation reaction that forms a covalent link between a biomolecule B and a payload D. Thus, in an especially preferred embodiment, the process according to the present invention is for the preparation of a bioconjugate, especially according to structure (3) or (4) defined below.

The process according to the present aspect of the invention is preferably a process for preparing a bioconjugate, wherein the SPANC reaction step as defined above is a bioconjugation reaction. Preferably, the present invention relates to a process for the preparation of a bioconjugate according to the invention, the process comprising:
(a) providing a modified biomolecule having the structure B(F¹)_{(x×y)}, wherein B is the biomolecule, preferably wherein B is an antibody, x is an integer in the range of 1 - 4, y is an integer in the range of 1 - 10, and F is a click probe selected from the nitrone group or the (hetero)cycloalkyne group as defined elsewhere;
(b) reacting the modified biomolecule with x × y equivalents of F²L(D)ᵣ, wherein F² is a click probe selected from the nitrone group or the (hetero)cycloalkyne group as defined elsewhere and that is reactive towards F¹, L is a linker, and r is an integer in the range of 1 - 10, to obtain an bioconjugate according to the invention.

Herein, steps (b) corresponds to the SPANC reaction as defined above.

### Step (a)

In step (a), a biomolecule having the structure B(F¹)_{(x × y)} is provided. Typically, two or four click probes F¹ are chemically or enzymatically introduced onto the antibody. Preferably, x = 1 and y = 2 and two click probes F¹ are introduced onto the antibody. In one embodiment, the click probe F¹ connected to the biomolecule is the (hetero)cycloalkynyl group Q as defined above. In another embodiment, the click probe F¹ connected to the biomolecule is the nitrone as defined above, wherein R^{2b} is L⁶B. If F¹ is the (hetero)cycloalkynyl as group Q defined above, than F² is the nitrone as defined above, and if F¹ is the nitrone as defined above, than F² is the (hetero)cycloalkynyl group Q as defined above.

In a preferred embodiment, an antibody comprising two or four, preferably two, core N-acetylglucosamine moieties is contacted with a compound of the formula S(F¹)ₓ-P in the presence of a catalyst, wherein S(F¹)ₓ is a sugar derivative comprising x click probes F¹, and P is a nucleoside mono- or diphosphate, and wherein the catalyst is capable of transferring the S(F¹)ₓ moiety to the core-GIcNAc moiety. Herein, the antibody is typically an antibody that has been trimmed to a core-GlcNAc residue as described further below.

The starting material, i.e. the antibody comprising two or four, preferably two, core-GIcNAc substituents, is known in the art and can be prepared by methods known by the skilled person. In one embodiment, the process according to the invention further comprises the deglycosylation of an antibody glycan having a core N-acetylglucosamine, in the presence of an endoglycosidase, in order to obtain an antibody comprising a core N-acetylglucosamine substituent, wherein said core N-acetylglucosamine and said core N-acetylglucosamine substituent are optionally fucosylated. Depending on the nature of the glycan, a suitable endoglycosidase may be selected. The endoglycosidase is preferably selected from the group consisting of EndoS, EndoA, EndoE, EfEndo18A, EndoF, EndoM, EndoD, EndoH, EndoT and EndoSH and/or a combination thereof, the selection of which depends on the nature of the glycan. EndoSH is described in PCT/EP2017/052792, see Examples 1 - 3, and SEQ. ID No: 1, which is incorporated by reference herein.

Structural feature S is defined below for the conjugate according to the invention, which equally applies to the present aspect. Compounds of the formula S(F¹)ₓ-P, wherein a nucleoside monophosphate or a nucleoside diphosphate P is linked to a sugar derivative S(F¹)ₓ, are known in the art. In a preferred embodiment nucleoside mono- or diphosphate P in S(F¹)ₓ-P is selected from the group consisting of uridine diphosphate (UDP), guanosine diphosphate (GDP), thymidine diphosphate (TDP), cytidine diphosphate (CDP) and cytidine monophosphate (CMP), more preferably P is selected from the group consisting of uridine diphosphate (UDP), guanosine diphosphate (GDP) and cytidine diphosphate (CDP), most preferably P = UDP.

Suitable catalyst that are capable of transferring the S(F¹)ₓ moiety to the core-GIcNAc moiety are known in the art. A suitable catalyst is a catalyst wherefore the specific sugar derivative nucleotide S(F¹)ₓ-P in that specific process is a substrate. More specifically, the catalyst catalyses the formation of a β(1,4)-glycosidic bond. Preferably, the catalyst is selected from the group of galactosyltransferases and N-acetylgalactosaminyltransferases, more preferably from the group of P(1,4)-N-acetylgalactosaminyltransferases (GaINAcT) and β(1,4)-galactosyltransferases (GalT), most preferably from the group of β(1,4)-N-acetylgalactosaminyltransferases having a mutant catalytic domain. Suitable catalysts and mutants thereof are disclosed in WO 2014/065661, WO 2016/022027 and WO 2016/170186, all incorporated herein by reference. In one embodiment, the catalyst is a wild-type galactosyltransferase or *N*-acetylgalactosaminyltransferase, preferably an N-acetylgalactosaminyltransferase. In an alternative embodiment, the catalyst is a mutant galactosyltransferase or N-acetylgalactosaminyltransferases, preferably a mutant N-acetylgalactosaminyltransferase. Mutant enzymes described in WO 2016/022027 and WO 2016/170186 are especially preferred. These galactosyltransferase (mutant) enzyme catalysts are able to recognize internal sugars and sugar derivatives as an acceptor. Thus, sugar derivative S(F¹)ₓ is linked to the core-GIcNAc substituent in step (a), irrespective of whether said GIcNAc is fucosylated or not.

Step (a) is preferably performed in a suitable buffer solution, such as for example phosphate, buffered saline (e.g. phosphate-buffered saline, tris-buffered saline), citrate, HEPES, tris and glycine. Suitable buffers are known in the art. Preferably, the buffer solution is phosphate-buffered saline (PBS) or tris buffer. Step (a) is preferably performed at a temperature in the range of about 4 to about 50 °C, more preferably in the range of about 10 to about 45 °C, even more preferably in the range of about 20 to about 40 °C, and most preferably in the range of about 30 to about 37 °C. Step (a) is preferably performed a pH in the range of about 5 to about 9, preferably in the range of about 5.5 to about 8.5, more preferably in the range of about 6 to about 8. Most preferably, step (a) is performed at a pH in the range of about 7 to about 8.

Alternatively, the nitrone or (hetero)cycloalkynyl group is introduced in a two-step procedure, wherein first an alternative reactive group F³ is introduced onto the biomolecule B via chemically or enzymatically introduction, which provides B(F³)_{(x × y)}. Preferably, S(F³)ₓ-P is introduced by the procedure as described above for S(F¹)ₓ-P. Preferred options for S(F³)ₓ-P are selected from the group consisting of GalNAz-UDP, F₂-GalNAz-UDP (*N*-(azidodifluoro)acetylgalactosamine), 6-AzGal-UDP, 6-AzGaINAc-UDP (6-azido-6-deoxy-N-acetylgalactosamine-UDP), 4-AzGalNAz-UDP, 6-AzGalNAz-UDP, GIcNAz-UDP, 6-AzGlc-UDP, 6-AzGlcNAz-UDP and 2-(but-3-yonic acid amido)-2-deoxy-galactose-UDP. Most preferably, S(F³)ₓ-P is GaINAz-UDP or 6-AzGaINAc-UDP. Alternatively, reactive group F³ may already be present naturally in the biomolecule B, for example the thiol group of a cysteine side chain or the amine group of a lysine side chain.

In a second step, B(F³)_{(x × y)} is reacted with x × y equivalents of a linker construct of structure F⁴-L⁸-F¹, wherein L⁸ is a linker connection F⁴ with F¹, and F⁴ is a reactive group that is reactive towards F³ and not towards F¹. This reaction yields B(Z¹-L⁸-F¹)_{(x × y)}, wherein Z¹ is a connecting group obtained by the reaction of F³ and F⁴. The obtained B(Z¹-L⁸-F¹)_{(x × y)} is then used as modified biomolecule having the structure B(F¹)_{(x × y)} in step (b).

Reactive groups F³ and F⁴ are complementary groups as known in the art. Several examples of suitable combinations of F³ and F⁴, and of connecting group Z¹ that is obtained by the reaction are shown in Figure 3. For example, when F³ comprises or is a thiol group, complementary groups F⁴ include N-maleimidyl groups, alkenyl groups and allenamide groups. For example, when F³ comprises or is an amino group, complementary groups F⁴ include ketone groups and activated ester groups. For example, when F³ comprises or is a ketone group, complementary groups F⁴ include (O-alkyl)hydroxylamino groups and hydrazine groups. For example, when F³ comprises or is an alkynyl group, complementary groups F⁴ include azido groups. For example, when F³ comprises or is an azido group, complementary groups F⁴ include alkynyl groups. For example, when F³ comprises or is a cyclopropenyl group, a trans-cyclooctene group, a cycloheptyne or a cyclooctyne group, complementary groups F⁴ include tetrazinyl groups. In these particular cases, Z is only an intermediate structure and will expel N₂, thereby generating a dihydropyridazine (from the reaction with alkene) or pyridazine (from the reaction with alkyne) as shown in Figure 3.

Additional suitable combinations of F³ and F⁴, and the nature of resulting connecting group Z¹ are known to a person skilled in the art, and are e.g. described in G.T. Hermanson, "Bioconjugate Techniques", Elsevier, 3rd Ed. 2013 (ISBN:978-0-12-382239-0), in particular in Chapter 3, pages 229 - 258, incorporated by reference. A list of complementary reactive groups suitable for bioconjugation processes is disclosed in Table 3.1, pages 230 - 232 of Chapter 3 of G.T. Hermanson, "Bioconjugate Techniques", Elsevier, 3rd Ed. 2013 (ISBN:978-0-12-382239-0), and the content of this Table is expressly incorporated by reference herein.

In a preferred embodiment, connecting group Z¹ is obtained by a cycloaddition or a nucleophilic reaction, preferably wherein the cycloaddition is a [4+2] cycloaddition or a 1,3-dipolar cycloaddition or the nucleophilic reaction is a Michael addition or a nucleophilic substitution. Such a cycloaddition or nucleophilic reaction occurs via a reactive group F³, connected to B, and reactive group F⁴, connected to F¹ via L⁸. Conjugation reactions via cycloadditions or nucleophilic reactions are known to the skilled person, and the skilled person is capable of selecting appropriate reaction partners F³ and F⁴, and will understand the nature of the resulting connecting group Z¹.

In one embodiment, F¹ and F⁴ are the same reactive group and reactive towards both F³ and F². Preferably, F¹ and F⁴ are both the (hetero)cycloalkynyl group as defined above, F³ is an azido group and F² is the nitrone group as defined above.

### Step (b)

In step (b), the modified biomolecule B(F¹)_{(x × y)} is reacted with a linker construct, comprising a click probe F² capable of reacting with F¹, to obtain a bioconjugate, containing connecting group Z resulting from the reaction between F² and F¹. Such reaction occurs under condition such that reactive group F² reacts with F¹ to covalently link the biomolecule to the payload. In step (b), the reaction occurs with x × y equivalents of F²L(D)ᵣ, although more equivalents of F²L(D)ᵣ may be present in the reaction mixture in order to ensure complete reaction. The skilled person is able to determine the optimal reaction conditions and stoichiometry of the reactants in order to obtain an optimal yield.

### The bioconjugate

In a further aspect, the invention concerns a bioconjugate. The bioconjugates may contain the 4-isoxazoline ring, i.e. which has not undergone any rearrangement according to Schemes 2 and 3. Alternatively, the bioconjugates may contain the oxazolidine ring or the 1 ,3-oxazinane ring, i.e. wherein the 4-isoxazoline ring has undergone a rearrangement according to Schemes 2 and 3. In the context of the present invention, the 4-isoxazoline ring, the oxazolidine ring and the 1,3-oxazinane ring are referred to as "connecting group" (or Z), as these are the groups that are - directly or indirectly after rearrangement - obtained by the SPANC bioconjugation reaction.

Thus, in a first embodiment, the bioconjugate comprises a 4-isoxazoline ring and is according to structure (3) or (4):

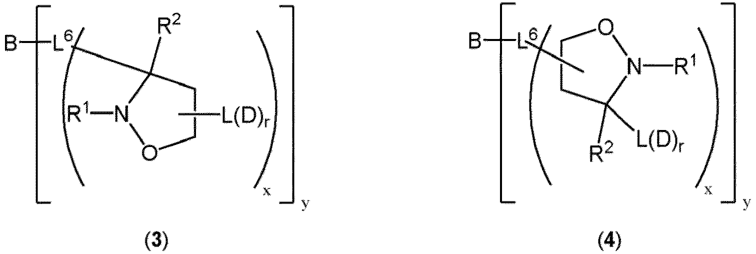

Alternatively, the bioconjugate comprises an oxazolidine ring or a 1,3-oxazinane ring and is according to structure (5) or (6):

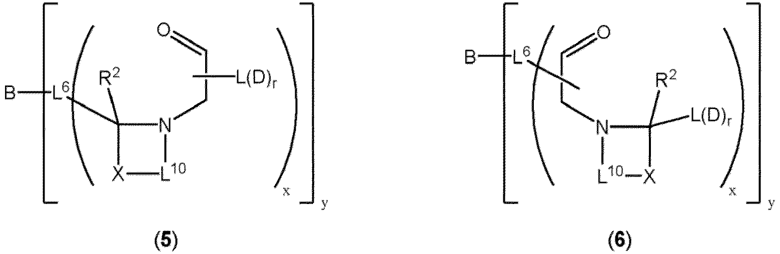

Herein, R¹, L¹⁰ and X are as defined for the nitrone compound according to the invention. R² corresponds to either one of R^{2a} or R^{2b}, as defined above for the nitrone compound. Typically, R² is as defined for R^{2a}. Further:
- B is a biomolecule;
- D is a payload;
- L⁶ is a linker covalently connecting B with the isoxazoline group;
- L is a linker covalently connecting the isoxazoline group with (D)ᵣ;
- r is an integer in the range of 1 - 10;
- x is an integer in the range of 1 - 4;
- y is an integer in the range of 1 - 10.

### Biomolecule B

The nature of the biomolecule B is not limited in the context of the present reaction. In a preferred embodiment, the biomolecule is selected from the group consisting of proteins (including glycoproteins such as antibodies), polypeptides, peptides, glycans, lipids, nucleic acids, oligonucleotides, polysaccharides, oligosaccharides, enzymes, hormones, amino acids and monosaccharides. More preferably, biomolecule B is selected from the group consisting of proteins, polypeptides, peptides and glycans. Preferably, the biomolecule contains a polypeptide part. An especially preferred class of biomolecules are glycoproteins, such as antibodies, which combine a hydrophilic peptide chain with a hydrophilic sugar chain (glycan). In a preferred embodiment, the biomolecule is selected from the group consisting of antibodies and fragments thereof.

Preferably, B is an antibody. Antibodies are known in the art and include IgA, IgD, IgE, IgG, IgM, Fab, VHH, scFv, diabody, minibody, affibody, affylin, affimers, atrimers, fynomer, Cys-knot, DARPin, adnectin/centryin, knottin, anticalin, FN3, Kunitz domain, OBody, bicyclic peptides and tricyclic peptides. Preferably, the antibody is a monoclonal antibody, more preferably selected from the group consisting of IgA, IgD, IgE, IgG and IgM antibodies. Even more preferably Ab is an IgG antibody. The IgG antibody may be of any IgG isotype. The antibody may be any IgG isotype, e.g. IgG1, IgG2, Igl3 or IgG4. Preferably Ab is a full-length antibody, but Ab may also be a Fc fragment.

The antibody Ab is typically specific for an extracellular receptor on a tumour cell, preferably wherein the extracellular receptor on the tumour cell is selected from the group consisting of consisting of 5T4, ADAM-9, AMHRII, ASCT2, ASLG659, ASPHD1, av-integrin, Axl, B7-H3, B7-H4, BAFF-R, BCMA, BMPR1B, brevican, c-KIT, c-Met, C4.4a, CA-IX, cadherin-6, CanAg, CD123, CD13, CD133, CD138/syndecan-1, CD166, CD19, CD20, CD203c, CD205, CD21, CD22, CD228, CD25, CD30, CD324, CD33, CD37, CD38, CD45, CD46, CD48a, CD56, CD70, CD71, CD72, CD74, CD79a, CD79b, CEACAM5, claudin-18.2, claudin-6, CLEC12A, CLL-1, Cripto, CRIPTO, CS1, CXCR5, DLK-1, DLL3, DPEP3, E16, EGFR, ENPP3, EpCAM, EphA2, EphB2R, ETBR, FAP, FcRH1, FcRH2, FcRH5, FGFR2, fibronectin, FLT3, folate receptor alpha (FOL-R1), Gal-3BP, GD3, GDNF-Ra1, GEDA, GFRA1, Globo H, gpNMB, GPR172A, GPR19, GPR54, guanyl cyclase C, HER2, HER3, HLA-DOB, IGF-1R, IL13R, IL20Rα, Lewis Y, LGR5, LIV-1, LRRC15, LY64, Ly6E, Ly6G6D, LY6K, MDP, MFI2, MICA/B, MOSPD2, MPF, MSG783, MUC1, MUC16, NaPi2b, NCA, nectin-4, Notch3, P-cadherin, P2X5, PD-L1, PMEL17, PRLR, PSCA, PSMA, PTK7, RET, RNF43, RON, ROR1, ROR2, SLITRK6, SSTR2, STEAP1, STEAP2, TAG72, TENB2, TF, TIM-1, TM4SF, TMEFF, TMEM118, TMEM46, transferrin, TROP-2, TrpM4, TWEAKR, receptor tyrosine kinases (RTK) and tenascin.

The antibody may also be specific to an extracellular protein resulting from a viral infection, e.g. human polio virus (HPV), human cytomegalovirus (HCMV) or human papillomavirus (HPV). The antibody may also be specific for a tumour-associated carbohydrate antigen (TACA) that is selected from the group of Tn, STn, T-antigen, LDN, Lewis^{c} (Le^{c}), Sialyl-Lewis^{c} (SLe^{c}), 6-Sialyl-Lewis^{c} (6SLe^{c}), LN, alpha-Gal, 3SLN, 6SLN, H-antigen, A-antigen, B-antigen, Lewis^{a} (Le^{a}), Sialyl-Lewis^{a} (SLe^{a}), 6-Sialyl-Lewis^{a} (6SLe^{a}), Lewis^{b} (Le^{b}), Sialyl-Lewis^{b} (SLe^{b}), 6-Sialyl-Lewis^{b} (6SLe^{b}), Lewis^{x} (Le^{x}), Sialyl-Lewis^{x} (SLe^{x}), 6-Sialyl-Lewis^{x} (6SLe^{x}), Lewis^{y} (Le^{y}), Sialyl-Lewis^{y} (SLe^{y}), 6-Sialyl-Lewis^{y} (6SLe^{y}) and or combinations thereof. The antibody may also be specific to both an extracellular protein and a TACA at the same time.

y is an integer that denotes the number of linkers L⁶ that are attached to a single biomolecule B. y is an integer in the range of 1 - 10, y is an integer in the range of 1 - 4, more preferably y = 2 or 4, most preferably y = 2.

x is an integer that denotes the number of connecting groups Z that are attached to a single linker L⁶. x is an integer in the range of 1 - 4, preferably x = 1 or 2, most preferably x = 1.

r is an integer that denotes the number of payloads D that are attached to a single linker L. r is an integer in the range of 1 - 10, preferably r is an integer in the range of 1 - 4, most preferably r = 1 or 2.

The amount of payloads D attached to a single biomolecule (typically antibody) is known in the art as the DAR (drug-to-antibody ratio). The bioconjugates according to the invention have a DAR of (x × y × r). In the context of the present invention, it is preferred that the DAR is an integer in the range 1 - 8, more preferably the DAR is 2 or 4, most preferably the DAR is 2. With preferred values for x of 1, for y of 2 and for r of 1 or 2, the DAR is most preferably 2 or 4. It will be appreciated that these are theoretical DAR values, and in practice the DAR may slightly deviate from this value, by virtue of incomplete conjugation. Typically, the conjugates are obtained as a stochastic mixture of antibody-drug conjugates, with DAR values varying between individual conjugates, and depending on the conjugation technique used the DAR may have a broad distribution (e.g. DAR = 0 - 10) or a narrow distribution (e.g. DAR = 3 - 4). In case of such mixtures, DAR often refers to the average DAR of the mixture. This is well-known in the art of bioconjugation. However, in case the conjugation occurs via the glycan, the conjugates according to the invention have a close-to-theoretical DAR. For example, when the theoretical DAR is 4, DAR values above 3.6 or even above 3.8 are readily obtained, indicating that most antibodies in the reaction mixture have reacted completely and have a DAR of 4.

In one embodiment of the conjugates according to the invention, only the conserved glycosylation site of an antibody is used for conjugation as described below, i.e. y = 2, and the antibody is functionalized with 2 × x × r occurrences of payload D. In an alternative embodiment of the conjugates according to the invention, the conserved glycosylation site and a second glycosylation site are both used for conjugation as described below, i.e. y = 4, and the antibody is functionalized with 4 × x × r occurrences of payload D.

### Linker L⁶

Part of the biomolecule, in particular a glycosylated biomolecule such as an antibody, may be a linker L⁶ that connects the connecting group Z to the peptide part of the antibody. Preferably, the connecting group Z is connected to the antibody via its glycan, represented by L⁶.

Linker L⁶ is preferably present, wherein connecting group Z may be introduced at a specific position of the antibody. This is for example the case for an artificially introduced click probe (nitrone group or (hetero)cycloalkyne group), such as for example using transglutaminase, using sortase or by enzymatic glycan modification (e.g. glycosyltransferase or α-1,3-mannosyl-glycoprotein-2-β-N-acetylglucosaminyl-transferase). For example, a modified sugar residue S(F)₂ may be introduced at the glycan, extending the glycan with one monosaccharide residue S, which introduces two click probes F on the glycan of an antibody. In a most preferred embodiment, conjugation occurs via the glycan of the antibody. The site of conjugation is preferably at the heavy chain of the antibody.

Linker L⁶ may be represented by Z¹-L⁸, wherein Z¹ is a connecting group obtained by the reaction of F³ and F⁴, and L⁸ is a linker that connects the biomolecule B with Z¹. Such a group may be formed by the two-step approach for obtaining the modified biomolecule of structure B(F¹)_{(x × y)}, as described above. L⁶ or L⁸ may also be part of the glycan.

All recombinant antibodies, generated in mammalian host systems, contain the conserved N-glycosylation site at the asparagine residue at or close to position 297 of the heavy chain (Kabat numbering), which is modified by a glycan of the complex type. This naturally occurring glycosylation site of antibodies is preferably used, but other glycosylation sites, including artificially introduced ones, may also be used for the connection of linker L⁶. Thus, in a preferred embodiment, L⁶ is connected to an amino acid of the antibody which is located at a position in the range of 250 - 350 of the heavy chain, preferably in the range of 280 - 310 of the heavy chain, more preferably in the range of 295 - 300 of the heavy chain, most preferably at position 297 of the heavy chain. Using this conserved glycosylation position of the antibody, the obtained conjugates are formed as symmetrical dimers, wherein each half antibody contains one click probe F, and both click probes F will form an arm with one or more payloads. Some antibodies may have a second glycosylation site per half antibody, which is not used as conjugation site in the embodiment where y = 2. The skilled person is able to perform the enzymatic conversions in such a way that only the main glycosylation site is utilized for conjugation. Alternatively, the skilled person is able to perform the enzymatic conversion in such a way that also the second glycosylation site is utilized for conjugation, thereby doubling the DAR of the antibody-drug conjugate. In this embodiment, y = 4 and each half antibody contains two occurrences of click probe F, and all four click probes F will form an arm with one or more payloads.

L⁶ is a linkerthat connects B to connecting group Z, and is preferably represented by-(H)ᵥ-S-(L⁷)_{w'}-, wherein H is a monosaccharide, v is an integer in the range of 0 - 10, S is a sugar or a sugar derivative, w' is 0 or 1 and L⁷ is -N(H)C(O)CH₂-, -N(H)C(O)CF₂- or -CH₂-. In one embodiment, (H)ᵥ is an oligosaccharide (i.e. v = 2 - 10), which may be linear or branched. S may be connected to any monosaccharide of (H)ᵥ. Typically, L⁶ is at least partly formed by the glycan of an antibody.

The -(H)ᵥ- part of L⁶ is the glycan, or part thereof. The -(H)ᵥ- of the glycan thus typically originates from the original antibody. The (H)ᵥ is preferably selected from structures (H1), (H2) and (H3).

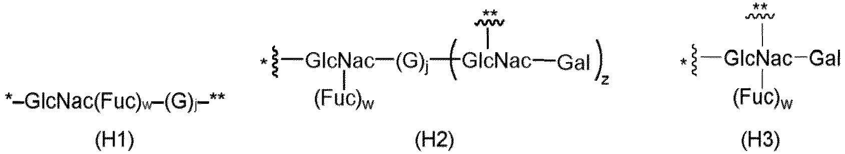

Herein, w is 0 or 1, j is an integer in the range of 0 - 9, GIcNAc is an N-acetylglucosamine moiety, Fuc is a fucose moiety, Gal is a galactose moiety, the (wavy) bond labelled with * represents the connection to the peptide of the antibody and the (wavy) bond labelled with ** represents the connection to S. Fuc is typically bound to GlcNAc via an α-1,6-glycosidic bond. Normally, antibodies may (w = 1) or may not be fucosylated (w = 0). In the context of the present invention, the presence of a fucosyl moiety is irrelevant, and similar effects are obtained with fucosylated (w = 1) and non-fucosylated (w = 0) antibody conjugates. The GIcNAc residue with the bond labelled * may also be referred to as the core-GIcNAc residue and is the monosaccharide that is directly attached to the peptide part of the antibody.

S may be directly connected to the core-GlcNAc(Fuc)_{w} moiety, i.e. j = 0, meaning that the remainder of the glycan is removed from the core-GlcNAc(Fuc)_{w} moiety before S is attached. Such trimming of glycans is well-known in the art and can be achieved by the action of an endoglycosidase. (H3) may be obtained by from a trimmed glycan by introducing a galactose with a galactosyltransferase and introducing S fucose with a fucosyltransferase as described in WO2022037665. Alternatively, there are one or more monosaccharide residues present in between the core-GlcNAc(Fuc)_{w} moiety and S, i.e. j is an integer in the range of 1 - 10, preferably j = 1-5. In one preferred embodiment, (G)ⱼ is an oligosaccharide fraction comprising j monosaccharide residues G, wherein j is an integer in the range of 2 - 5. In another preferred embodiment, (G)ⱼ is a monosaccharide fraction comprising j monosaccharide residues G, wherein j is 1. (G)ⱼ is connected to the GIcNAc moiety of GlcNAc(Fuc)_{w}, typically via a β-1,4 bond. In a preferred embodiment, j is 0, 1, 3, 4 or 5, more preferably, j is 0 or 1, most preferably j is 0.

Although any monosaccharide that may be present in a glycan may be employed as G, each G is preferably individually selected from the group consisting of galactose, glucose, N-acetylgalactosamine, N-acetylglucosamine, mannose and N-acetylneuraminic acid. More preferred options for G are galactose, N-acetylglucosamine and mannose. In case j = 1, it is preferred that G = galactose and S = *N-*acetylneuraminic acid.

When j is 3 - 10, (G)ⱼ may be linear or branched. Preferred examples of branched oligosaccharides (G)ⱼ are (a), (b), (c), (d), (e), (f), (h) and (h) as shown below.

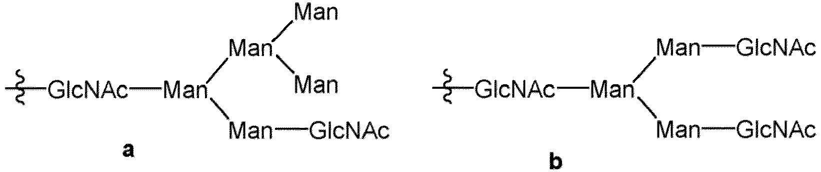

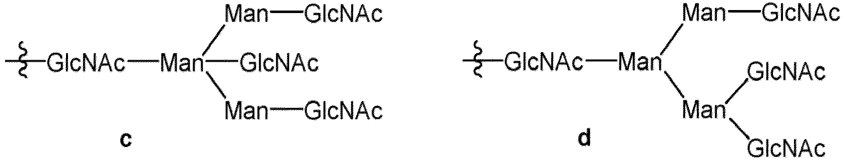

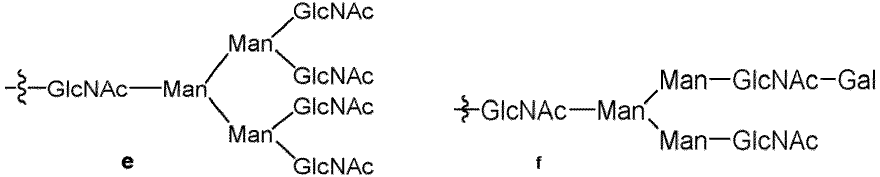

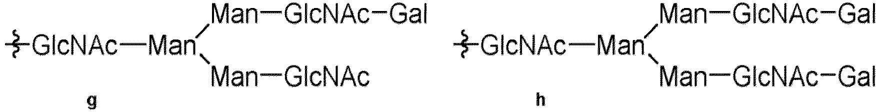

Herein, the wavy lines represent the connection to the core GlcNac(Fuc)_{w}. In case (G)ⱼ is present with j > 2, it is preferred that it ends in GIcNAc or Gal, preferably GlcNAc. In other words, the monosaccharide residue directly connected to S is preferably GIcNAc or Gal. The presence of a GIcNAc moiety facilitates the synthesis of the functionalized antibody, as monosaccharide derivative S can readily be introduced by glycosyltransfer onto a terminal GlcNAc residue. The presence of a Gal moiety facilitates the synthesis of the functionalized antibody, as monosaccharide derivative S sialic acid can readily be introduced by sialyltransferase onto a terminal Gal residue. Alternatively, the presence of a Gal moiety enables the introduction of monosaccharide derivative S fucose by fucosyltransferase onto the adjacent GIcNac as described in WO2022037665. In the above preferred embodiments for (G)ⱼ, having structure **(a)** - **(h),** moiety S may be connected to any of the terminal GlcNAc residues, i.e. not the one with the wavy bond, which is connected to the core GlcNAc residue on the antibody.

Antibodies and antibody conjugates having j = 0 or 1 show no or significantly reduced binding to Fc-gamma receptors, while antibodies and antibody conjugates having j in the range of 4 - 10 do bind to Fc-gamma receptors. Thus, by selecting a certain value for j, the desired extent of binding to Fc-gamma receptors can be obtained. It is thus preferred that j = 0, 1, 4, 5, 6, 7, 8, 9 or 10, more preferably j = 0, 1, 4 or 5, most preferably the antibody is trimmed and j = 0.

S is a sugar or sugar derivative. The term "sugar derivative" is herein used to indicate a derivative of a monosaccharide sugar, i.e. a monosaccharide sugar comprising substituents and/or functional groups. Suitable examples for S include glucose (Glc), galactose (Gal), mannose (Man), fucose (Fuc), amino sugars and sugar acids, e.g. glucosamine (GlcNH₂), galactosamine (GalNH₂) N-acetylglucosamine (GIcNAc), N-acetylgalactosamine (GaINAc), sialic acid (Sia) which is also referred to as N-acetylneuraminic acid (NeuNAc), and N-acetylmuramic acid (MurNAc), glucuronic acid (GlcA) and iduronic acid (IdoA). Preferably, S is selected from Gal, GalNAc and NeuNAc. In an especially preferred embodiment, S is GaINAc.

Connecting group Z may be attached directly to S, or there may be a linker L⁷ present in between S and Z or F¹. Thus, L⁷ may be present (w' = 1 or 2) or absent (w' = 0). Typically, each moiety Z may be connected to S via a linker L⁷, thus in one embodiment w' = 0 or x. Preferably, L⁷ is absent and each connecting moiety Z is directly attached to S. If present, L⁷ may be selected from -N(H)C(O)CH₂-, -N(H)C(O)CF₂- or -CH₂-. In a preferred embodiment, x = 1 and w' = 0 or 1, most preferably x = 1 and w' = 0.

### Connecting group Z

Z is an connecting group, which covalently connects the biomolecule B with the payload(s) D of the conjugate according to the invention. The term "connecting group" herein refers to the structural element, resulting from a reaction, here between nitrone and (hetero)cycloalkyne, connecting one part of the conjugate with another part of the same conjugate. Z is formed by a click reaction between the nitrone group and the (hetero)cycloalkyne group. As will be understood by the person skilled in the art, the exact nature of the connecting group depends on the exact structure of the click probes.

In a preferred embodiment, Z has the structure (Z1): 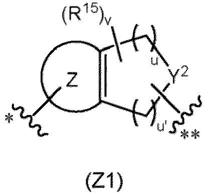 Herein:
- ring Z is (Za) or (Zb) as defined below, wherein the carbon atoms labelled with ** correspond to the two carbon atoms of the double bond in (Z1) to which ring Z is fused;
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y² is C(R³¹)₂, O, S, S⁽⁺⁾R³¹, S(O)R³¹, S(O)=NR³¹ or NR³¹, wherein S⁽⁺⁾ is a cationic sulphur atom counterbalanced by B⁽⁻⁾, wherein B⁽⁻⁾ is an anion, and wherein each R³¹ individually is R¹⁵ or a connection with D, connected via L;
- u is 0, 1, 2, 3, 4 or 5;
- u' is 0, 1, 2, 3, 4 or 5, wherein u + u' = 4, 5, 6, 7 or 8;
- v = an integer in the range 8-16.

In a preferred embodiment, u + u' = 4 or 5. The wavy bonds labelled with * and ** are connected to B, optionally via L⁶, and to L(D)ᵣ. Preferably, the wavy bond labelled with * is connected to B via L⁶, and the wavy bond labelled with ** is connected to L.

It is especially preferred that Z comprises a (hetero)cycloalkene moiety, i.e. the bond depicted as - - - is a double bond. In a preferred embodiment, Z is selected from the structures (Z2) - (Z20c), depicted here below:

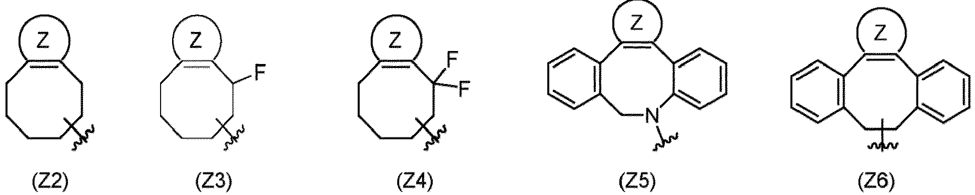

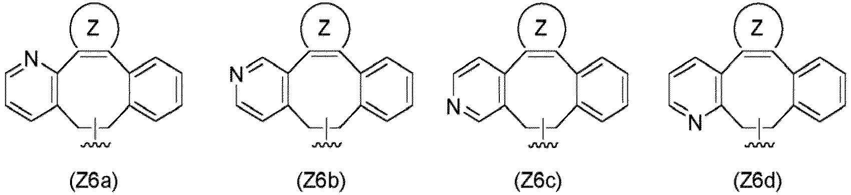

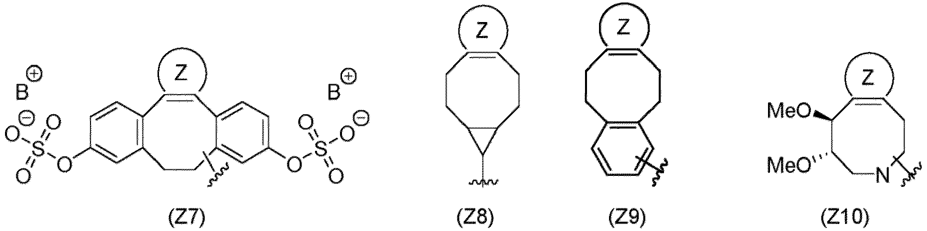

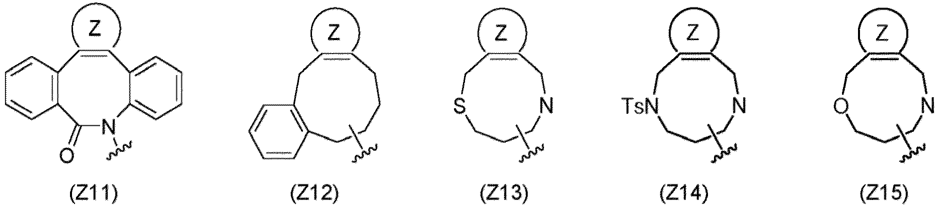

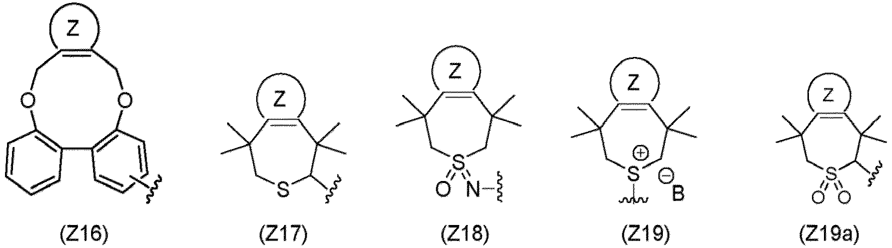

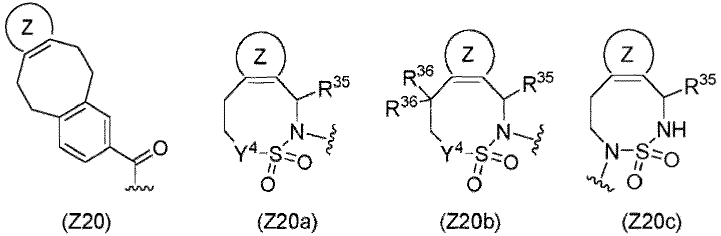

Herein, the connection to L is depicted with the wavy bond. B⁽⁻⁾ is an anion, preferably a pharmaceutically acceptable anion. B⁽⁺⁾ is a cation, preferably a pharmaceutically acceptable cation. R³⁶ is an halogen selected from fluor, chlorine, bromine and iodine, preferably R³⁶ is fluor. Y⁴ is a heteroatom, preferably Y⁴ is O or NH. R³⁵ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, Si, S and NR¹⁴ wherein R¹⁴ is independently selected from the group consisting of hydrogen and C₁ - C₄ alkyl groups, preferably R³⁵ is selected from H, C₅H₁₁, CH₃, CH₂CH₃, CH₂OH or CH₂OTBS.

Ring Z is formed by the cycloaddition reaction and optionally the subsequent rearrangement. Ring Z has a structure (Za) or (Zb) depicted below, wherein the carbon atoms labelled with ** correspond to the two carbon atoms of the (hetero)cycloalkane ring of (Z2) - (Z20c), to which ring Z is fused. In structure (Za), the bond between those two carbon atoms is a double bond, whereas in structure (Zb) this is a single bond.

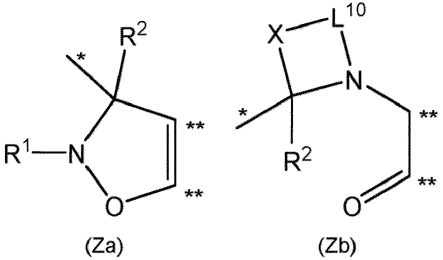

Herein, R¹, R², L¹⁰ and X are as defined above.

In a further preferred embodiment, Z is selected from the structures (Z21) - (Z38d), depicted here below:

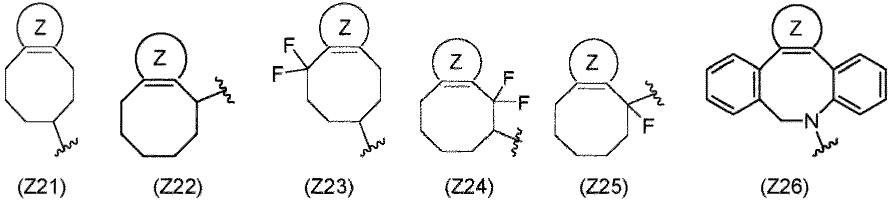

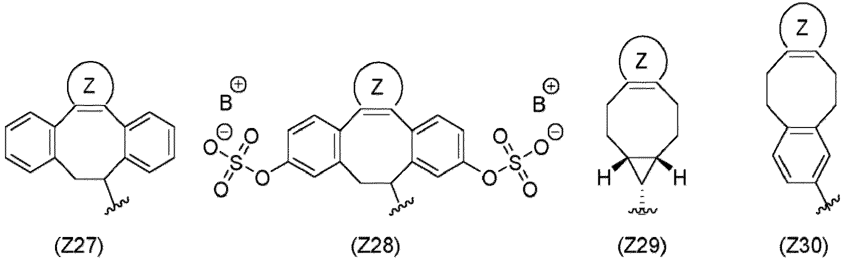

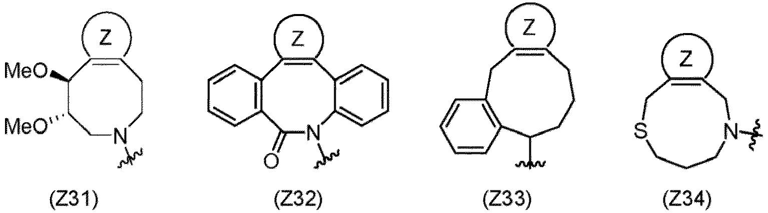

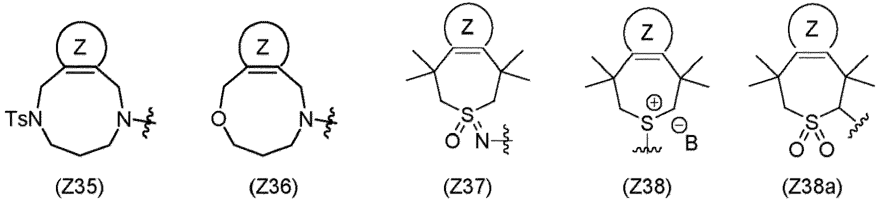

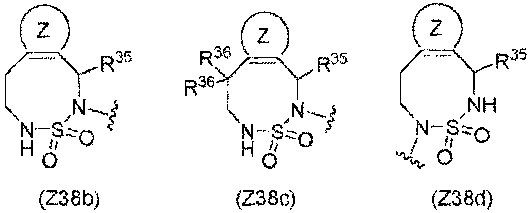

Herein, the connection to L is depicted with the wavy bond. Structure (Z29) can be in endo or exo configuration, preferably it is in endo configuration. In structure (Z38), B⁽⁻⁾ is an anion, preferably a pharmaceutically acceptable anion. In structure (Z28), B⁽⁺⁾ is a cation, preferably a pharmaceutically acceptable cation. Ring Z is has structure (Za) or (Zb), as defined above.

In a preferred embodiment, Z comprises a (hetero)cyclooctene moiety or a (hetero)cycloheptene moiety, preferably according to structure (Z8), (Z26), (Z27), (Z28), (Z37) or (Z38a), which are optionally substituted. Each of these preferred options for Z are further defined here below.

Thus, in a preferred embodiment, Z comprises a heterocycloheptene moiety according to structure (Z37) or (Z38a), which is optionally substituted. Preferably, the heterocycloheptene moiety according to structure (Z37) or (Z38a) is not substituted.

In a preferred embodiment, Z comprises a (hetero)cyclooctene moiety according to structure (Z8), more preferably according to (Z29), which is optionally substituted. Preferably, the cyclooctene moiety according to structure (Z8) or (Z29) is not substituted. In the context of the present embodiment, Z preferably comprises a (hetero)cyclooctene moiety according to structure (Z39) as shown below, wherein V is (CH₂)_{I} and I is an integer in the range of 0 to 10, preferably in the range of 0 to 6. More preferably, I is 0, 1, 2, 3 or 4, more preferably I is 0, 1 or 2 and most preferably I is 0 or 1. In the context of group (Z39), I is most preferably 1. Most preferably, Z is according to structure (Z42), defined further below.

In an alternative preferred embodiment, Z comprises a (hetero)cyclooctene moiety according to structure (Z26), (Z27) or (Z28), which is optionally substituted. In the context of the present embodiment, Z preferably comprises a (hetero)cyclooctene moiety according to structure (Z40) or (Z41) as shown below, wherein Y¹ is O or NR¹¹, wherein R¹¹ is independently selected from the group consisting of hydrogen, a linear or branched C₁ - C₁₂ alkyl group or a C₄ - C₁₂ (hetero)aryl group. The aromatic rings in (Z40) are optionally O-sulfated at one or more positions, whereas the rings of (Z41) may be halogenated at one or more positions. Preferably, the (hetero)cyclooctene moiety according to structure (Z40) or (Z41) is not further substituted. Most preferably, Z is according to structure (Z43), defined further below.

In an alternative preferred embodiment, Z comprises a heterocycloheptenyl group and is according to structure (Z37).

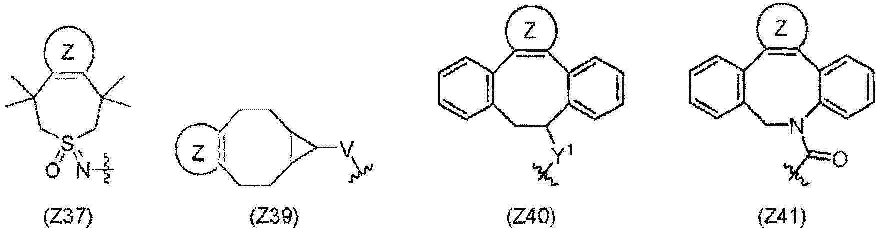

In an especially preferred embodiment, Z comprises a cyclooctenyl group and is according to structure (Z42): 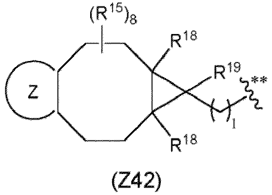 Herein:
- the bond labelled with ** is connected to (L⁶)_{b}B or L(D)ᵣ;
- ring Z is has structure (Za) or (Zb);
- R¹⁵ is independently selected from the group consisting of hydrogen, halogen, -OR¹⁶, -NO₂,
- CN, -S(O)₂R¹⁶, -S(O)₃⁽⁻⁾,C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of D connected via a spacer moiety; and
- I is an integer in the range 0 to 10.

In a preferred embodiment of the group according to structure (Z42), R¹⁵ is independently selected from the group consisting of hydrogen, halogen, -OR¹⁶, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and C₁ - C₆ alkyl, most preferably all R¹⁵ are H. In a preferred embodiment of the group according to structure (Z42), R¹⁸ is independently selected from the group consisting of hydrogen, C₁ - C₆ alkyl groups, most preferably both R¹⁸ are H. In a preferred embodiment of the group according to structure (Z42), R¹⁹ is H. In a preferred embodiment of the group according to structure (Z42), I is 0 or 1, more preferably I is 1.

In an especially preferred embodiment, Z comprises a (hetero)cyclooctenyl group and is according to structure (Z43): 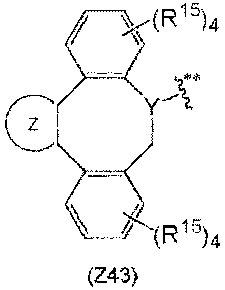 Herein:
- the bond labelled with ** is connected to (L⁶)_{b}B or L(D)ᵣ;
- ring Z is has structure (Za) or (Zb);
- R¹⁵ is independently selected from the group consisting of hydrogen, halogen, -OR¹⁶, -NO₂,
- CN, -S(O)₂R¹⁶, -S(O)₃⁽⁻⁾, C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y is N or CR¹⁵;
- a carbon atom in the fused aromatic rings may be replaced by a nitrogen atom, as in (Z6a) - (Z6d), preferably wherein Y is CR¹⁵.

In a preferred embodiment of the group according to structure (Z43), R¹⁵ is independently selected from the group consisting of hydrogen, halogen, -OR¹⁶, -S(O)₃⁽⁻⁾, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and -S(O)₃⁽⁻⁾. In a preferred embodiment of the group according to structure (Z43), Y is N or CH, more preferably Y = N.

In an especially preferred embodiment, Z comprises a heterocycloheptenyl group and is according to structure (Z37) or (Z38a):

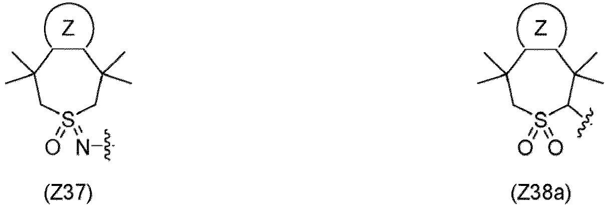

### The linker L

Linker L connects payload D with connecting group Z (in the conjugates according to the invention) or connects payload D with reactive group Q (in the linker-payload constructs). Linkers are known in the art and may be cleavable or non-cleavable.

In a preferred embodiment, linker L is represented by structure

-(L¹)ₙ-(L²)ₒ-(L³)ₚ-(L⁴)_{q}-

wherein:
- (L¹)ₙ is connected to Z and (L⁴)_{q} is connected to D;
- L¹, L², L³ and L⁴ are each individually linkers that together link Z to D;
- n, o, p and q are each individually 0 or 1, wherein n + o + p + q is at least 1. In a preferred embodiment, at least linkers L¹ and L² are present (i.e. n = 1; o = 1; p = 0 or 1), more preferably linkers L¹, L² and L³ are present (i.e. n = 1; o = 1; p = 1).

A linker, especially linker L', may contain one or more branch-points for attachment of multiple payloads to a single connecting group. In a preferred embodiment, the linker of the conjugate according to the invention contains a branching moiety (BM). A "branching moiety" in the context of the present invention refers to a moiety that is embedded in a linker connecting three moieties. In other words, the branching moiety comprises at least three bonds to other moieties, typically one bond connecting to Z or a click probe, one bond to the payload D and one bond to a second payload D. The branching moiety, if present, is preferably embedded in linker L¹, more preferably part of Sp³ or as the nitrogen atom of NR¹³. Any moiety that contains at least three bonds to other moieties is suitable as branching moiety in the context of the present invention.

Suitable branching moieties include a carbon atom (**BM-1**), a nitrogen atom (**BM-3**), a phosphorus atom (phosphine (**BM-5**) and phosphine oxide (**BM-6**)), aromatic rings such as a phenyl ring (e.g. **BM-7**) or a pyridyl ring (e.g. **BM-9**), a (hetero)cycle (e.g. **BM-11** and **BM-12**) and polycyclic moieties (e.g. **BM-13**, **BM-14** and **BM-15**). Preferably, BM is selected from a carbon atom, a nitrogen atom, a phosphorus atom, a (hetero)aromatic ring, a (hetero)cycle or a polycyclic moiety, more preferably BM is a carbon atom or a nitrogen atom. In case BM is a nitrogen atom, the branching nitrogen may be part of an amide group, for example with the connection to Z or a click probe, via the C=O group and the connection to two occurrences of payload D via the two substituents on nitrogen. In case BM is a carbon atom, the carbon atom is preferably part of a trivalent amino acid, such as lysine, aspartic acid or glutamic acid.

Suitable branching moieties BM are selected from structures (**BM-1**) to (**BM-15**) depicted here below, wherein the three branches, i.e. bonds to other moieties as defined above, are indicated by * (a bond labelled with *).

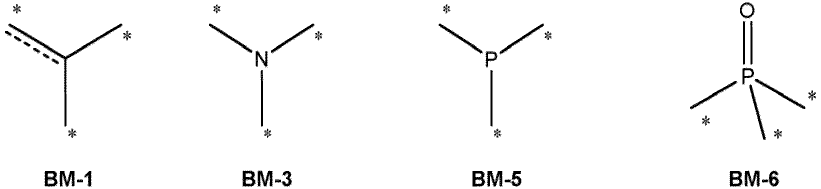

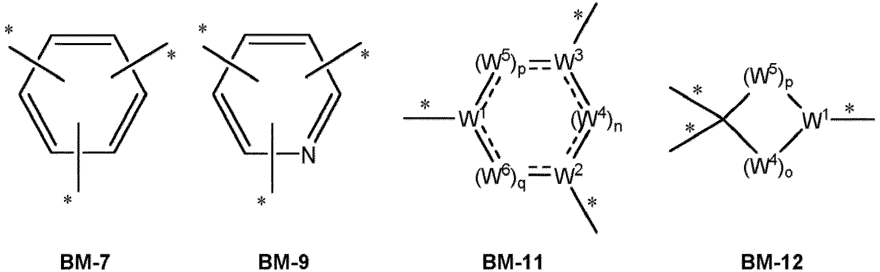

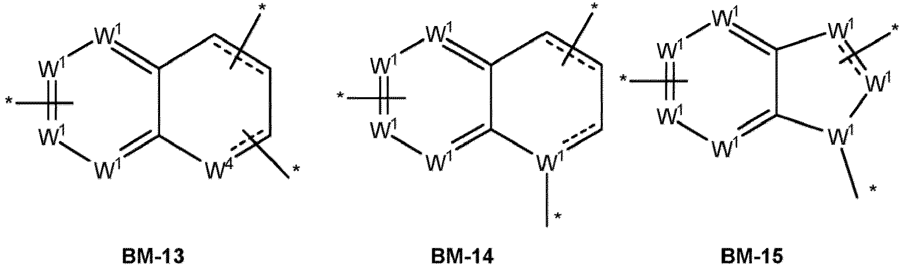

In **(BM-1),** one of the branches labelled with * may be a single or a double bond, indicated with - - - -. In **(BM-11)** to **(BM-15),** the following applies:
- each of n, p, q and q is individually an integer in the range of 0 - 5, preferably 0 or 1, most preferably 1;
- each of W¹, W² and W³ is independently selected from C(R²¹)_{w} and N;
- each of W⁴, W⁵ and W⁶ is independently selected from C(R²¹)_{w+1}, N(R²²)_{w}, O and S;
- each - - - - represents a single or double bond;
- w is 0 or 1 or 2, preferably 0 or 1;
- each R²¹ is independently selected from the group consisting of hydrogen, OH, C₁ - C₂₄ alkyl groups, C₁ - C₂₄ alkoxy groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, wherein the C₁ - C₂₄ alkyl groups, C₁ - C₂₄ alkoxy groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups are optionally substituted and optionally interrupted by one or more heteroatoms selected from O, S and NR³ wherein R³ is independently selected from the group consisting of hydrogen and C₁ - C₄ alkyl groups; and
- each R²² is independently selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, wherein the C₁ - C₂₄ alkyl groups, C₁ - C₂₄ alkoxy groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups are optionally substituted and optionally interrupted by one or more heteroatoms selected from O, S and NR³ wherein R³ is independently selected from the group consisting of hydrogen and C₁ - C₄ alkyl groups.

The skilled person appreciates that the values of w and the bond order of the bonds represented by - - - - are interdependent. Thus, whenever an occurrence of W is bonded to an endocyclic double bond, w = 1 for that occurrence of W, while whenever an occurrence of W is bonded to two endocyclic single bonds, w = 0 for that occurrence of W. For **BM-12,** at least one of o and p is not 0.

Representative examples of branching moieties according to structure **(BM-11)** and **(BM-12)** include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, cyclooctenyl, aziridine, azetidine, diazetidine, oxetane, thietane, pyrrolidine, dihydropyrrolyl, tetrahydrofuranyl, dihydrofuranyl, thiolanyl, imidazolinyl, pyrazolidinyl, oxazolidinyl, isoxazolidinyl, thiazolidinyl, isothiazolidinyl, dioxolanyl, dithiolanyl, piperidinyl, oxanyl, thianyl, piperazinyl, morpholino, thiomorpholino, dioxanyl, trioxanyl, dithyanyl, trithianyl, azepanyl, oxepanyl and thiepanyl. Preferred cyclic moieties for use as branching moiety include cyclopropenyl, cyclohexyl, oxanyl (tetrahydropyran) and dioxanyl. The substitution pattern of the three branches determines whether the branching moiety is of structure **(BM-11)** or of structure **(BM-12)**.

Representative examples of branching moieties according to structure **(BM-13)** to **(BM-15)** include decalin, tetralin, dialin, naphthalene, indene, indane, isoindene, indole, isoindole, indoline, isoindoline, and the like.

In a preferred embodiment, BM is a carbon atom. In case the carbon atom is according to structure (**BM-1**) and has all four bonds to distinct moieties, the carbon atom is chiral. The stereochemistry of the carbon atom is not crucial for the present invention, and may be *S* or *R*. The same holds for the phosphine (**BM-6**). Most preferably, the carbon atom is according to structure (**BM-1**). One of the branches indicated with * in the carbon atom according to structure (**BM-1**) may be a double bond, in which case the carbon atom may be part of an alkene or imine. In case BM is a carbon atom, the carbon atom may be part of a larger functional group, such as an acetal, a ketal, a hemiketal, an orthoester, an orthocarbonate ester, an amino acid and the like. Preferred amino acids in this respect are Asp, Gly, Lys and iGlu. This also holds in case BM is a nitrogen or phosphorus atom, in which case it may be part of an amide, an imide, an imine, a phosphine oxide (as in **BM-6**) or a phosphotriester.

In a preferred embodiment, BM is a phenyl ring. Most preferably, the phenyl ring is according to structure (**BM-7**). The substitution pattern of the phenyl ring may be of any regiochemistry, such as 1,2,3-substituted phenyl rings, 1,2,4-substituted phenyl rings, or 1,3,5-substituted phenyl rings. To allow optimal flexibility and conformational freedom, it is preferred that the phenyl ring is according to structure (**BM-7**), most preferably the phenyl ring is 1,3,5-substituted. The same holds for the pyridine ring of (**BM-9**).

In the preferred structures for BM defined above, BM typically contains three connection points. The skilled person is capable to determine corresponding BMs with four or five connection points. For example **(BM-1)** and **(BM-7)** - **(BM-15)** would also be suitable as BM with more connection points. Alternatively, the linker may contain more than one BM to create four or five connection points.

In a preferred embodiment, the branching moiety BM is selected from a carbon atom, a nitrogen atom, a phosphorus atom, a (hetero)aromatic ring, a (hetero)cycle or a polycyclic moiety, more preferably a carbon atom or a nitrogen atom.

### Linker L ¹

Linker L¹ is either absent (n = 0) or present (n = 1). Preferably, linker L¹ is present and n = 1. L¹ may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S(O)_{y'} and NR²¹, wherein y' is 0, 1 or 2, preferably y' = 2, and R²¹ is independently selected from the group consisting of hydrogen, halogen, C₁ - C₂₄ alkyl groups, C₆ - C₂₄ (hetero)aryl groups, C₇ - C₂₄ alkyl (hetero) aryl groups and C₇ - C₂₄ (hetero)arylalkyl groups. In one embodiment, the optional substituents may be selected from polar groups, such as oxo groups, (poly)ethylene glycol diamines, (poly)ethylene glycol or (poly)ethylene oxide chains, (poly)propylene glycol or (poly)propylene oxide chains, carboxylic acid groups, carbonate groups, carbamate groups, cyclodextrins, crown ethers, saccharides (e.g. monosaccharides, oligosaccharides), phosphates or esters thereof, phosphonic acid or ester, phosphinic acid or ester, sulfoxides, sulfones, sulfonic acid or ester, sulfinic acid, or sulfenic acid.

In a preferred embodiment, linker L¹ contains a polar group, which may also be present in the chain of L¹. Such a polar group may be selected from (poly)ethylene glycol diamines (e.g. 1,8-diamino-3,6-dioxaoctane or equivalents comprising longer ethylene glycol chains), (poly)ethylene glycol or (poly)ethylene oxide chains, (poly)propylene glycol or (poly)propylene oxide chains and 1,x'-diaminoalkanes (wherein x' is the number of carbon atoms in the alkane, preferably x' = 1 - 10), -(O)ₐ-C(O)-NH-S(O)₂-NR¹³- (as further defined below, see structure (**23**)), -C(S(O)₃⁽⁻⁾)-, -C(C(O)₂⁽⁻⁾)-, -S(O)₂-, -P(O)₂⁽⁻⁾-, -O(CH₂CH₂O)ₜ-, -NR³⁰(CH₂CH₂NR³⁰)ₜ-, and the following two structures:

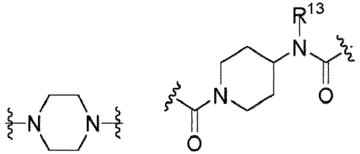

For the polar groups defined here above, it is irrelevant which end is connected to Z and which end to (L²)ₒ.

The polar group may also contain an amino acid including an unnatural amino acid, preferably selected from Arg, Glu, Asp, Ser, Thr or cysteic acid. Herein, R¹³ is further defined below for structure (23). t is an integer in the range of integer in the range of 0 - 15, preferably 1-10, more preferably 2-5, most preferably t = 2 or 4. Each R³⁰ is individually H, C₁₋₁₂ alkyl, C₁₋₁₂ aryl, C₁₋₁₂ alkaryl or C₁₋₁₂ aralkyl. Linker L¹ may contain more than one such polar group, such as at least two polar groups. The polar group may also be present in a branch of linker L¹, which branches off a branching moiety as defined elsewhere. In the context of L¹, a nitrogen or carbon atom is preferably used as branching moiety. It is especially preferred to have a -O(CH₂CH₂O)ₜ- polar group present in a branch.

In a preferred embodiment, Linker L¹ is or comprises a sulfamide group, preferably a sulfamide group according to structure (**23**):

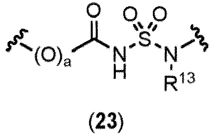

The wavy lines represent the connection to the remainder of the compound, typically to Z, and to L², L³, L⁴ or D. Preferably, the (O)ₐC(O) moiety is connected to Z and the NR¹³ moiety to L², L³, L⁴ or D, preferably to L²

In structure (**23**), a = 0 or 1, preferably a = 1, and R¹³ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups. Alternatively, R¹³ is D connected to N optionally via a spacer moiety, preferably via Sp² as defined below, in one embodiment D is connected to N via -(B)ₑ-(A)_{f}-(B)_{g}-C(O)-. Alternatively, R¹³ is connected to elsewhere in the linker, optionally via a spacer moiety, to form a cyclic structure. For example, R¹³ may be connected to the linker via a CH₂CH₂ spacer moiety to form a piperazinyl ring, where the connection to D is via the second nitrogen of the piperazinyl ring.

In a preferred embodiment, R¹³ is hydrogen, a C₁ - C₂₀ alkyl group, preferably a C₁-- C₁₆ alkyl group, more preferably a C₁ - C₁₀ alkyl group, or connected to a further occurrence of D or to elsewhere in the linker optionally via a spacer moiety. Herein, the alkyl group is optionally substituted and optionally interrupted by one or more heteroatoms selected from O, S and NR¹⁴, preferably O, wherein R¹⁴ is independently selected from the group consisting of hydrogen and C₁ - C₄ alkyl groups. In another preferred embodiment, R¹³ is a C₁ - C₂₀ alkyl group, more preferably a C₁ -C₁₆ alkyl group, even more preferably a C₁ - C₁₀ alkyl group, wherein the alkyl group is optionally interrupted by one or more O-atoms, and wherein the alkyl group is optionally substituted with an -OH group, preferably a terminal -OH group. In this embodiment it is further preferred that R¹³ is a (poly)ethylene glycol chain comprising a terminal -OH group. In another preferred embodiment, R¹³ is selected from the group consisting of hydrogen, methyl, ethyl, n-propyl, i-propyl, n-butyl, s-butyl and t-butyl, or connected to a further occurrence of D or to elsewhere in the linker optionally via a spacer moiety, more preferably from the group consisting of hydrogen, methyl, ethyl, n-propyl and i-propyl, or connected to a further occurrence of D or to elsewhere in the linker optionally via a spacer moiety, and even more preferably from the group consisting of hydrogen, methyl and ethyl, or connected to a further occurrence of D or to elsewhere in the linker optionally via a spacer moiety. Yet even more preferably, R¹³ is hydrogen or connected to a further occurrence of D or to elsewhere in the linker optionally via a spacer moiety, and most preferably R¹³ is hydrogen.

In a preferred embodiment, L¹ is according to structure (**24**):

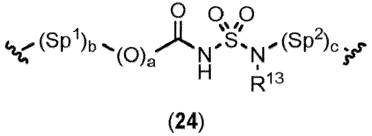

Herein, a and R¹³ are as defined above, Sp¹ and Sp² are independently spacer moieties and b and c are independently 0 or 1. Preferably, b = 0 or 1 and c = 1, more preferably b = 0 and c = 1. In one embodiment, spacers Sp¹ and Sp² are independently selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups and C₉-C₂₀₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂- C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted. When the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups are interrupted by one or more heteroatoms as defined above, it is preferred that said groups are interrupted by one or more O-atoms, and/or by one or more S-S groups

More preferably, spacer moieties Sp¹ and Sp², if present, are independently selected from the group consisting of linear or branched C₁-C₁₀₀ alkylene groups, C₂-C₁₀₀ alkenylene groups, C₂-C₁₀₀ alkynylene groups, C₃-C₁₀₀ cycloalkylene groups, C₅-C₁₀₀ cycloalkenylene groups, C₈-C₁₀₀ cycloalkynylene groups, C₇-C₁₀₀ alkylarylene groups, C₇-C₁₀₀ arylalkylene groups, C₈-C₁₀₀ arylalkenylene groups and C₉-C₁₀₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted.

Even more preferably, spacer moieties Sp¹ and Sp², if present, are independently selected from the group consisting of linear or branched C₁-C₅₀ alkylene groups, C₂-C₅₀ alkenylene groups, C₂-C₅₀ alkynylene groups, C₃-C₅₀ cycloalkylene groups, C₅-C₅₀ cycloalkenylene groups, C₈-C₅₀ cycloalkynylene groups, C₇-C₅₀ alkylarylene groups, C₇-C₅₀ arylalkylene groups, C₈-C₅₀ arylalkenylene groups and C₉-C₅₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted.

Yet even more preferably, spacer moieties Sp¹ and Sp², if present, are independently selected from the group consisting of linear or branched C₁-C₂₀ alkylene groups, C₂-C₂₀ alkenylene groups, C₂-C₂₀ alkynylene groups, C₃-C₂₀ cycloalkylene groups, C₅-C₂₀ cycloalkenylene groups, Ca-C₂₀ cycloalkynylene groups, C₇-C₂₀ alkylarylene groups, C₇-C₂₀ arylalkylene groups, C₈-C₂₀ arylalkenylene groups and C₉-C₂₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted.

In these preferred embodiments it is further preferred that the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups are unsubstituted and optionally interrupted by one or more heteroatoms selected from the group of O, S and NR¹⁶, preferably O, wherein R¹⁶ is independently selected from the group consisting of hydrogen and C₁ - C₄ alkyl groups, preferably hydrogen or methyl.

Most preferably, spacer moieties Sp¹ and Sp², if present, are independently selected from the group consisting of linear or branched C₁-C₂₀ alkylene groups, the alkylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted. In this embodiment, it is further preferred that the alkylene groups are unsubstituted and optionally interrupted by one or more heteroatoms selected from the group of O, S and NR¹⁶, preferably O and/or S-S, wherein R¹⁶ is independently selected from the group consisting of hydrogen and C₁ - C₄ alkyl groups, preferably hydrogen or methyl.

Preferred spacer moieties Sp¹ and Sp² thus include -(CH₂)ᵣ-, -(CH₂CH₂)ᵣ-, -(CH₂CH₂O)ᵣ-, -(OCH₂CH₂)ᵣ-, -(CH₂CH₂O)ᵣCH₂CH₂-, -CH₂CH₂(OCH₂CH₂)ᵣ-, -(CH₂CH₂CH₂O)ᵣ-, -(OCH₂CH₂CH₂)ᵣ-, -(CH₂CH₂CH₂O)ᵣCH₂CH₂CH₂- and -CH₂CH₂CH₂(OCH₂CH₂CH₂)ᵣ-, wherein r is an integer in the range of 1 to 50, preferably in the range of 1 to 40, more preferably in the range of 1 to 30, even more preferably in the range of 1 to 20 and yet even more preferably in the range of 1 to 15. More preferably n is 1, 2, 3, 4, 5, 6, 7, 8, 9 or 10, more preferably 1, 2, 3, 4, 5, 6, 7 or 8, even more preferably 1, 2, 3, 4, 5 or 6, yet even more preferably 1, 2, 3 or 4.

Alternatively, preferred linkers L¹ may be represented by -(W)ₖ-(A)_{d}-(B)ₑ-(A)_{f}-(C(O))_{g}- or [-(W)ₖ-(A)_{d}-(B)ₑ-(A)ₜ-]₂BM-(C(O))_{g}- (A)_{d'}-(B)_{e'}-(A)_{f'}-(C(O))_{g'}-, wherein:
- d and d' are individually 0 or 1,
- e and e' are individually an integer in the range 1 - 10,
- f and f' are individually 0 or 1,
- g and g' are individually an integer in the range 0 - 10,
- k = 0 or 1 with the proviso that if k = 1 then d = 0,
- A is a sulfamide group according to structure (**23**) 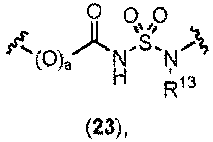
- B is a -CH₂-CH₂-O- or a -O-CH₂-CH₂- moiety, or (B)ₑ is a -(CH₂-CH₂-O)ₑ₁-CH₂-CH₂- or a -(CH₂-CH₂-O)ₑ₁-CH₂- moiety, wherein e1 is defined the same way as e;
- W is -OC(O)-, -C(O)O-, -C(O)NH-, -NHC(O)-, -OC(O)NH-, -NHC(O)O-, -C(O)(CH₂)ₘC(O)-, -C(O)(CH₂)ₘC(O)NH- or -(4-Ph)CH₂NHC(O)(CH₂)ₘC(O)NH-, preferably wherein W is -OC(O)NH-, -C(O)(CH₂)ₘC(O)NH- or -C(O)NH-, and wherein m is an integer in the range 0 - 10, preferably m = 0, 1, 2, 3, 4, 5 or 6, most preferably m = 2 or 3;
- preferably wherein L¹ is connected to Q via (W)ₖ and to L², L³ or D, preferably to L², via (C(O))_{g}, preferably via C(O);
- BM is a branching moiety, preferably selected from a carbon atom, a nitrogen atom, a phosphorus atom, a (hetero)aromatic ring, a (hetero)cycle or a polycyclic moiety, more preferably BM is a nitrogen atom.

In the context of the present embodiment, the wavy lines in structure (**23**) represent the connection to the adjacent groups such as (W)ₖ, (B)ₑ and (C(O))_{g}. It is preferred that A is according to structure (**23**), wherein a = 1 and R¹³ = H or a C₁ - C₂₀ alkyl group, more preferably R¹³ = H or methyl, most preferably R¹³ = H.

Preferred linkers L¹ have structure -(W)ₖ-(A)_{d}-(B)ₑ-(A)_{f}-(C(O))_{g}-, wherein:
(a) k = 0; d = 1; g = 1; f = 0; B = -CH₂-CH₂-O-; e = 1, 2, 3 or 4, preferably e = 2.
(b) k = 1; W = -C(O)(CH₂)ₘC(O)NH-; m = 2; d = 0; (B)ₑ = -(CH₂-CH₂-O)ₑ₁-CH₂-CH₂-; f = 0; g = 1; e1 = 1, 2, 3 or 4, preferably e = 1.
(c) k = 1; W = -OC(O)NH-; d = 0; B = -CH₂-CH₂-O-; g = 1; f = 0; e = 1, 2, 3 or 4, preferably e = 2.
(d) k = 1; W = -C(O)(CH₂)ₘC(O)NH-; m = 2; d = 0; (B)ₑ = -(CH₂-CH₂-O)ₑₗ-CH₂-CH₂-; f = 0; g = 1; e1 = 1, 2, 3 or 4, preferably e1 = 4.
(e) k = 1; W = -OC(O)NH-; d = 0; (B)ₑ = -(CH₂-CH₂-O)ₑ₁-CH₂-CH₂-; g = 1; f = 0; e1 = 1, 2, 3 or 4, preferably e1 = 4.
(f) k = 1; W = -(4-Ph)CH₂NHC(O)(CH₂)ₘC(O)NH-, m = 3; d = 0; (B)ₑ = -(CH₂-CH₂-O)ₑ₁-CH₂-CH₂-; g = 1; f = 0; e1 = 1, 2, 3 or 4, preferably e1 = 4.
(g) k = 0; d = 0; g = 1; f = 0; B = -CH₂-CH₂-O-; e = 1, 2, 3 or 4, preferably e = 2.
(h) k = 1; W = -C(O)NH-; d = 0; g = 1; f = 0; B = -CH₂-CH₂-O-; e = 1, 2, 3 or 4, preferably e = 2.

Herein, it is preferred that when d and/or f = 1, than a = 1 and R¹³ = H. Most preferred is the linker is structure (a).

In a preferred embodiment, linker L¹ comprises a branching moiety as defined above, preferably a branching nitrogen atom, which is located in the backbone between Z and (L²)ₒ and which contains a further moiety Z or click probe as substituent, which is preferably linked to the branching nitrogen atom via a linker. An example of a branching nitrogen atom is the nitrogen atom NR¹³ in structure (**23**), wherein R¹³ is connected to a second occurrence of D via a spacer moiety. Alternatively, a branching nitrogen atoms may be located within L¹ according to structure -(W)ₖ-(A)_{d}-(B)ₑ-(A)_{f}-(C(O))_{g}-. In one embodiment, L¹ is represented by [-(W)ₖ-(A)_{d}-(B)ₑ-(A)_{f}-(C(O))_{g}-]₂ -BM-(A)_{d}-(B)ₑ-(A)_{f}-(C(O))_{g}-, wherein A, B, W, d, e, f, g and k are as defined above and individually selected for each occurrence, and BM is a branching moiety, preferably a branching nitrogen atom, to which two instances of -(A)_{d}-(B)ₑ-(A)_{f}-(C(O))_{g}- are connected. Herein, both (C(O))_{g} moieties are connected to -(L²)ₒ-(L³)ₚ-(L⁴)_{q}-D, wherein L², L³, L⁴, o, p, q and D are as defined above and are each selected individually. In a preferred embodiment, each of L², L³, L⁴, o, p, q and D are the same for both moieties connected to (C(O))_{g}.

Preferred linkers L¹ comprising a branching moiety have structure [-(W)ₖ-(A)_{d}-(B)ₑ-(A)_{f}-(C(O))_{g}-]₂-BM-(A')_{d'}-(B')_{e'}-(A')_{f'}-(C(O))_{g'}- wherein:
(i) k = d = g = e' = 1; f = d' = g' = 0; W = -C(O)-; B = B' = -CH₂-CH₂-O-; A is according to structure (**23**) with a = 0 and R¹³ = H; e = 1, 2, 3 or 4, preferably e = 2.
(j) k = d = g = e' = g' = 1; f = d' = 0; W = -C(O)-; B = B' = -CH₂-CH₂-O-; A is according to structure (**23**) with a = 0 and R¹³ = H; e = 1, 2, 3 or 4, preferably e = 2.

Herein, it is preferred the branching moiety BM is a nitrogen atom.

### Linker L²

Linker L² is a peptide spacer. Linker L² is either absent (o = 0) or present (o = 1). Preferably, linker L² is present and o = 1. The combination of a peptide spacer L² and a cleavable linker L³ is well-known in the art. Linker L² functions as recognition and cleave site for cleaving-enzymes, more preferably the peptides are recognized by specific cleaving enzymes. This allows for cleavage at specific environments in which these cleaving enzymes are expressed such as specific tumors. Since different peptide sequences are cleaved by different enzymes, the L² group also allows customizing the conjugate for specific treatments. The peptide sequences may be cleaved by intracellular enzymes and/or extracellular enzymes, preferably extracellular enzymes. Intracellular enzymes may leak outside of the target cells and are thus capable of cleaving the antibody cytokine conjugate without absorbing the conjugate in a lysosome.

The peptide spacer may also be defined by (NH-CR¹⁷-CO)ₙ, wherein R¹⁷ represents an amino acid side chain as known in the art. Also covered within this definition is proline, which has R¹⁷ joined with the nitrogen atom to form a cyclic moiety. Herein, the amino acid may be a natural or a synthetic amino acid. Preferably, the amino acid(s) are all in their L-configuration. n is an integer in the range of 1 - 5, preferably in the range of 2 - 4. Thus, the peptide spacer contains 1 - 5 amino acids. Preferably, the peptide is a dipeptide (n = 2), tripeptide (n = 3) or tetrapeptide (n = 4), most preferably the peptide spacer is a dipeptide. R¹⁷ represents the amino acid side chain, preferably selected from the side chains of alanine, cysteine, aspartic acid, glutamic acid, phenylalanine, glycine, histidine, isoleucine, lysine, acetyllysine, leucine, methionine, asparagine, pyrrolysine, proline, glutamine, arginine, serine, threonine, selenocysteine, valine, tryptophan, tyrosine and citrulline. Preferred amino acid side chains are those of Val, Cit, Ala, Lys, Arg, AcLys, Phe, Leu, Ile, Trp, Glu, Asp and Asn, more preferably from the side chains of Val, Cit, Ala, Glu and Lys. Alternatively worded, R¹⁷ is preferably selected from CH₃ (Ala), CH₂CH(CH₃)₂ (Leu), CH₂CH₂CH₂NHC(O)NH₂ (Cit), CH₂CH₂CH₂CH₂NH₂ (Lys), CH₂CH₂CH₂NHC(O)CH₃ (AcLys), CH₂CH₂CH₂NHC(=NH)NH₂ (Arg), CH₂Ph (Phe), CH(CH₃)₂ (Val), CH(CH₃)CH₂CH₃ (Ile), CH₂C(O)NH₂ (Asn), CH₂CH₂C(O)OH (Glu), CH₂C(O)OH (Asp) and CH₂(1*H*-indol-3-yl) (Trp). Especially preferred embodiments of R¹⁷ are CH₃ (Ala), CH₂CH₂CH₂NHC(O)NH₂ (Cit), CH₂CH₂CH₂CH₂NH₂ (Lys), CH₂CH₂C(O)OH (Glu) and CH(CH₃)₂ (Val). Most preferably, R¹⁷ is CH₃ (Ala), CH₂CH₂CH₂NHC(O)NH₂ (Cit), CH₂CH₂CH₂CH₂NH₂ (Lys), or CH(CH₃)₂ (Val).

Although any peptide spacer may be used, preferably the peptide spacer is selected from Val-Cit, Val-Ala, Val-Lys, Val-Arg, AcLys-Val-Cit, AcLys-Val-Ala, Glu-Val-Ala, Asp-Val-Ala, iGlu-Val-Ala, Glu-Val-Cit, Glu-Gly-Cit, Glu-Gly-Val, Asp-Val-Cit, iGlu-Val-Cit, Phe-Cit, Phe-Ala, Phe-Lys, Phe-Arg, Ala-Lys, Leu-Cit, Ile-Cit, Trp-Cit, Asn-Asn, Ala-Ala-Asn, Ala-Asn, Asn-Ala, Phe-Phe, Gly, Gly-Gly, Gly-Gly-Gly, Gly-Gly-Gly-Gly, Leu-Gly, Tyr-Gly, Ala-Gly, Pro-Gly, Phe-Gly, Phe-Gly, Ser-Gly, Gly-Phe-Gly, Gly-Gly-Phe-Gly, Gly-Phe-Gly-Gly, Phe-Gly-Gly-Gly, Gly-Gly-Gly-Phe, Phe-Phe-Gly-Gly, Gly-Gly-Phe-Phe, Gly-Gly-Gly-Phe-Gly and Lys, more preferably Val-Cit, Val-Ala, Glu-Val-Ala, Val-Lys, Phe-Cit, Phe-Ala, Phe-Lys, Ala-Ala-Asn, more preferably Glu-Gly-Cit, Val-Cit, Val-Ala, Asn-Asn, Ala-Ala-Asn, Asn-Ala most preferably Glu-Gly-Cit, Val-Cit, Val-Ala or Asn-Ala. Herein, AcLys is e-N-acetyllysine and iGlu is isoglutamate. In one embodiment, L² = Val-Cit. In another embodiment, L² = Val-Ala. In another embodiment, L² = Asn-Ala. In another embodiment, L² = Glu-Gly-Cit.

R¹⁷ represents the amino acid side chain, preferably selected from the side chains of alanine, cysteine, aspartic acid, glutamic acid, phenylalanine, glycine, histidine, isoleucine, lysine, acetyllysine, leucine, methionine, asparagine, pyrrolysine, proline, glutamine, arginine, serine, threonine, selenocysteine, valine, tryptophan, tyrosine and citrulline. Preferred amino acid side chains are those of Val, Cit, Ala, Lys, Arg, AcLys, Phe, Leu, Ile, Trp, Glu, Asp and Asn, more preferably from the side chains of Val, Cit, Ala, Glu and Lys. Alternatively worded, R¹⁷ is preferably selected from CH₃ (Ala), CH₂CH(CH₃)₂ (Leu), CH₂CH₂CH₂NHC(O)NH₂ (Cit), CH₂CH₂CH₂CH₂NH₂ (Lys), CH₂CH₂CH₂NHC(O)CH₃ (AcLys), CH₂CH₂CH₂NHC(=NH)NH₂ (Arg), CH₂Ph (Phe), CH(CH₃)₂ (Val), CH(CH₃)CH₂CH₃ (Ile), CH₂C(O)NH₂ (Asn), CH₂CH₂C(O)OH (Glu), CH₂C(O)OH (Asp) and CH₂(1*H-*indol-3-yl) (Trp). Especially preferred embodiments of R¹⁷ are CH₃ (Ala), CH₂CH₂CH₂NHC(O)NH₂ (Cit), CH₂CH₂CH₂CH₂NH₂ (Lys), CH₂CH₂C(O)OH (Glu) and CH(CH₃)₂ (Val). Most preferably, R¹⁷ is CH₃ (Ala), CH₂CH₂CH₂NHC(O)NH₂ (Cit), CH₂CH₂CH₂CH₂NH₂ (Lys), or CH(CH₃)₂ (Val).

In one embodiment, the amino acid side chain R¹⁷ is substituted with a polar group, preferably selected from oxo groups, (poly)ethylene glycol diamines, (poly)ethylene glycol or (poly)ethylene oxide chains, (poly)propylene glycol or (poly)propylene oxide chains, carboxylic acid groups, carbonate groups, carbamate groups, cyclodextrins, crown ethers, saccharides (e.g. monosaccharides, oligosaccharides), phosphates or esters thereof, phosphonic acid or ester, phosphinic acid or ester, sulfoxides, sulfones, sulfonic acid or ester, sulfinic acid, or sulfenic acid.

In an especially preferred embodiment, L² comprises the peptide spacer represented by general structure (**25**), preferably L² is represented by general structure (**25**):

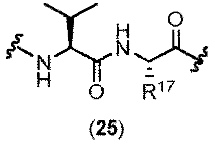

Herein, R¹⁷ is as defined above, preferably R¹⁷ is CH₃ (Ala) or CH₂CH₂CH₂NHC(O)NH₂ (Cit). The wavy lines indicate the connection to (L¹)ₙ and (L³)ₚ, preferably L² according to structure (**25**) is connected to (L¹)ₙ via NH and to (L³)ₚ via C(O).

L² may comprise a certain peptide sequence that is cleavable by specific enzymes. If those enzymes are exclusively expressed or overexpressed in the tumor microenvironment or in the endosomal/lysosomal compartment and leaked to the tumor microenvironment of cancer cells there is an increased probability of targeted release of the cytokine in the tumor and reduced release in healthy tissue.

### Linker L³

Linker L³ is a self-cleavable spacer, also referred to as self-immolative spacer. Linker L³ is either absent (p = 0) or present (p = 1). Preferably, linker L³ is present and p = 1. Cleavage of L² results in 1,6-β elimination in linker L³, resulting in decarboxylation and the release of the payload, D. This advantageously allows for increased probability of release of the payload in regions wherein enzymes that are able to cleave L² are overexpressed. In addition, release of a payload can induce bystander killing which is advantageous for tumours in which not all cancer cells have overexpression of the targeted receptor. Hence, in this embodiment, it is preferred that both linker L² and L³ are present (o = p = 1).

Preferably, L³ is para-aminobenzyloxycarbonyl (PABC) derivative, more preferably a PABC derivative according to structure (L3a):

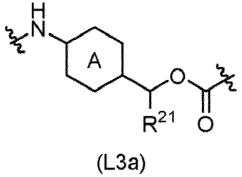

Herein, the wavy lines indicate the connection to L¹ or L², and to L⁴ or D. Typically, the PABC derivative is connected via NH to L¹ or L², preferably to L², and via OC(O) to L⁴ or D.

Ring A is a 5- or 6-membered aromatic or heteroaromatic ring, preferably a 6-membered aromatic or heteroaromatic ring. Suitable 5-membered rings are oxazole, thiazole and furan. Suitable 6-membered rings are phenyl and pyridyl. Ring A may be substituted with a substituent selected from halogen, X²R⁴, N(R⁴)₂, C₁₋₄ alkyl and NO₂. Herein, X² and R⁴ are as defined above, including preferred embodiments thereof. In a preferred embodiment, the optional substituent is selected from F, Cl, Br, OH, OR⁴, SH, NH₂, Et, Me and NO₂. In an especially preferred embodiment, ring A comprises 0 - 2 substituents, more preferably 0 or 1 substituent, most preferably ring A is not substituted. In a preferred embodiment, ring A is 1,4-phenyl, 1,2-phenyl, 2,5-pyridyl or 3,6-pyridyl. Most preferably, A is 1,4-phenyl.

R²¹ is selected from H, R²⁶, C(O)OH and C(O)R²⁶, wherein R²⁶ is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²⁸ wherein R²⁸ is independently selected from the group consisting of hydrogen and C₁ - C₄ alkyl groups. Preferably, R²⁶ is C₃ - C₁₀ (hetero)cycloalkyl or polyalkylene glycol. The polyalkylene glycol is preferably a polyethylene glycol or a polypropylene glycol, more preferably -(CH₂CH₂O)ₛH or -(CH₂CH₂CH₂O)ₛH. The polyalkylene glycol is most preferably a polyethylene glycol, preferably -(CH₂CH₂O)ₛH, wherein s is an integer in the range 1 - 10, preferably 1 - 5, most preferably s = 1, 2, 3 or 4. More preferably, R²¹ is H or C(O)R²⁶, wherein R²⁶ = 4-methyl-piperazine or morpholine. Most preferably, R²¹ is H.

In an alternative embodiment, linker L³ is present (p = 1) and linker L² is not (o = 0) and L³ is a para-glucuronide-meta-amide-benzyloxycarbonyl derivative, preferably a glucuronide derivative according to structure (L3c):

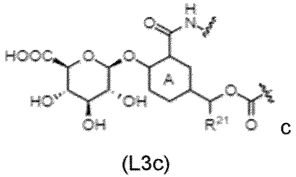

Herein, the wavy lines indicate the connection to L¹, and to L⁴ or D. Typically, the glucuronide derivative is connected via NH to L¹, and via (O)CO to L⁴ or D. Ring A and R²¹ are defined as for the PABC derivative according to structure (L3a). Preferably, ring A is a 6-membered aromatic or heteroaromatic ring, such as oxazole, thiazole, furan, phenyl and pyridyl. In a preferred embodiment, ring A is 1 ,3,4-phenyl, 2,4,5-pyridyl or 2,5,6-pyridyl. Most preferably, A is 1,3,4-phenyl. Preferably, R²¹ is H or C(O)R²⁶, wherein R²⁶ = 4-methyl-piperazine or morpholine. Most preferably, R²¹ is H.

Linker L³ according to structure (L3c) is cleavable by β-glucuronidase, similar to the mechanism in PABC, which results in self-immolation of the para-hydroxybenzyloxy group, decarboxylation and the release of the payload. An ADC comprising the glucuronide derivative according to structure (L3c) is especially useful for treating cancers having an overexpression of β-glucuronidase. β Glucuronidase concentrations in many solid tumours, including lung, breast, and gastrointestinal cancers, as well as in the tumour microenvironment, are reported to be higher than those in normal tissues, and the enzyme is not found in the general circulation. Thus, it is preferred that the conjugates according to the invention comprising the glucuronide derivative according to structure (L3c) are used to treat patients suffering from lung, breast, and gastrointestinal cancers

### Linker L⁴

Linker L⁴ is either absent (q = 0) or present (q = 1). Preferably, linker L⁴ is present and q = 1. Linker L⁴ is selected from:
- an aminoalkanoic acid spacer according to the structure - NR²²-(Cₓ-alkylene)-C(O)-, wherein x is an integer in the range 1 - 20 and R²² is H or C₁ - C₄ alkyl;
- an ethyleneglycol spacer according to the structure -NR²²-(CH₂-CH₂-O)ₑ₆-(CH₂)ₑ₇-C(O)-, wherein e6 is an integer in the range 1 - 10, e7 is an integer in the range 1 - 3 and R²² is H or C₁ - C₄ alky; and
- an amine spacer according to the structure NR²²-(SO₂-NH-)ⱼ(C(O))ₕ-, wherein R²² is H or C₁ - C₄ alkyl, z is an integer in the range 1 - 10, j is 0 or 1, h is 0 or 1..

Linker L⁴ may be an aminoalkanoic acid spacer, i.e. -NR²²-(Cₓ-alkylene)-C(O)-, wherein x is an integer in the range 1 to 20, preferably 1 - 10, most preferably 1 - 6. Herein, the aminoalkanoic acid spacer is typically connected to L³ via the nitrogen atom and to D via the carbonyl moiety. Preferred linkers L⁴ are selected from 6-aminohexanoic acid (Ahx, x= 5), β-alanine (x = 2) and glycine (Gly, x = 1), even more preferably 6-aminohexanoic acid or glycine. In one embodiment, L⁴= 6-aminohexanoic acid. In one embodiment, L⁴= glycine. Herein, R²² is H or C₁ - C₄ alkyl, preferably R²² is H or methyl, most preferably R²² is H.

Alternatively, linker L⁴ may be an ethyleneglycol spacer according to the structure -NR²²-(CH₂-CH₂-O)ₑ₆-(CH₂)ₑ₇-(C(O)-, wherein e6 is an integer in the range 1 - 10, preferably e6 is in the range 2-6, and e7 is an integer in the range 1 - 3, preferably e7 is 2. Herein, R²² is H or C₁ - C₄ alkyl, preferably R²² is H or methyl, most preferably R²² is H.

Alternatively, linker L⁴ may be a diamine spacer according to the structure - NR²²-(Cₓ-alkylene)-NR²²-(C(O))ₕ-, wherein h is 0 or 1, x is an integer in the range 1 - 20, preferably an integer in the range 2-6, even more preferably x = 2 or 5, most preferably x = 2. R²² is H or C₁ - C₄ alkyl. Herein, R²² is H or C₁ -C₄ alkyl, preferably R²² is H or methyl, most preferably R²² is methyl. Herein, h is preferably 1, in which case linker L⁴ is especially suited for conjugation via a phenolic hydroxyl group present on payload D.

### Payload D

D, also referred to in the art as the "payload", represents the compound that is or is to be connected to biomolecule B. Payload molecules are well-known in the art, especially in the field of antibody-drug conjugates, as the moiety that is covalently attached to the antibody and that is released therefrom upon uptake of the conjugate and/or cleavage of the linker. In a preferred embodiment, the payload is selected from the group consisting of an active substance, a reporter molecule, a polymer, a solid surface, a hydrogel, a nanoparticle, a microparticle and a biomolecule. Especially preferred payloads are active substances and reporter molecules, in particular active substances.

The term "active substance" herein relates to a pharmacological and/or biological substance, i.e. a substance that is biologically and/or pharmaceutically active, for example a drug, a prodrug, a cytotoxin, a diagnostic agent, a protein, a peptide, a polypeptide, a peptide tag, an amino acid, a glycan, a lipid, a vitamin, a steroid, a nucleotide, a nucleoside, a polynucleotide, RNA or DNA. Examples of peptide tags include cell-penetrating peptides like human lactoferrin or polyarginine. An example of a glycan is oligomannose. An example of an amino acid is lysine.

When the payload is an active substance, the active substance is preferably selected from the group consisting of drugs and prodrugs. More preferably, the active substance is selected from the group consisting of pharmaceutically active compounds, in particular low to medium molecular weight compounds (e.g. about 200 to about 2500 Da, preferably about 300 to about 1750 Da). In a further preferred embodiment, the active substance is selected from the group consisting of cytotoxins, antiviral agents, antibacterial agents, peptides and oligonucleotides.

Preferred cytotoxins are selected from the groups of nitrogen mustards, nitrosoureas, alkylsulphonates, triazenes, platinum containing compounds, plant alkaloids, DNA topoisomerase inhibitors, anti-metabolites, hormonal therapies, kinase inhibitors, antibiotics, and further cytotoxins defined here below.

Suitable nitrogen mustards include chlorambucil, chlornaphazine, cyclophosphamide, dacarbazine, estramustine, ifosfamide, mechlorethamine, mechlorethamine oxide hydrochloride, mannomustine, mitobronitol, melphalan, mitolactol, pipobroman, novembichin, phenesterine, prednimustine, thiotepa, trofosfamide, uracil mustard; CC-1065 (including its adozelesin, carzelesin and bizelesin synthetic analogues); duocarmycin (including the synthetic analogues, KW-2189 and CBI-TMI); benzodiazepine monomers or dimers (e.g., pyrrolobenzodiazepine (PBD), tomaymycin, indolinobenzodiazepine, isoindolinebenzodiazepine, imidazobenzothiadiazepine and oxazolidinobenzodiazepine).

Suitable nitrosoureas include carmustine, lomustine, chlorozotocin, fotemustine, nimustine and ranimustine.

Suitable alkylsulphonates include busulfan, treosulfan, improsulfan and piposulfan.

Suitable triazenes include dacarbazine.

Suitable platinum containing compounds include carboplatin, cisplatin, oxaliplatin; aziridines, such as benzodopa, carboquone, meturedopa, and uredopa; ethylenimines and methylamelamines including altretamine, triethylenemelamine, trietylenephosphoramide, triethylenethiophosphaoramide and trimethylolomelamine.

Suitable plant alkaloids include vinca alkaloids (e.g. vincristine, vinblastine, vindesine, vinorelbine, navelbin), toxoids (e.g. paclitaxel, docetaxel and their analogs), maytansinoids and their analogs (e.g. DM1, DM2, DM3, DM4, maytansine and ansamitocins), cryptophycins (particularly cryptophycin 1 and cryptophycin 8), epothilones, eleutherobin, discodermolide, bryostatins, dolostatins, auristatins, tubulysins, cephalostatins, pancratistatin, sarcodictyin and spongistatin.

Suitable DNA topoisomerase inhibitors include any camptothecin, including 9-aminocamptothecin, exatecan, DXd (DX-8951 derivative), crisnatol, daunomycin, etoposide, etoposide phosphate, irinotecan, mitoxantrone, novantrone, retinoic acids (retinols), teniposide, topotecan, 9-nitrocamptothecin (RFS 2000); mitomycins and mitomycin C.

Suitable anti-metabolites include anti-folate, DHFR inhibitors (e.g. methotrexate, trimetrexate, denopterin, pteropterin, aminopterin (4-aminopteroic acid) or other folic acid analogues), IMP dehydrogenase inhibitors (e.g. mycophenolic acid, tiazofurin, ribavirin, EICAR), ribonucleotide reductase inhibitors (e.g. hydroxyurea, deferoxamine), pyrimidine analogs (e.g. uracil analogs such as ancitabine, azacitidine, 6-azauridine, capecitabine (Xeloda), carmofur, cytarabine, dideoxyuridine, doxifluridine, enocitabine, 5-fluorouracil, floxuridine, ratitrexed (Tomudex); cytosine analogs such as cytarabine, cytosine arabinoside, fludarabine; purine analogs such as azathioprine, fludarabine, mercaptopurine, thiamiprine, thioguanine), folic acid replenisher (e.g. frolinic acid).

Suitable hormonal therapies include receptor antagonists (e.g. anti-estrogen such as megestrol, raloxifene, tamoxifen, LHRH agonists such as goscrclin, leuprolide acetate; antiandrogens such as bicalutamide, flutamide, calusterone, dromostanolone propionate, epitiostanol, goserelin, leuprolide, mepitiostane, nilutamide, testolactone, trilostane and other androgens inhibitors), retinoids/deltoids (e.g. Vitamin D3 analogs such as CB 1093, EB 1089 KH 1060, cholecalciferol, ergocalciferol), photodynamic therapies (e.g. verteporfin, phthalocyanine, photosensitizer Pc4, demethoxy-hypocrellin A).

Suitable kinase inhibitors include BIBW 2992 (anti-EGFR/Erb2), imatinib, gefitinib, pegaptanib, sorafenib, dasatinib, sunitinib, erlotinib, nilotinib, lapatinib, axitinib, pazopanib, vandetanib, E7080 (anti-VEGFR2), mubritinib, ponatinib (AP24534), bafetinib (INNO-406), bosutinib (SKI-606), cabozantinib, vismodegib, iniparib, ruxolitinib, CYT387, axitinib, tivozanib, sorafenib and ispinesib.

Suitable antibiotics include the enediyne antibiotics (e.g. calicheamicins, especially calicheamicin .γ1, δ1, α1 and β1), dynemicin (e.g. dynemicin A and deoxydynemicin), esperamicin, kedarcidin, C-1027, maduropeptin, as well as neocarzinostatin chromophore and related chromoprotein enediyne antiobiotic chromomophores, aclacinomysins, actinomycin, authramycin, azaserine, bleomycins, cactinomycin, carabicin, carminomycin, carzinophilin; chromomycins, dactinomycin, daunorubicin, nemorubicin, detorubicin, 6-diazo-5-oxo-L-norleucine, doxorubicin, morpholino-doxorubicin, cyanomorpholino-doxorubicin, 2-pyrrolino-doxorubicin and deoxydoxorubicin, epirubicin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin and zorubicin.

Suitable polyketides include acetogenins (in particualr bullatacin and bullatacinone), gemcitabine, epoxomicins (e. g. carfilzomib), bortezomib, thalidomide, lenalidomide, pomalidomide, tosedostat, zybrestat, PLX4032, STA-9090, Stimuvax, allovectin-7, Xegeva and Provenge.

The cytotoxin may further be selected from isoprenylation inhibitors (such as lovastatin), dopaminergic neurotoxins (such as 1-methyl-4-phenylpyridinium ion), cell cycle inhibitors (such as staurosporine), actinomycins (such as actinomycin D and dactinomycin), bleomycins (such as bleomycin A2, bleomycin B2, peplomycin), anthracyclines (e.g. daunorubicin, doxorubicin (adriamycin), idarubicin, epirubicin, pirarubicin, zorubicin, mitoxantrone), MDR inhibitors (e.g. verapamil), Ca2+ ATPase inhibitors (e.g. thapsigargin), histone deacetylase inhibitors (e.g. Vorinostat, Romidepsin, Panobinostat, Valproic acid, Mocetinostat (MGCD0103), Belinostat, PCI-24781, Entinostat, SB939, Resminostat, Givinostat, AR-42, CUDC-101, sulforaphane, Trichostatin A), thapsigargin, Celecoxib, glitazones, epigallocatechin gallate, disulfiram, salinosporamide A.; anti-adrenals (e.g. aminoglutethimide, mitotane, trilostane; aceglatone; aldophosphamide glycoside; aminolevulinic acid; amsacrine; arabinoside, bestrabucil; bisantrene; edatraxate; defofamine; demecolcine; diaziquone; eflornithine (DFMO), elfomithine; elliptinium acetate, etoglucid; gallium nitrate; gacytosine, hydroxyurea; ibandronate, lentinan; lonidamine; mitoguazone; mitoxantrone; mopidamol; nitracrine; pentostatin; phenamet; pirarubicin; podophyllinic acid; 2-ethylhydrazide; procarbazine; PSK^{®}; razoxane; rhizoxin; sizofiran; spirogermanium; tenuazonic acid; triaziquone; 2, 2',2"-trichlorotriethylamine; trichothecenes (especially T-2 toxin, verrucarin A, roridin A and anguidine).

The term "reporter molecule" herein refers to a molecule whose presence is readily detected, for example a diagnostic agent, a dye, a fluorophore, a radioactive isotope label, a contrast agent, a magnetic resonance imaging agent or a mass label.

A wide variety of fluorophores, also referred to as fluorescent probes, is known to a person skilled in the art. Several fluorophores are described in more detail in e.g. G.T. Hermanson, "Bioconjugate Techniques", Elsevier, 3rd Ed. 2013, Chapter 10: "Fluorescent probes", p. 395 - 463, incorporated by reference. Examples of a fluorophore include all kinds of Alexa Fluor (e.g. Alexa Fluor 555), cyanine dyes (e.g. Cy3 or Cy5) and cyanine dye derivatives, coumarin derivatives, fluorescein and fluorescein derivatives, rhodamine and rhodamine derivatives, boron dipyrromethene derivatives, pyrene derivatives, naphthalimide derivatives, phycobiliprotein derivatives (e.g. allophycocyanin), chromomycin, lanthanide chelates and quantum dot nanocrystals.

Examples of a radioactive isotope label include ^{99m}Tc, ¹¹¹In, ^{114m} In, ¹¹⁵In,¹⁸F, ¹⁴C, ⁶⁴Cu, ¹³¹I, ¹²⁵I, ¹²³I, ²¹²Bi, ⁸⁸Y, ⁹⁰Y, ⁶⁷Cu, ¹⁸⁶Rh, ¹⁸⁸Rh, ⁶⁶Ga, ⁶⁷Ga and ¹⁰B, which is optionally connected via a chelating moiety such as e.g. DTPA (diethylenetriaminepentaacetic anhydride), DOTA (1,4,7,10-tetraazacyclododecane-*N,N',N",N"'*-tetraacetic acid), NOTA (1,4,7-triazacyclononane *N,N',N"-*triacetic acid), TETA (1,4,8,11-tetraazacyclotetradecane-*N,N',N",N‴*-tetraacetic acid), DTTA *(N¹*-(*p-*isothiocyanatobenzyl)-diethylenetriamine-*N¹*,*N²,N³,N³*-tetraacetic acid), deferoxamine or DFA (*N'-*[5-[[4-[[5-(acetylhydroxyamino)pentyl]amino]-1,4-dioxobutyl]hydroxyamino]pentyl]-*N*-(5-aminopentyl)-N-hydroxybutanediamide) or HYNIC (hydrazinonicotinamide). Isotopic labelling techniques are known to a person skilled in the art, and are described in more detail in e.g. G.T. Hermanson, "Bioconjugate Techniques", Elsevier, 3rd Ed. 2013, Chapter 12: "Isotopic labelling techniques", p. 507 - 534, incorporated by reference.

Polymers suitable for use as a payload D in the compound according to the invention are known to a person skilled in the art, and several examples are described in more detail in e.g. G.T. Hermanson, "Bioconjugate Techniques", Elsevier, 3rd Ed. 2013, Chapter 18: "PEGylation and synthetic polymer modification", p. 787 - 838, incorporated by reference. When payload D is a polymer, payload D is preferably independently selected from the group consisting of a poly(ethyleneglycol) (PEG), a polyethylene oxide (PEG), a polypropylene glycol (PPG), a polypropylene oxide (PPO), a 1 ,q-diaminoalkane polymer (wherein q is the number of carbon atoms in the alkane, and preferably q is an integer in the range of 2 to 200, preferably 2 to 10), a (poly)ethylene glycol diamine (e.g. 1 ,8-diamino-3,6-dioxaoctane and equivalents comprising longer ethylene glycol chains), a polysaccharide (e.g. dextran), a poly(amino acid) (e.g. a poly(L-lysine)) and a poly(vinyl alcohol).

Solid surfaces suitable for use as a payload D are known to a person skilled in the art. A solid surface is for example a functional surface (e.g. a surface of a nanomaterial, a carbon nanotube, a fullerene or a virus capsid), a metal surface (e.g. a titanium, gold, silver, copper, nickel, tin, rhodium or zinc surface), a metal alloy surface (wherein the alloy is from e.g. aluminum, bismuth, chromium, cobalt, copper, gallium, gold, indium, iron, lead, magnesium, mercury, nickel, potassium, plutonium, rhodium, scandium, silver, sodium, titanium, tin, uranium, zinc and/or zirconium), a polymer surface (wherein the polymer is e.g. polystyrene, polyvinylchloride, polyethylene, polypropylene, poly(dimethylsiloxane) or polymethylmethacrylate, polyacrylamide), a glass surface, a silicone surface, a chromatography support surface (wherein the chromatography support is e.g. a silica support, an agarose support, a cellulose support or an alumina support), etc. When payload D is a solid surface, it is preferred that D is independently selected from the group consisting of a functional surface or a polymer surface.

Hydrogels are known to the person skilled in the art. Hydrogels are water-swollen networks, formed by cross-links between the polymeric constituents. See for example A. S. Hoffman, Adv. Drug Delivery Rev. 2012, 64, 18, incorporated by reference. When the payload is a hydrogel, it is preferred that the hydrogel is composed of poly(ethylene)glycol (PEG) as the polymeric basis.

Micro- and nanoparticles suitable for use as a payload D are known to a person skilled in the art. A variety of suitable micro- and nanoparticles is described in e.g. G.T. Hermanson, "Bioconjugate Techniques", Elsevier, 3rd Ed. 2013, Chapter 14: "Microparticles and nanoparticles", p. 549 - 587, incorporated by reference. The micro- or nanoparticles may be of any shape, e.g. spheres, rods, tubes, cubes, triangles and cones. Preferably, the micro- or nanoparticles are of a spherical shape. The chemical composition of the micro- and nanoparticles may vary. When payload D is a micro- or a nanoparticle, the micro- or nanoparticle is for example a polymeric micro- or nanoparticle, a silica micro- or nanoparticle or a gold micro- or nanoparticle. When the particle is a polymeric micro- or nanoparticle, the polymer is preferably polystyrene or a copolymer of styrene (e.g. a copolymer of styrene and divinylbenzene, butadiene, acrylate and/or vinyltoluene), polymethylmethacrylate (PMMA), polyvinyltoluene, poly(hydroxyethyl methacrylate (pHEMA) or polyethylene glycol dimethacrylate/2-hydroxyethylmetacrylae) [poly(EDGMA/HEMA)]. Optionally, the surface of the micro- or nanoparticles is modified, e.g. with detergents, by graft polymerization of secondary polymers or by covalent attachment of another polymer or of spacer moieties, etc.

Payload D may also be a biomolecule. Biomolecules, and preferred embodiments thereof, are described in more detail below. When payload D is a biomolecule, it is preferred that the biomolecule is selected from the group consisting of proteins (including glycoproteins such as antibodies), polypeptides, peptides, glycans, lipids, nucleic acids, oligonucleotides, polysaccharides, oligosaccharides, enzymes, hormones, amino acids and monosaccharides.

In the context of the present invention, cytotoxic payloads are especially preferred. Thus, D is preferably, a cytotoxin, more preferably selected from the group consisting of colchicine, vinca alkaloids, anthracyclines, camptothecins, doxorubicin, daunorubicin, taxanes, calicheamicins, tubulysins, irinotecans, an inhibitory peptide, amanitins, amatoxins, duocarmycins, epothilones, mytomycins, combretastatins, maytansines, auristatins, enediynes, pyrrolobenzodiazepines (PBDs) or indolinobenzodiazepine dimers (IGN). The present invention is especially suitable for cytotoxic payloads with a moderate cytotoxicity. Normally, conjugates of such payloads need to be administered in a higher dose, but in view of the high DAR (high payload loading) of the conjugates of the present invention, even conjugates of moderately toxic payloads can be administered in a desirably low dose. In other words, D is preferably a payload that enables a moderate to high administered dose in patients (3-20 mg/kg) even at high DAR (DAR >> 6). In view of the high payload loading in the conjugates of the present invention, it is especially preferred that the payload (in unconjugated form) has an average cytotoxicity (IC₅₀) of >50 nM. As such, even payloads with a lower cytotoxicity can be employed in antibody-conjugates with high efficacy.

### Preferred bioconjugates

Preferred conjugates have structure (**3a**), (**4a**), (**5a**) or (**6a**), wherein the 4-isoxazoline is connected to a (hetero)cycloalkenyl group (in structures (**3a**) and (**4a**)), or the rearranged products thereof containing an oxazolidine ring or an 1 ,3-oxazinane ring (in structures (**5a**) and (**6a**)). These conjugates are obtained when (hetereo)cycloalkynyl group is (Q1).

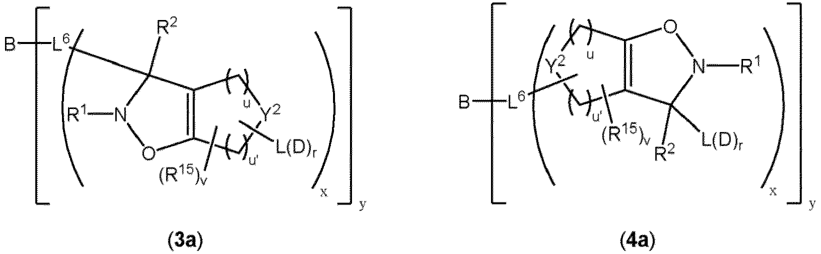

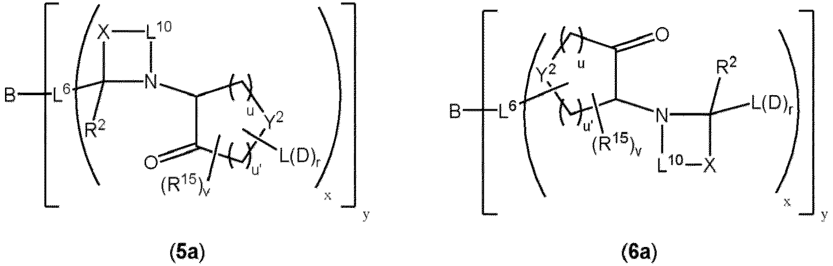

The structural elements B, L⁶, R¹, R², R¹⁵, Y², L, D, X, L¹⁰, u, u', v, x, y and r are defined above, which equally applies to the present embodiment.

Especially preferred conjugates of the above embodiment have a structure selected from (**3b**) - (**6b**), (**3c**) - (**6c**) and (**3d**) - (**6d**) as defined below. Most preferred among those conjugates are (**3b**) - (**6b**). In one embodiment, the nitrone compound was connected to the payload D (R^{2b} = L(D)ᵣ), and the preferred conjugates are according to structure (**4a**) or (**6a**), more preferably selected from structures **(4b), (6b), (4c), (6c), (4d)** and **(6d).** In one embodiment, the nitrone compound was connected to the biomolecule V (R^{2b} = L⁶B), and the preferred conjugates are according to structure **(3a)** or **(5a),** more preferably selected from structures **(3b), (5b), (3c), (5c), (3d)** and **(5d).**

Preferred conjugates have structure **(3b), (4b), (5b)** or **(6b),** wherein the 4-isoxazoline is connected to a (hetero)cyclooctenyl group (in structures **(3b)** and **(4b)),** or the rearranged products thereof containing an oxazolidine ring or an 1 ,3-oxazinane ring (in structures **(5b)** and **(6b)).** These conjugates are obtained when (hetereo)cycloalkynyl group is (Q42).

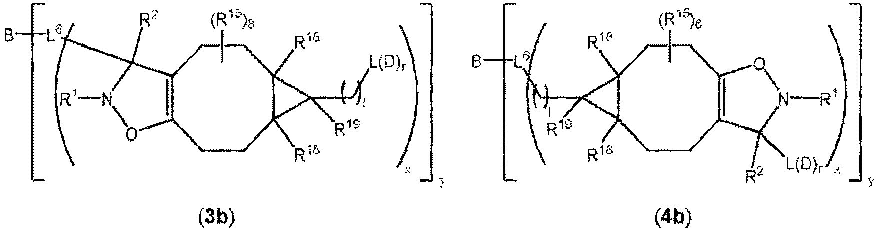

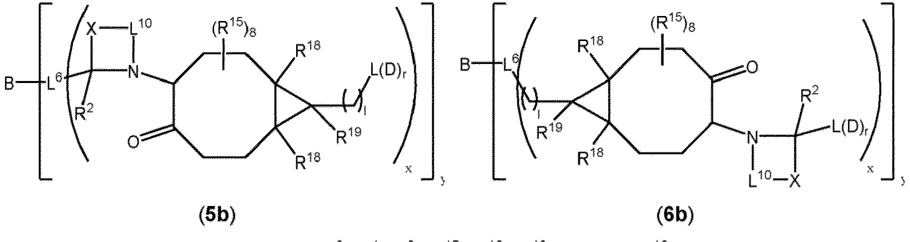

The structural elements B, L⁶, R¹, R², R¹⁵, R¹⁸, R¹⁹, L, D, X, L¹⁰, I, x, y and r are defined above, which equally applies to the present embodiment.

Preferred conjugates have structure **(3c), (4c), (5c)** or **(6c),** wherein the 4-isoxazoline is connected to a (hetero)cyclooctenyl group (in structures **(3c)** and **(4c)),** or the rearranged products thereof containing an oxazolidine ring or an 1 ,3-oxazinane ring (in structures **(5c)** and **(6c)).** These conjugates are obtained when (hetereo)cycloalkynyl group is (Q**43**)**.**

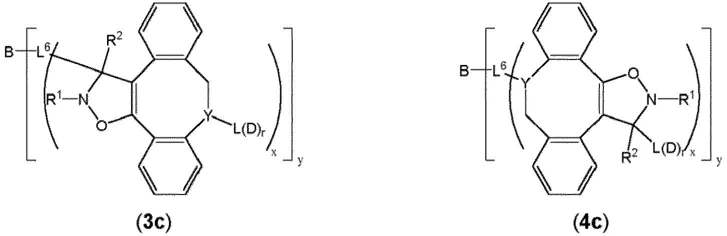

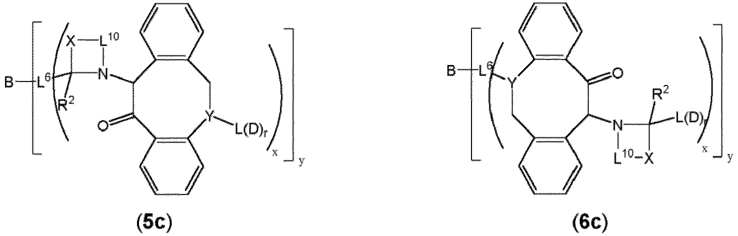

The structural elements B, L⁶, R¹, R², Y, L, D, X, L¹⁰, x, y and r are defined above, which equally applies to the present embodiment. These connecting groups occur in two regioisomers, wherein the relative positioning of the Y-L(D)ᵣ with respect to the isoxazoline oxygen atom (in structures **(3c)** and **(4c)),** of the C=O moiety (in structures **(5c)** and **(6c))** around the 8-membered ring differs. The regioisomers depicted above are not limiting, and the other reiosiomer for each of structure **(3c), (4c), (5c)** and **(6c)** is equally covered by the present embodiment. In other words, the positions of Y-L(D)ᵣ and CH₂ within the 8-membered ring may be reversed.

Preferred conjugates have structure **(3d), (4d), (5d)** or **(6d),** wherein the 4-isoxazoline is connected to a (hetero)cyclooctenyl group (in structures **(3d)** and **(4d)),** or the rearranged products thereof containing an oxazolidine ring or an 1 ,3-oxazinane ring (in structures **(5d)** and **(6d)).** These conjugates are obtained when (hetereo)cycloalkynyl group is (Q37).

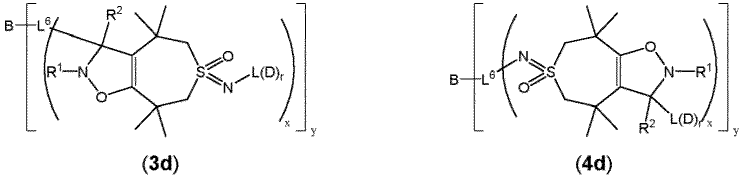

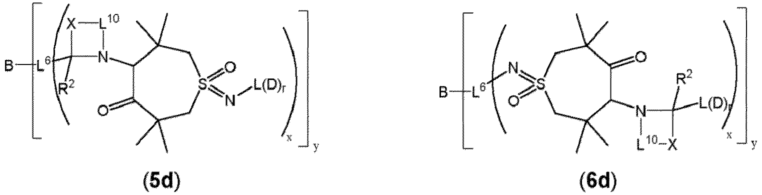

The structural elements B, L⁶, R¹, R², L, D, X, L¹⁰, x, y and r are defined above, which equally applies to the present embodiment.

### Application

The bioconjugates of the present invention are especially suitable in the treatment of diseases, such as cancer, . Herein, it is preferred that the biomolecule is a cell-targeting polypeptide, such as an antibody.

The bioconjugates of the present invention are especially suitable in the treatment of cancer. In that light, the invention further concerns a method for the treatment of cancer, comprising administering to a subject in need thereof the conjugate according to the invention. The subject in need thereof is typically a cancer patient. The use of conjugates, such as antibody-drug conjugates, is well-known in the field of cancer treatment, and the conjugates according to the invention are especially suited in this respect. The method as described is typically suited for the treatment of cancer. In the method according to this aspect, the antibody-conjugate is typically administered in a therapeutically effective dose. A preferred dose for administration of the conjugates according to the invention is in the range of 3 -20 mg per kg bodyweight, every three weeks, or every two weeks, or every week, preferably every three weeks. The present aspect of the invention can also be worded as a conjugate according to the invention for use in the treatment of cancer. In other words, this aspect concerns the use of a conjugate according to the invention for the preparation of a medicament or pharmaceutical composition for use in the treatment of cancer. In the present context, treatment of cancer is envisioned to encompass treating, imaging, diagnosing, preventing the proliferation of, containing and reducing tumours.

This aspect of the present invention may also be worded as a method for targeting a tumour cell expressing a specific extracellular receptor, comprising contacting the conjugate according to the invention with cells that may possibly express the extracellular receptor, and wherein the antibody specifically targets the extracellular receptor. The method according to this aspect is thus suitable to determine whether the cells are expressing the desired extracellular receptor. These tumour cells may be present in a subject, in which case the method comprises administering to a subject in need thereof the conjugate according to the invention. Alternatively, the method occurs *ex vivo* or *in vitro.* In a preferred embodiment, the cells that may possibly express the extracellular receptor are cells that express the extracellular receptor. The targeting of tumour cells preferably includes one or more of treating, imaging, diagnosing, preventing the proliferation of, containing and reducing the tumour cells.

In the context of diagnosis, it is typically unknown whether the cells that are being contacted in fact express the specific extracellular receptor that is being investigated. For example, in the diagnosis of HER2-positive breast cancer, a conjugate containing an antibody that targets HER2, such as trastuzumab, may be contacted with the cells. In case the tumour cells are in fact HER2-expressing, the conjugate will target the cells, while in case the tumour cells are not HER2-expressing, the conjugate will not target the cells. Likewise, in the treatment of a cancer cells specifically expressing an extracellular receptor, the skilled person will understand that a cell-binding agent, such as an antibody, is to be used that targets that specific extracellular receptor.

In the methods of the present invention, it is preferred that the extracellular receptor is selected from the group consisting of 5T4, ADAM-9, AMHRII, ASCT2, ASLG659, ASPHD1, av-integrin, Axl, B7-H3, B7-H4, BAFF-R, BCMA, BMPR1B, brevican, c-KIT, c-Met, C4.4a, CA-IX, cadherin-6, CanAg, CD123, CD13, CD133, CD138/syndecan-1, CD166, CD19, CD20, CD203c, CD205, CD21, CD22, CD228, CD25, CD30, CD324, CD33, CD37, CD38, CD45, CD46, CD48a, CD56, CD70, CD71, CD72, CD74, CD79a, CD79b, CEACAM5, claudin-18.2, claudin-6, CLEC12A, CLL-1, Cripto, CRIPTO, CS1, CXCR5, DLK-1, DLL3, DPEP3, E16, EGFR, ENPP3, EpCAM, EphA2, EphB2R, ETBR, FAP, FcRH1, FcRH2, FcRH5, FGFR2, fibronectin, FLT3, folate receptor alpha (FOL-R1), Gal-3BP, GD3, GDNF-Ra1, GEDA, GFRA1, Globo H, gpNMB, GPR172A, GPR19, GPR54, guanyl cyclase C, HER2, HER3, HLA-DOB, IGF-1R, IL13R, IL20Rα, Lewis Y, LGR5, LIV-1, LRRC15, LY64, Ly6E, Ly6G6D, LY6K, MDP, MFI2, MICA/B, MOSPD2, MPF, MSG783, MUC1, MUC16, NaPi2b, NCA, nectin-4, Notch3, P-cadherin, P2X5, PD-L1, PMEL17, PRLR, PSCA, PSMA, PTK7, RET, RNF43, RON, ROR1, ROR2, SLITRK6, SSTR2, STEAP1, STEAP2, TAG72, TENB2, TF, TIM-1, TM4SF, TMEFF, TMEM118, TMEM46, transferrin, TROP-2, TrpM4, TWEAKR, receptor tyrosine kinases (RTK), tenascin. Likewise, it is preferred that the tumour cells express an extracellular receptor selected from the same group. The skilled person is capable of matching the desired extracellular receptor with a suitable cell-binding agent capable of targeting that extracellular receptor.

The conjugates of the present invention are also especially suitable as antibiotic, antiviral, anti-inflammatory and anti-autoimmune agent. Thus, in an alternative embodiment, the invention concerns a method for the treatment of infection, inflammation of an autoimmune disorder, comprising administering to a subject in need thereof the conjugate according to the invention. In the method according to this aspect, the antibody-conjugate is typically administered in a therapeutically effective dose. The present aspect of the invention can also be worded as a conjugate according to the invention for use in the treatment of infection, inflammation of an autoimmune disorder. In other words, this aspect concerns the use of a conjugate according to the invention for the preparation of a medicament or pharmaceutical composition for use in the treatment of infection, inflammation of an autoimmune disorder. Herein, the infection may be bacterial or viral.

In the present embodiment, the antibody is preferably specific to an extracellular protein resulting from a viral infection and/or tumour-associated carbohydrate antigen. Herein, the extracellular protein resulting from a viral infection may be human polio virus (HPV), human cytomegalovirus (HCMV) or human papillomavirus (HPV). The tumour-associated carbohydrate antigen (TACA) may be selected from the group of Tn, STn, T-antigen, LDN, Lewis^{c} (Le^{c}), Sialyl-Lewis^{c} (SLe^{c}), 6-Sialyl-Lewis^{c} (6SLe^{c}), LN, alpha-Gal, 3SLN, 6SLN, H-antigen, A-antigen, B-antigen, Lewis^{a} (Le^{a}), Sialyl-Lewis^{a} (SLe^{a}), 6-Sialyl-Lewis^{a} (6SLe^{a}), Lewis^{b} (Le^{b}), Sialyl-Lewis^{b} (SLe^{b}), 6-Sialyl-Lewis^{b} (6SLe^{b}), Lewis^{x} (Le^{x}), Sialyl-Lewis^{x} (SLe^{x}), 6-Sialyl-Lewis^{x} (6SLe^{x}), Lewis^{y} (Le^{y}), Sialyl-Lewis^{y} (SLe^{y}), 6-Sialyl-Lewis^{y} (6SLe^{y}) and or combinations thereof. The antibody may also be specific to both an extracellular protein and a TACA at the same time.

In this light, the invention also concerns a pharmaceutical composition comprising the conjugate according to the invention and a pharmaceutically acceptable carrier. The pharmaceutical composition typically contains the conjugate according to the invention in a pharmaceutically effective dose.

### Description of the figures

Figure 1 shows the general scheme for preparation of antibody-drug conjugates by reaction of a monoclonal antibody (in most cases a symmetrical dimer) containing an x number of functionalities F. By incubation of antibody-(F)ₓ with excess of a linker-drug construct (Q-spacer-linker-payload) a conjugate is obtained by reaction of F with Q, forming connecting group Z.
Figure 2 shows the general process for non-genetic conversion of a monoclonal antibody (mAb) into an antibody containing probes for click conjugation (F). The click probe may be on various positions in the antibody, depending on the technology employed. For example, the antibody may be converted into an antibody containing two click probes (structure on the left) or four click probes (bottom structure) or eight probes (structure on the right) for click conjugation.
Figure 3 shows a representative (but not comprehensive) set of functional groups (F) that can be introduced into an antibody by engineering, by chemical modification, or by enzymatic means, which upon metal-free click reaction with a complementary reactive group Q lead to connecting group Z. Functional group F may be artificially introduced (engineered) into an antibody at any position of choice. Some functional groups F (*e*.*g*. nitrile oxide, quinone), may besides strained alkynes also react with strained alkenes, which as an example is depicted for triazine or tetrazine (bottom line). The pyridine or pyridazine connecting group is the product of the rearrangement of the tetrazabicyclo[2.2.2]octane connecting group, formed upon reaction of triazine or tetrazine with alkyne (but not alkene), respectively, with loss of N₂.
Figure 4 shows cyclic alkynes suitable for metal-free click chemistry, and preferred embodiments for reactive moiety Q. The list is not comprehensive, for example alkynes can be further activated by fluorination, by substitution of the aromatic rings or by introduction of heteroatoms in the aromatic ring. Cyclic alkynes Q depicted in Figure 4 are preferred cyclic alkynes to be used in the present invention.

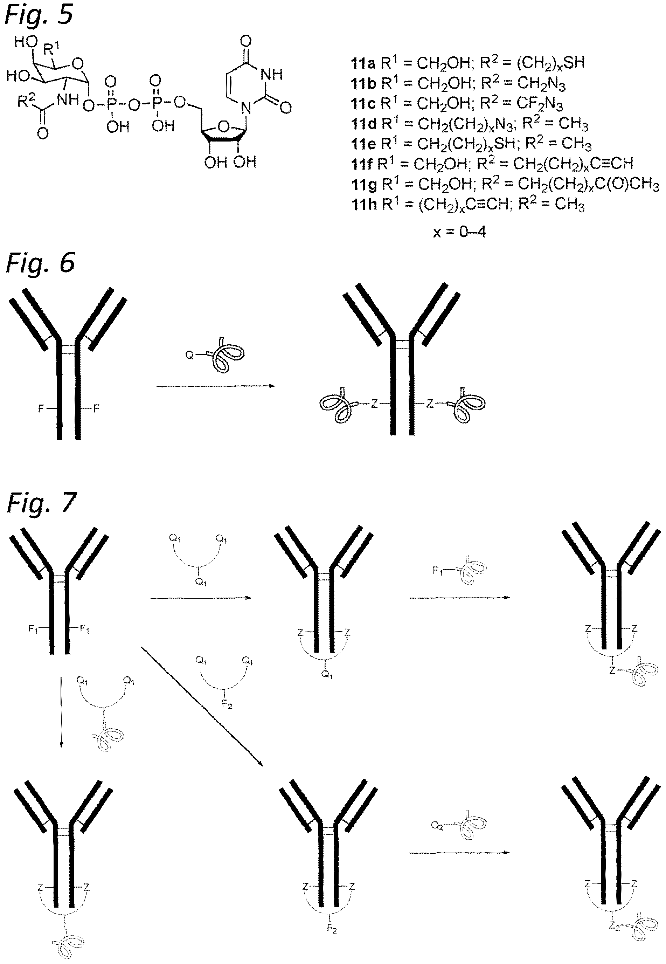

Figure 5 shows several structures of derivatives of UDP sugars of galactosamine, which may be modified with e.g. a 3-mercaptopropionyl group **(11a),** an azidoacetyl group **(11b),** or an azidodifluoroacetyl group (**11c**) at the 2-position, or with an azido group at the 6-position of N-acetyl galactosamine **(11d),** with a thiol group at the 6-position of N-acetyl galactosamine **(11e),** with an alkynyl group at the 2-position **(11f)** or 6-position **(11h**), or with a keto group at the 2-position (**11g**). The monosaccharide (i.e. with UDP removed) are preferred moieties S to be used in the present invention.
Figure 6 depicts how an IgG antibody modified with two click probes (F) can react with a polypeptide modified with the complementary click probe (Q) to form a stable bond (Z) upon reaction. In case the polypeptide is able to bind to an immune cell a bispecific antibody would be formed as such. Modification of the polypeptide with a single click probe Q may be achieved by any selective genetic or non-genetic method. Probes for click conjugation may be elected from any suitable combination depicted in Figure 3. Stoichiometry of the resulting bispecific antibody depends on the number of click probes F installed in the first modification of the antibody.
Figure 7 shows three alternative methods to install a single polypeptide onto a full-length antibody. The full-length antibody therefore has first been modified with two click probes F. In one approach (arrow down), the IgG(F₂) is subjected to a polypeptide that has been modified with two complementary click probes Q, connected via a suitable spacer, both of which will react with one occurrence of F on the antibody. In the second approach (arrow right), the IgG(F₂) is subjected to a trivalent construct containing three complementary probes Q of which two will react with IgG(F₂), leaving one unit of Q free for subsequent reaction with F-modified polypeptide. In the third approach (diagonal arrow), the IgG(F₂) is subjected to a trivalent construct containing two complementary probes Q and one non-reactive click probe F₂ (which is also different from F). The two click probes Q will react with IgG(F₂), leaving F₂ for subsequent reaction with Q₂-modified polypeptide.

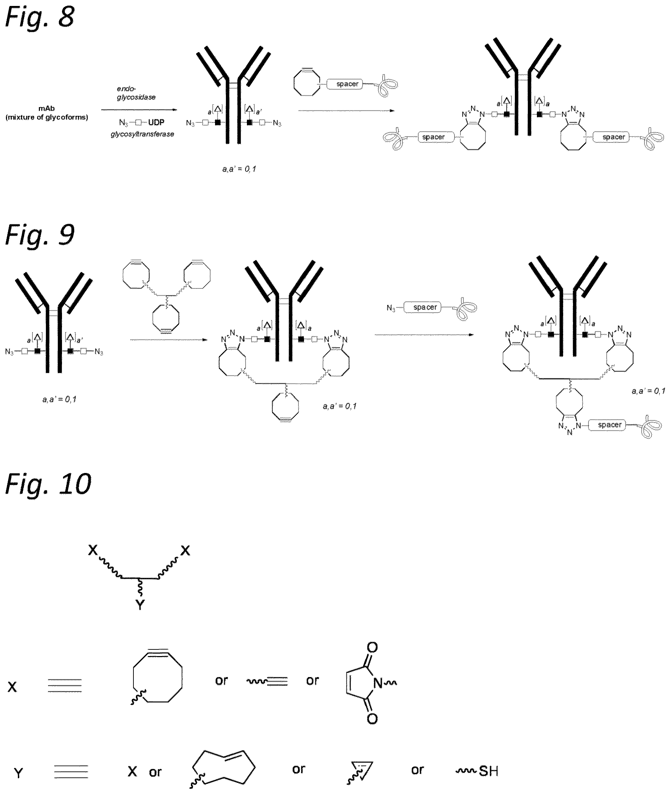

Figure 8 depicts a specific example of forming a bispecific antibody based on glycan remodeling of a full-length IgG and azide-cyclooctyne click chemistry. The IgG is first enzymatically remodeled by endoglycosidase-mediated trimming of all different glycoforms, followed by glycosyltransferase-mediated transfer of azido-sugar onto the core GlcNAc liberated by endoglycosidase. In the next step, the azido-remodeled IgG is subjected to a payload (here a polypeptide), which has been modified with a single cyclooctyne for metal-free click chemistry (SPAAC), leading to a bispecific antibody of 2:2 molecular format. It is also depicted that the cyclooctyne-polypeptide construct will have a specific spacer between cyclooctyne and polypeptide, which enables tailoring of IgG-polypeptide distance or impart other properties onto the resulting bispecific antibody. In the context of the present invention, the cyclooctyne may be subjected to SPANC with an appropriately functionalized antibody, wherein instead of an azide a nitrone group is introduced.
Figure 9 is an illustration of how a azido-sugar remodeled antibody can be converted into a bispecific with a 2:1 molecular format by subjecting first to trivalent cyclooctyne construct suitable for clipping onto bis-azido antibody, leaving one cyclooctyne free for subsequent SPAAC with azido-modified polypeptide, effectively installing only one polypeptide onto the IgG. The latter polypeptide may also be modified with other complement click probes for reaction with cyclooctyne, *e.g*. a tetrazine moiety for inverse electron-demand Diels-Alder cycloaddition. In the context of the present invention, the non-reacted cyclooctyne may be subjected to SPANC with an appropriately functionalized payload, wherein instead of an azide a nitrone group is present.
Figure 10 shows various options for trivalent constructs for reaction with a bis-azidosugar modified mAb. The trivalent construct may be homotrivalent or heterotrivalent (2+1 format). A homotrivalent contract (X = Y) may consist of 3x cyclooctyne or 3x acetylene or 3x maleimide or 3x other thiol-reactive group. A heterotrivalent construct (X # Y) may for example consist of two cyclooctyne groups and one maleimide group or two maleimides groups and one trans-cyclooctene group. The heterotrivalent construct may exist of any combination of X and Y unless X and Y and reactive with each other (e.g. maleimide + thiol). In the context of the present invention, at least one of X and Y may be the cycloalkyne click probe that is capable of a SPANC reaction with a nitrone group.

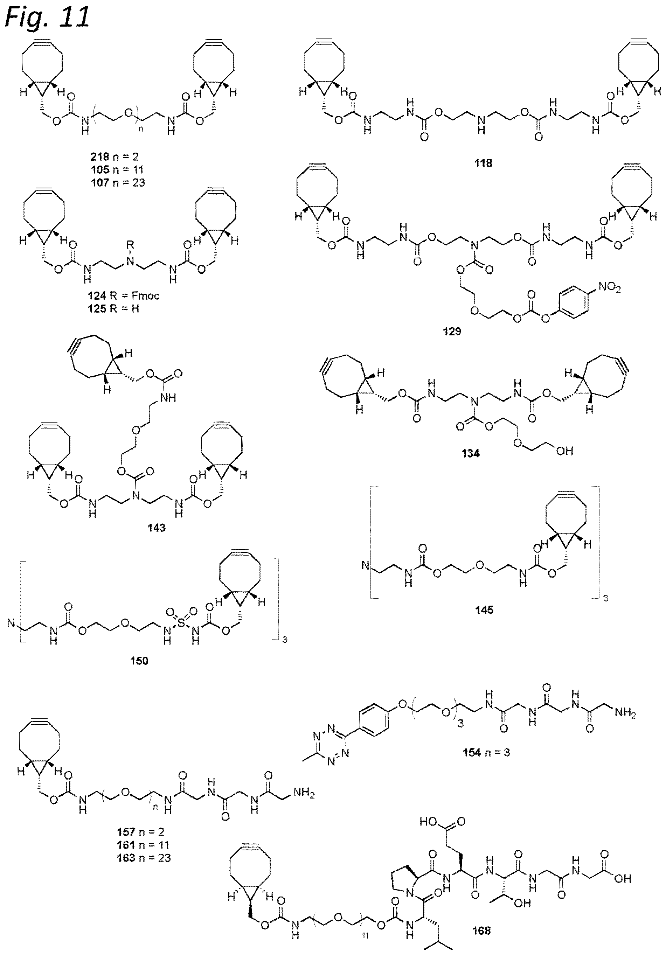

Figure 11 shows a range of bivalent BCN reagents **(105, 107, 118, 125, 129, 134, 218),** trivalent BCN reagents **(143, 145, 150),** and monovalent BCN reagents for sortagging **(154, 157, 161, 163, 168).**

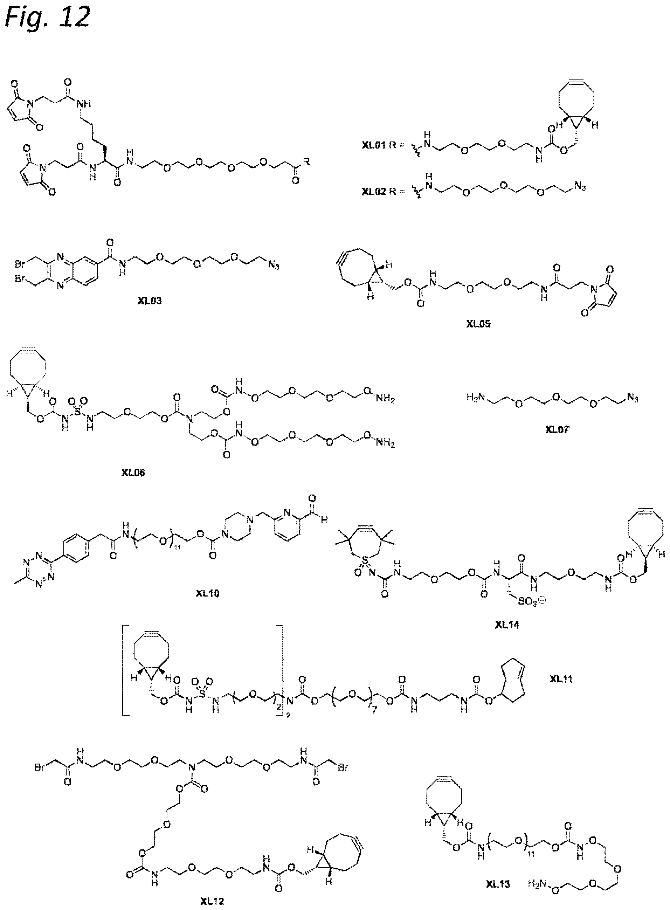

Figure 12 shows a range of bivalent or trivalent cross-linkers **(XL01-XL13).**

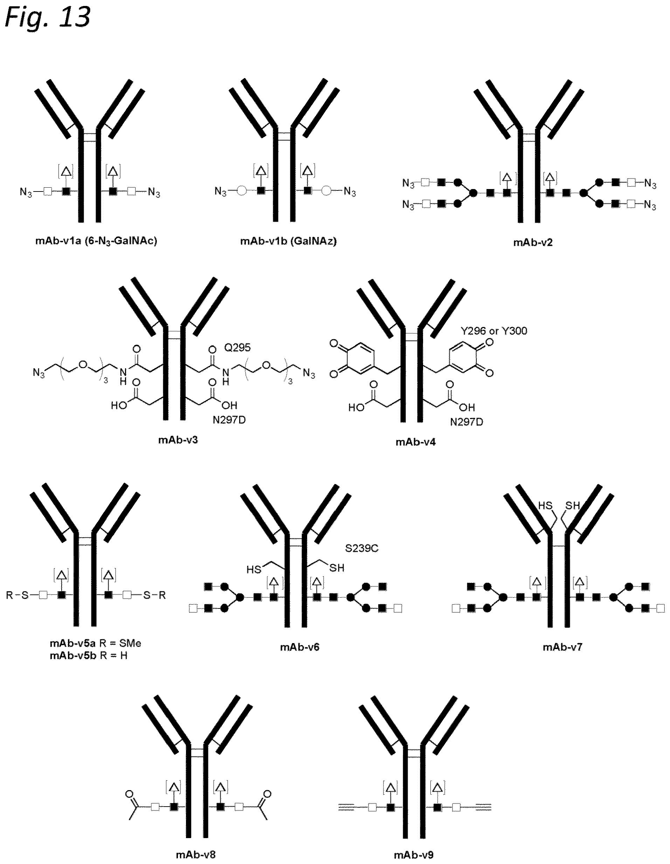

Figure 13 shows a range of antibody variants as starting materials for subsequent conversion to antibody conjugates.

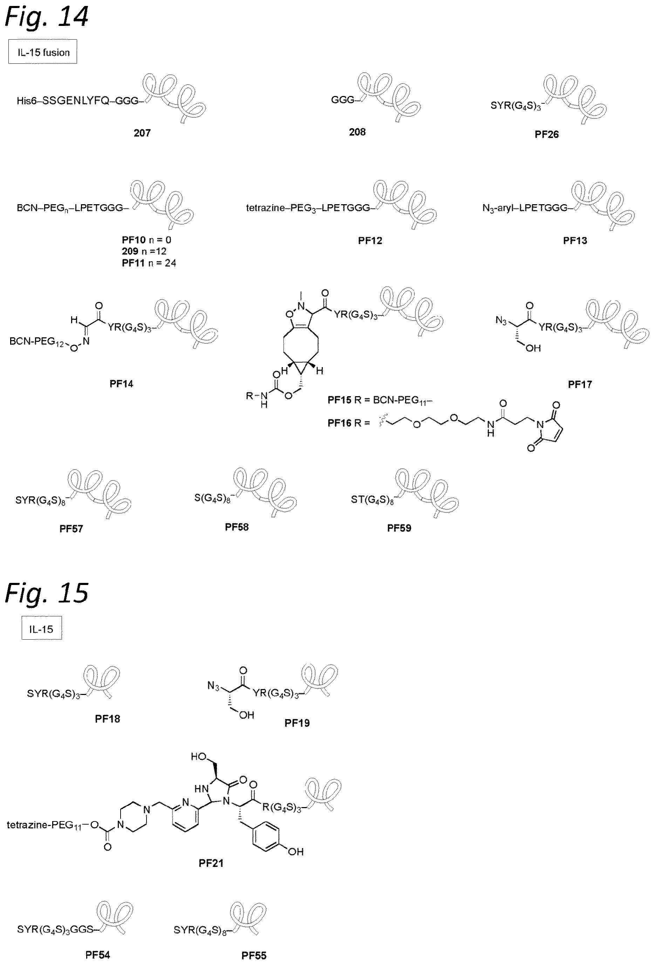

Figures 14 and 15 shows structures of IL-15 variants with suitable groups for SPAAC or SPANC conjugations, or precursors thereof.

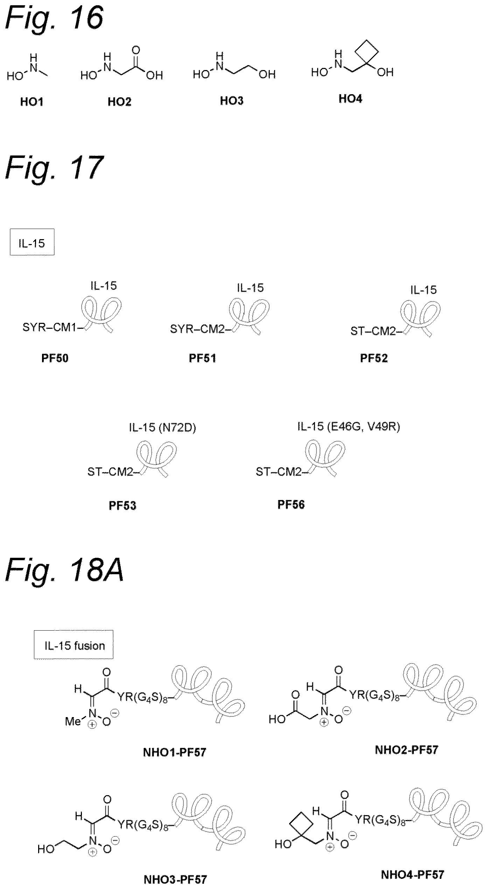

Figure 16 shows structures of hydroxylamines **HO1-HO4.**
Figure 17 shows structures of IL-15 variants with different N-terminal sequence tags **(PF50-52)** and IL-15 mutants **(PF53** and **PF56)**

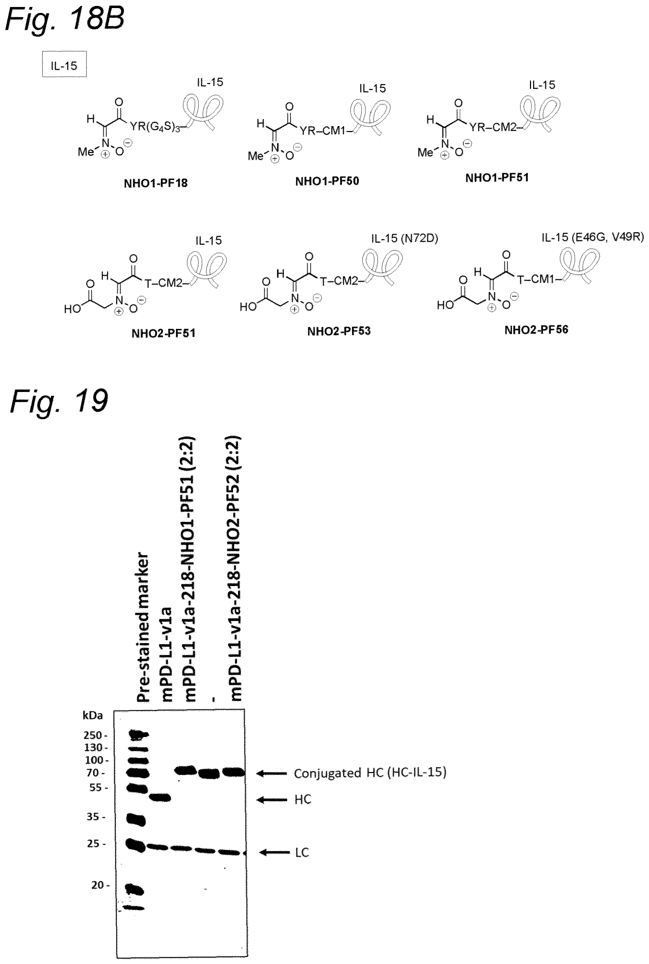

Figure 18A shows structures of various nitrone-variants of IL-15R-IL-15 fusion protein **(PF57)** obtained by incorporation of hydroxylamine **HO1-HO4.** Figure 18B shows structures of various nitrone-variants of various IL-15 mutants **(PF18, PF50, PF51, PF53** and **PF56)** obtained by incorporation of hydroxylamine **HO1** and **HO2.**
Figure 19 shows the SDS-page analysis under reducing conditions for various antibody-cytokine conjugates based on mPD-L1 and IL-15 variants **PF51** and **FPF52.**

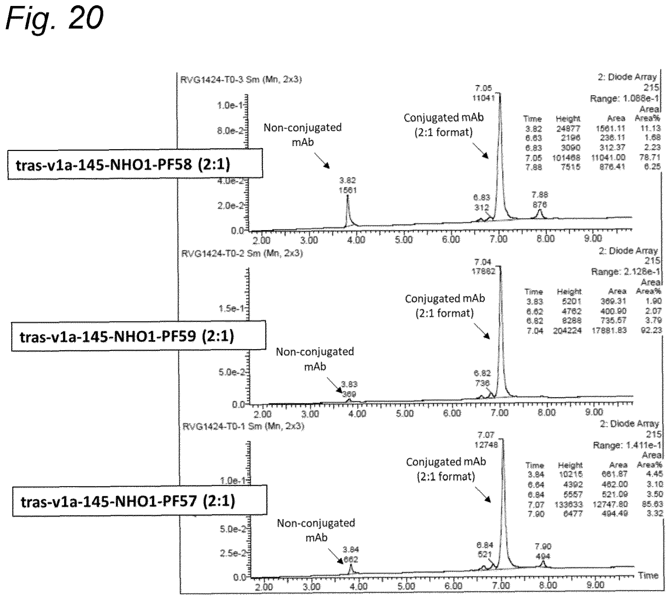

Figure 20 shows RP-UPLC analysis under non-reducing conditions showing formation of the 2:1 format for trastuzumab and IL-15 variants with different N-terminal sequences.

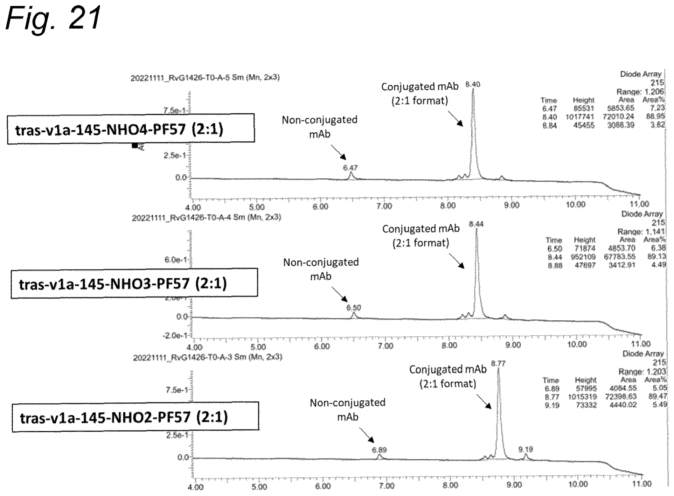

Figure 21 shows RP-UPLC analysis under nonreducing conditions showing formation of the 2:1 format for trastuzumab and IL-15 variants modified with hydroxylamines **HO2-HO4.**

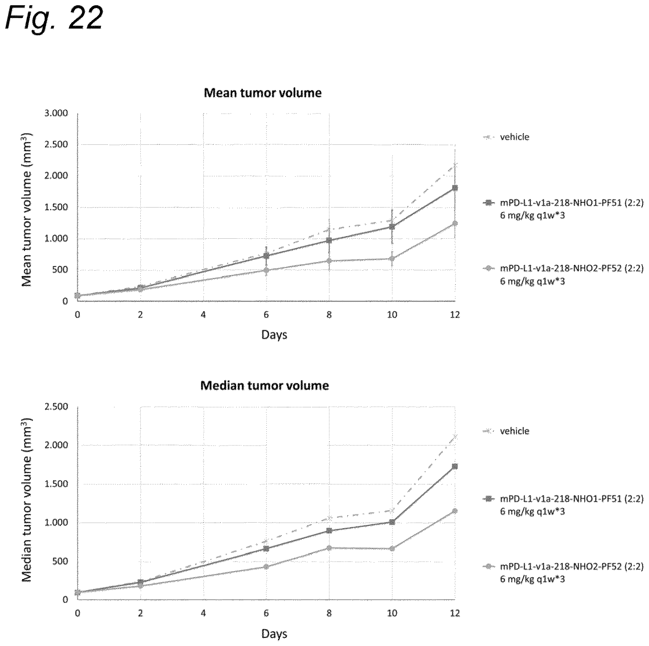

Figure 22 shows the in vivo efficacy of antibody-cytokine conjugates **mPD-L1-v1a-218-NHO1-PF51** and **mPD-L1-v1a-218-NHO2-PF52,** dosed at 6 mg/kg on day 0 and day 7 and mice were monitored for tumor volume. Data are plotted as mean values +/- standard error of the mean (top graph) and as median tumor volume (bottom graph).

### Examples

The invention is illustrated by the following examples.

### Synthesis of hydroxylamine compounds

### Example 1. Synthesis of compound HO2

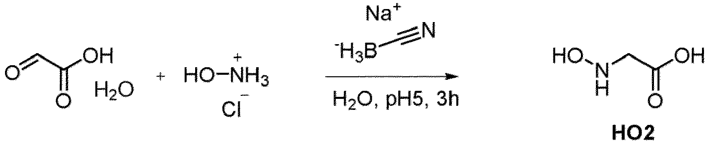

2-oxoacetic acid hydrate (1.24 g, 1 Eq, 13.5 mmol) and hydroxylamine hydrochloride (936 mg, 1 Eq, 13.5 mmol) were stirred in water (60 mL) at room temperature. 1M NaOH was added to raise the pH to 5 before addition of sodium cyanoborohydride (2.12 g, 2.5 Eq, 33.7 mmol). The reaction was left to stir for 3h.The mixture was concentrated in vacuo and the resulting crude was purified by crystallization from a minimum of water to afford **HO2** (754 mg, 8.28 mmol, 62 %) as a white solid. 1H NMR (400 MHz, D2O) δ 3.77 (s, 1H). 13C NMR (101 MHz, DMSO) δ 172.40, 55.10.

### Example 2. Synthesis of compound HO3

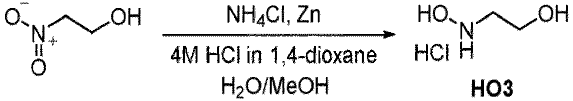

To a solution of ammonium chloride (793 mg, 2.7 Eq, 14.8 mmol) in water (3 mL) was added 2-nitroethan-1-ol (500 mg, 394 µL, 1 Eq, 5.49 mmol) and methanol (12 mL). The resulting solution was stirred and zinc (729 mg, 2.03 Eq, 11.1 mmol) was added in small portions keeping the temperature below 65 °C. The mixture was stirred at RT for 30 min and filtered. The precipitate was washed with methanol and H2O. Hydrogen chloride in 1,4-dioxane (400 mg, 2.75 mL, 4 molar, 2 Eq, 11.0 mmol) was added to the filtrate and the solution was concentrated in vacuo. The resulting crude was triturated with IPA to remove insoluble NH4Cl crystals. The filtrate was concentrated in vacuo and the resulting crude was taken up in H2O. Aqueous phase was extracted with Et2O and EtOAc to remove residual 2-nitroethan-1-ol. The aqueous phase was concentrated in vacuo to afford as **HO3** (444 mg, 3.91 mmol, 71 %) as a yellow oil. 1H NMR (400 MHz, D2O) δ 3.82 - 3.78 (m, 2H), 3.32 - 3.28 (m, 2H). 13C NMR (101 MHz, D2O) δ 54.26, 52.38.

### Example 3. Synthesis of compound HO4

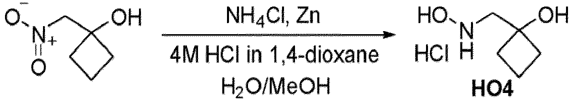

To a solution of ammonium chloride (551 mg, 2.7 Eq, 10.3 mmol) in water (3 mL) was added 1-(nitromethyl)cyclobutan-1-ol (500 mg, 1 Eq, 3.81 mmol) and methanol (12 mL). The resulting solution was stirred and zinc (506 mg, 2.03 Eq, 7.74 mmol) was added in small portions keeping the temperature below 65 °C. The mixture was stirred at RT for 15 min and filtered. The precipitate was washed with methanol and H2O. hydrogen chloride in Dioxane (278 mg, 1.91 mL, 4 molar, 2 Eq, 7.63 mmol) was added to the filtrate and the solution was concentrated in vacuo. The resulting crude was triturated with IPA to remove insoluble NH4Cl crystals. The filtrate was concentrated in vacuo and the resulting crude was taken up in H2O. Aqueous phase was extracted with Et2O and EtOAc to remove residual 1-(nitromethyl)cyclobutan-1-ol. The aqueous phase was concentrated in vacuo to afford as **HO4** (495 mg, 3.22 mmol, 85 %) as a yellow oil. 1H NMR (400 MHz, D2O) δ 3.49 (s, 2H), 2.24 - 2.09 (m, 4H), 1.89 - 1.50 (m, 2H). 13C NMR (101 MHz, D2O) δ 70.80, 57.41, 33.44, 11.41.

### Synthesis of BCN-compounds

### Example 4. Synthesis of compound 218

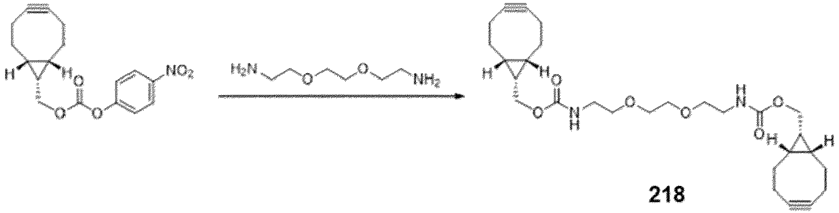

To a solution of 2-[2-(2-aminoethoxy)ethoxy]ethan-1-amine (71 mg, 0.48 mmol, 1 eq) in anhydrous DCM (1 mL) was added BCN-OPNP (339.2 mg, 1.07 mmol, 2.2 eq) and triethylamine (100 µL, 0.71 mmol, 1.5 eq). After stirring for 3 at room temperature, the reaction mixture was purified by flash column chromatography over silicagel ((0% → 30% EtOAc in DCM (to remove p-nitrophenol) followed by 0% ---> 20% MeOH in DCM ) to give **218** as a colorless oil (191.5 mg, 0.36 mmol, 74%). LCMS (ESI+) calculated for C48H41N2O6S+ (M+H+) 501.63 found 501.56. 1H NMR (400 MHz, CDCl3) δ (ppm) 4.16 (d, J = 8.0 Hz, 4H), 3.62 (s, 4H), 3.57 (dd, J = 5.6, 4.7 Hz, 4H), 3.40 (q, J = 5.4 Hz, 4H), 2.36 - 2.17 (m, 12H), 1.61 (m, 4H), 1.37 (p, J = 8.6 Hz, 2H), 1.01 - 0.90 (m, 4H).

### Example 5. Synthesis of compound 127 and 128

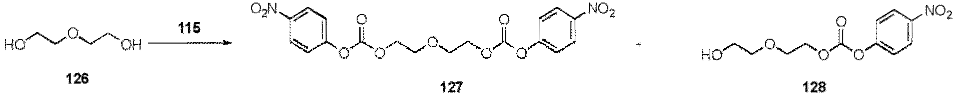

To a solution of diethyleneglycol **126** (446 µL, 0.50 g, 4.71 mmol) in DCM (20 mL) were added 4-nitrophenol chloroformate (1.4 g, 7.07 mmol) and Et3N (3.3. mL, 2.4 g, 23.6 mmol). The mixture was stirred, filtered and concentrated in vacuo (at 55°C). The residue was purified by silica gel chromatography (15% → 75% EtOAc in heptane) and two products were isolated. Product **127** was obtained as a white solid (511 mg, 1.17 mmol, 25%).1H NMR (400 MHz, CDCl3) δ (ppm) 8.31-8.23 (m, 4H), 7.43-7.34 (m, 4H), 4.54-4.44 (m, 4H), 3.91-3.83 (m, 4H). Product **128** was obtained as a colorless oil (321 mg, 1.18 mmol, 25%).1H NMR (400 MHz, CDCl3) δ (ppm) 8.32-8.24 (m, 2H), 7.43-7.36 (m, 2H), 4.50-4.44 (m, 2H), 3.86-3.80 (m, 2H), 3.81-3.74 (m, 2H), 3.69-3.64 (m, 2H).

### Example 6. Synthesis of compound 145

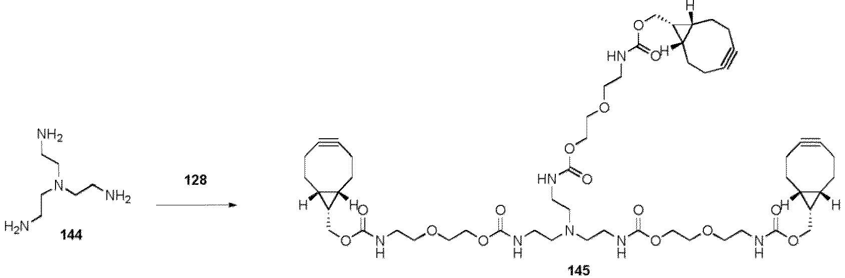

To a solution of **128** (200 mg, 0.45 mmol) in DCM (1 mL) were added triethylamine (41.6 uL, 0.30 mmol) and tris(2-aminoethyl)amine **144** (14.9 uL, 0.10 mmol). After stirring the mixture for 150 minutes, it was concentrated in vacuo. The residue was purified by silica gel column chromatography (25% ---> 100% EtOAc in DCM then 0% ---> 10% MeOH in DCM) and gave **145** in 43% yield (45.4 mg, 42.5 umol) as a yellow oil. 1H NMR (400 MHz, CDCl3): δ (ppm) 5.68-5.18 (m, 6H), 4.32-4.18 (m, 6H), 4.18-4.11 (d, J = 7.9 Hz, 6H), 3.74-3.61 (m, 6H), 3.61-3.51 (m, 6H), 3.43-3.29 (m, 6H), 3.29-3.15 (m, 6H), 2.65-2.47 (m, 6H), 2.37-2.16 (m, 18H), 1.69-1.49 (m, 6H), 1.35 (quintet, J = 8.9 Hz, 3H), 1.03-0.87 (m, 6H).

### Expression and isolation of cytokines

### General procedure for mass spectral analysis of cytokines, antibodies and antibody-cytokine fusions

Prior to mass spectral analysis, IgG was treated with IdeS, which allows analysis of the Fc/2 fragment. For analysis of the Fc/2 fragment, a solution of 20 µg (modified) IgG was incubated for 1 hour at 37 °C with IdeS/Fabricator^{™} (1.25 U/µL) in PBS Ph 7.4 in a total volume of 10 µL. Samples were diluted to 80 µL using PBS pH 7.4. For analysis of cytokines, a solution of 4 µg (modified) cytokine was diluted to 80 µL using PBS pH 7.4. MS analysis is performed on JEOL AccuTOF LC-plus JMS-T100LP system (ESI-TOF) combined with a HPLC system (Agilent 1100 series, Hewlett Packard). On the HPLC system a MassPREP^{™} On-line Desalting Cartridge (Waters PIN 186002785) is installed. For analysis of cytokines, a solution of 4 µg (modified) cytokine was diluted to 80 µL using PBS followed by analysis electrospray ionization time-of-flight (ESI-TOF) on a JEOL AccuTOF. Deconvoluted spectra were obtained using Magtran software.

### General procedure for expression of cytokines and inclusion body isolation

Expression of cytokines starts with transformation of the corresponding into BL21(DE3) cells (Novagen). Transformed cells were plated on LB-agar with ampicillin and incubated overnight at 37 °C. A single colony was picked and used to inoculate a small culture of TB medium + ampicillin followed by incubated overnight at 37 °C. Next, the overnight culture was used to inoculate a larger volume by performing a dilution of 20 fold in TB medium + ampicillin. The culture was incubated at 37 °C at 160 RPM and, when OD600 reached 2.5-4.0, induced with 1 mM IPTG (addition of 1 mL of 1 M stock solution). After >16 hour induction at 37 °C at 160 RPM, the culture was pelleted by centrifugation (5000 xg for 5 min). The culture cell pellet was lysed in BugBuster^{™} containing Benzonase and incubated on roller bank for 30 min at room temperature. After lysis the insoluble fraction was separated from the soluble fraction by centrifugation (15 minutes, 15000 x g). Half of the insoluble fraction was dissolved in BugBuster^{™} with lysozyme (final concentration of 200 pg/mL) and incubated on the roller bank for 10 min. Next the solution was diluted with 10 volumes of miliQ and centrifuged 15 min, 15000 x g . The pellet was washed in a solution of 1 :10 diluted BugBuster^{™} and centrifuged at 10 min, 12000 x g. This washing round was performed 1-3 times until a very white inclusion body pellet was obtained.

### General procedure for refolding of cytokines

The purified inclusion bodies were dissolved and denatured in 5 M guanidine with 40 mM Cysteamine and 20 mM Tris pH 8.0, to a final concentration of 1 mg/mL. Afterwards, the solution was incubated for 2 hours at RT on a rollerbank. Followingly, the 1 mg/mL solution is added dropwise to 10 volumes of refolding buffer (50 mM Tris, 10.53 mM NaCl, 0.44 mM KCI, 2.2 mM MgCl2 , 2.2 mM CaCl2 , 0.055% PEG- 4000, 0.55 M L-arginine, 4 mM cysteamine, 4 mM cystamine, at pH 8.0) in a cold room at 4°C, stirring required. Leave solution at least 24 hours at 4°C. Dialyze the solution to 10 mM NaCl and 20 mM Tris pH 8.0, 1x overnight and 2x 4 hours, using a Spectrum^{™} Spectra/Por^{™} 2 RC Dialysis Membrane Tubing 12-14 kDa MWCO. The refolded product was loaded onto a equilibrated Q-trap anion exchange column (GE health care) on an AKTA Purifier-10 (GE Healthcare). The column was first washed with buffer A (20 mM Tris, 10 mM NaCl, pH 8.0). Retained protein was eluted with buffer B (20 mM Tris buffer, 1 M NaCl, pH 8.0) on a gradient of 60 mL from buffer A to buffer B. Mass spectrometry was performed for the analysis of the product.

### Example 7. Design of IL-15 variants

Cleavable IL-15 variants were designed consisting of an N-terminal sequence suitable for chemical modification, consisting of either (M)SYR-, (M)ST- or (M)S-, fused to a protease-cleavable linker (amino acid sequence being identified by SEQ ID NO: 1 and SEQ ID NO: 2) and either wild-type or mutated human IL-15 (amino acid sequence of wild-type IL-15 being identified by SEQ ID NO: 3). The N-terminal methionine will be cleaved after expression leaving an N-terminal serine needed for chemical modification. Both cleavable linkers have been designed for recognition by several tumor-specific proteases including matriptase (MT-SP1), legumain and urokinase (uPA). Non-cleavable variants have been designed by replacing the cleavable linker by either-(G₄S)₈-, - (G₄S)₃- or a -(G₄S)₃GGS- linker. In some cases the sushi domain of IL-15Ra is fused to IL-15 via a flexible linker (amino acid sequence of IL-15Rα-linker-IL-15 being identified by SEQ ID NO: 4).
*Sequence identification* of protease-cleavable linker 1 *(SEQ. ID NO: 1):* GSSGGSGGSGGSGLSGRSDNHGSSGS
*Sequence identification of* protease-cleavable linker 2 *(SEQ. ID NO: 2):* GGGGSGGGGSRASRANGS
*Sequence identification of human IL-15 (SEQ. ID NO: 3):* 
NWVNVISDLKKIEDLIQSMHIDATLYTESDVHPSCKVTAMKCFLLELQVISLESGDASIHDTVENLIIL
ANNSLSSNGNVTESGCKECEELEEKNIKEFLQSFVHIVQMFINTS
*Sequence identification of* IL-15Ra-IL-15 fusion protein *(SEQ. ID NO: 4):*

ITCPPPMSVEHADIWVKSYSLYSRERYICNSGFKRKAGTSSLTECVLNKATNVAHWTTPSLKCIR
DPALVHQRPAPPSGGSGGGGSGGGSGGGGSLQNWVNVISDLKKIEDLIQSMHIDATLYTESDVH
PSCKVTAMKCFLLELQVISLESGDASIHDTVENLIILANNSLSSNGNVTESGCKECEELEEKNIKEF
LQSFVHIVQMFINTS

### Example 8. Gene synthesis and cloning into expression vector

Codon-optimized DNA encoding **PF51** (amino acid sequence being identified by SEQ ID NO: 5), **PF52** (amino acid sequence being identified by SEQ ID NO: 6), **PF57** (amino acid sequence being identified by SEQ ID NO: 7), **PF58** (amino acid sequence being identified by SEQ ID NO: 8), **PF59** (amino acid sequence being identified by SEQ ID NO: 9) were obtained from Genscript, Piscataway, USA. All codon-optimized DNA sequences were cloned into a pET15b-vector for tag-free expression of the proteins in E. coli.
*Sequence identification of* SYR-cleavable linker 2-IL1 5 **(PF51)** *(SEQ. ID NO: 5):* 
MSYRGGGGSGGGGSRASRANGSNWVNVISDLKKIEDLIQSMHIDATLYTESDVHPSCKVTAMKC
FLLELQVISLESGDASIHDTVENLIILANNSLSSNGNVTESGCKECEELEEKNIKEFLQSFVHIVQMF
INTS
*Sequence identification of* ST-cleavable linker 2-IL15 (**PF52**) *(SEQ. ID NO: 6):* 
STGGGGSGGGGSRASRANGSNWVNVISDLKKIEDLIQSMHIDATLYTESDVHPSCKVTAMKCFL
LELQVISLESGDASIHDTVENLIILANNSLSSNGNVTESGCKECEELEEKNIKEFLQSFVHIVQMFIN
TS
*Sequence identification of* SYR-(G₄S)₈-IL15Rα-IL-15 (**PF57**) *(SEQ. ID NO: 7):* 
(M)SYRGGGGSGGGGSGGGGSGGGGSGGGGSGGGGSGGGGSGGGGSITCPPPMSVEHADI
WVKSYSLYSRERYICNSGFKRKAGTSSLTECVLNKATNVAHWTTPSLKCIRDPALVHQRPAPPS
GGSGGGGSGGGSGGGGSLQNWVNVISDLKKIEDLIQSMHIDATLYTESDVHPSCKVTAMKCFLL
ELQVISLESGDASIHDTVENLIILANNSLSSNGNVTESGCKECEELEEKNIKEFLQSFVHIVQMFINT
S
*Sequence identification of* S-(G₄S)₈-IL15Rα-IL-15 (**PF58**) *(SEQ. ID NO: 8):* 
SGGGGSGGGGSGGGGSGGGGSGGGGSGGGGSGGGGSGGGGSITCPPPMSVEHADIWVKSY
SLYSRERYICNSGFKRKAGTSSLTECVLNKATNVAHWTTPSLKCIRDPALVHQRPAPPSGGSGG
GGSGGGSGGGGSLQNWVNVISDLKKIEDLIQSMHIDATLYTESDVHPSCKVTAMKCFLLELQVIS
LESGDASIHDTVENLIILANNSLSSNGNVTESGCKECEELEEKNIKEFLQSFVHIVQMFINTS
*Sequence identification of* ST-(G₄S)₈-IL15Rα-IL-15 (**PF59**) *(SEQ. ID NO: 9):*

STGGGGSGGGGSGGGGSGGGGSGGGGSGGGGSGGGGSGGGGSITCPPPMSVEHADIWVKS
YSLYSRERYICNSGFKRKAGTSSLTECVLNKATNVAHWTTPSLKCIRDPALVHQRPAPPSGGSG
GGGSGGGSGGGGSLQNWVNVISDLKKIEDLIQSMHIDATLYTESDVHPSCKVTAMKCFLLELQVI
SLESGDASIHDTVENLIILANNSLSSNGNVTESGCKECEELEEKNIKEFLQSFVHIVQMFINTS

### Example 9. Expression and refolding of cytokines PF51, PF52 and PF57-PF59

Expression and inclusion body isolation for each cytokine mentioned in table 1. was performed according to *General procedure for expression of cytokines and inclusion body isolation.* The refolding of the cytokines was performed according to *General procedure for refolding of cytokines* with small adaptations. For cytokine **PF51** and **PF52** the refolding was performed according to the standard procedure. For cytokine **PF57** and **PF59,** the cytokine was dissolved and denatured in 5 M guanidine with 40 mM Cysteamine and 20 mM Tris pH 8.0, to a final concentration of 3 mg/mL instead of 1 mg/mL. For cytokine **PF58,** the cytokine was dissolved and denatured in 5 M guanidine with 40 mM Cysteamine and 20 mM Tris pH 8.0, to an unknown concentration instead of 1 mg/mL.

**Table 1. Expression and refolding conditions for the purified cytokines.**

| Cytokine | Expression scale (L) | Inclusion body yield (mg) | Refolding scale (mg) | Final yield | Observed mass (Da) | Calculated mass (Da |
|---|---|---|---|---|---|---|
| **PF51** | 1.5 | 407 | 203 | 63.3 mg (31.3%) | 14606 | 14606 |
| **PF52** | 1 | 313 | 313 | 166.9 mg (53.4%) | 14388 | 14392 |
| **PF57** | 1.5 | 143 | 143 | 16.7 mg (11.7 %) | 25719 | 25721 |
| **PF58** | 1 | N.D. | N.D. | 2.4 mg | 25401 | 25402 |
| **PF59** | 0.5 | 172 | 172 | 1.3 mg (0.7%) | 25506 | 25503 |

### Preparation of modified cytokines

### Example 10. N-terminal nitrone functionalization of PF51 to obtain NHO1-PF51

To **PF51** (56100 µL, 61 µM in PBS) was added 2 eq NalO₄ (68.5 µL of 100 mM stock in PBS). The reaction was incubated for 5 minutes at RT. Mass spectral analysis showed oxidation of the serine into the corresponding hydrate (observed masses 14594 Da). To quench the remaining NalO₄ in the reaction, 6.6 eq of p-methoxybenzenethiol were added (2260 µL, 10 mM in DMF). To the quenched reaction was added 160 eq **NHO1** (5500 µL of 100 mM stock in PBS) and 160 eq p-Anisidine (5500 µL of 100 mM stock in PBS). The reaction mixture was incubated overnight at RT. Mass spectral analysis showed one single peak (observed mass 14604 Da) corresponding to **NHO1-PF51.** The reaction mixture was dialyzed to PBS, 1x overnight and 2x 3 hours, using a Spectrum^{™} Spectra/Por^{™} 2 RC Dialysis Membrane Tubing 12-14 kDa MWCO.

### Example 11. N-terminal nitrone functionalization of PF57 to obtain NHO1-PF57

To **PF57** (8000 µL, 69 µM in PBS) was added 2 eq NalO₄ (11 µL of 100 mM stock in PBS). The reaction was incubated for 5 minutes at RT. Mass spectral analysis showed oxidation of the serine into the corresponding hydrate (observed mass 25707 Da). To quench the remaining NalO₄ in the reaction, 6.6 eq of p-methoxybenzenethiol were added (364 µL, 10 mM in DMF). To the quenched reaction was added 160 eq **NHO1** (870 µL of 100 mM stock in PBS) and 160 eq p-Anisidine (870 µL of 100 mM stock in PBS). The reaction mixture was incubated overnight at RT. Mass spectral analysis showed one single peak (observed mass 25720 Da) corresponding to **NHO1-PF57.** The reaction mixture was directly loaded onto a desalting column (Cytiva, HiPrep^{™} 26/10 desalting column) using PBS pH 7.5 as a mobile phase on an AKTA explorer (GE Healthcare).

### Example 12. N-terminal nitrone functionalization of PF58 to obtain NHO1-PF58

To **PF58** (1443 µL, 65 µM in PBS) was added 2 eq NalO₄ (18.8 µL of 10 mM stock in PBS). The reaction was incubated for 5 minutes at RT. Mass spectral analysis showed oxidation of the serine into the corresponding hydrate (observed mass 25388 Da). To quench the remaining NalO₄ in the reaction, 6.6 eq of p-methoxybenzenethiol were added (62 µL, 10 mM in DMF). To the quenched reaction was added 160 eq **NHO1** (150 µL of 100 mM stock in PBS) and 160 eq p-Anisidine (150 µL of 100 mM stock in PBS). The reaction mixture was incubated overnight at RT. Mass spectral analysis showed one single peak (observed mass 25401 Da) corresponding to **NHO1-PF58.** The reaction mixture was directly loaded on a Superdex^{™}75 Increase (Cytiva) using PBS pH 7.5 as a mobile phase on an AKTA explorer (GE Healthcare).

### Example 13. N-terminal nitrone functionalization of PF59 to obtain NHO1-PF59

To **PF59** (1000 µL, 30 µM in PBS) was added 2 eq NalO₄ (6 µL of 10 mM stock in PBS). The reaction was incubated for 5 minutes at RT. Mass spectral analysis showed oxidation of the serine into the corresponding hydrate (observed mass 25494 Da). To quench the remaining NalO₄ in the reaction, 6.6 eq of p-methoxybenzenethiol were added (19.8 µL, 10 mM in DMF). To the quenched reaction was added 160 eq **NHO1** (49 µL of 100 mM stock in PBS) and 160 eq p-Anisidine (49 µL of 100 mM stock in PBS). The reaction mixture was incubated overnight at RT. Mass spectral analysis showed one single peak (observed mass 25503 Da) corresponding to **NHO1-PF59.** The reaction mixture was purified using Amicon Ultra-0.5 Centrifugal Filter Units (10 kDa MWCO) by performing 5 rounds of buffer exchange to PBS pH 7.5.

### Example 14. N-terminal nitrone functionalization of PF52 to obtain NHO2-PF52

To **PF52** (88679 µL, 61 µM in PBS) was added 2 eq NalO₄ (1113 µL of 10 mM stock in PBS). The reaction was incubated for 5 minutes at RT. Mass spectral analysis showed oxidation of the serine into the corresponding hydrate (observed mass 14375 Da). To quench the remaining NalO₄ in the reaction, 6.6 eq of p-methoxybenzenethiol were added (3672 µL, 10 mM in DMF). To the quenched reaction was added 160 eq **NHO2** (8901 µL of 100 mM stock in PBS) and 160 eq p-Anisidine (8901 µL of 100 mM stock in PBS). The reaction mixture was incubated overnight at RT. Mass spectral analysis showed one single peak (observed mass 14429 Da) corresponding to **NHO2-PF52.** Dialyze the solution to 10 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 10 mM NaCl, pH 7.5). Retained protein was eluted with a linear gradient to buffer B (20 mM Tris buffer, 1 M NaCl, pH 7.5).The elute was directly loaded onto a desalting column (Cytiva, HiPrep^{™} 26/10 desalting column) using PBS pH 7.5 as a mobile phase on an AKTA explorer (GE Healthcare).

### Example 15. N-terminal nitrone functionalization of PF57 to obtain NHO2-PF57

To **PF57** (1350 µL, 61 µM in PBS) was added 2 eq NalO₄ (16.3 µL of 10 mM stock in PBS). The reaction was incubated for 5 minutes at RT. Mass spectral analysis showed oxidation of the serine into the corresponding hydrate (observed mass 25708 Da). To quench the remaining NalO₄ in the reaction, 6.6 eq of p-methoxybenzenethiol were added (54 µL, 10 mM in DMF). To the quenched reaction was added 160 eq **NHO2** (133 µL of 100 mM stock in PBS) and 160 eq p-Anisidine (133 µL of 100 mM stock in PBS). The reaction mixture was incubated overnight at RT. Mass spectral analysis showed one single peak (observed mass 25763 Da) corresponding to **NHO2-PF57.** The reaction mixture was purified using Amicon Ultra-0.5 Centrifugal Filter Units (10 kDa MWCO) by performing 5 rounds of buffer exchange to PBS pH 7.5.

### Example 16. N-terminal nitrone functionalization of PF57 to obtain NHO3-PF57

To **PF57** (1350 µL, 61 µM in PBS) was added 2 eq NalO₄ (16.3 µL of 10 mM stock in PBS). The reaction was incubated for 5 minutes at RT. Mass spectral analysis showed oxidation of the serine into the corresponding hydrate (observed mass 25708 Da). To quench the remaining NalO₄ in the reaction, 6.6 eq of p-methoxybenzenethiol were added (54 µL, 10 mM in DMF). To the quenched reaction was added 160 eq **NHO3** (133 µL of 100 mM stock in PBS) and 160 eq p-Anisidine (133 µL of 100 mM stock in PBS). The reaction mixture was incubated overnight at RT. Mass spectral analysis showed one single peak (observed mass 25749 Da) corresponding to **NHO3-PF57.** The reaction mixture was purified using Amicon Ultra-0.5 Centrifugal Filter Units (10 kDa MWCO) by performing 5 rounds of buffer exchange to PBS pH 7.5.

### Example 17. N-terminal nitrone functionalization of PF57 to obtain NHO4-PF57

To **PF57** (1350 µL, 61 µM in PBS) was added 2 eq NalO₄ (16.3 µL of 10 mM stock in PBS). The reaction was incubated for 5 minutes at RT. Mass spectral analysis showed oxidation of the serine into the corresponding hydrate (observed mass 25708 Da). To quench the remaining NalO₄ in the reaction, 6.6 eq of p-methoxybenzenethiol were added (54 µL, 10 mM in DMF). To the quenched reaction was added 160 eq **NHO4** (133 µL of 100 mM stock in PBS) and 160 eq p-Anisidine (133 µL of 100 mM stock in PBS). The reaction mixture was incubated overnight at RT. Mass spectral analysis showed one single peak (observed mass 25789 Da) corresponding to **NHO4-PF57.** The reaction mixture was purified using Amicon Ultra-0.5 Centrifugal Filter Units (10 kDa MWCO) by performing 5 rounds of buffer exchange to PBS pH 7.5.

### Example 18. N-terminal BCN functionalization of NHO1-PF51 by SPANC to obtain 218-NHO1-PF51

To **NHO1-PF51** (77055 µL, 44 µM in PBS) was added 10 eq BCN-PEG₂-BCN **218** (8562 µL, 4 mM in DMF). The reaction was incubated overnight at room temperature. Dialyze the solution to 10 mM NaCl and 20 mM Tris pH 8.0, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 10 mM NaCl, pH 8.0). Retained protein was eluted with a linear gradient to buffer B (20 mM Tris buffer, 1 M NaCl, pH 8.0). The elute was dialyzed to PBS, 1x overnight and 2x 3 hours, using a Spectrum^{™} Spectra/Por^{™} 2 RC Dialysis Membrane Tubing 12-14 kDa MWCO. Mass spectometry showed the correct product (observed mass 15105 Da) corresponding to **218-NHO1-PF51**

### Example 19. N-terminal BCN functionalization of NHO2-PF52by SPANC to obtain 218-NHO2-PF52

To **NHO2-PF52** (173349 µL, 22 µM in PBS) was added 10 eq BCN-PEG₂-BCN **218** (17335 µL, 2 mM in DMF). The reaction was incubated overnight at room temperature while stirring. Dialyze the solution to 100 mM NaCl and 20 mM Tris pH 7.5, 2x 1 hour, using a Spectrum^{™} Spectra/Por^{™} 2 RC Dialysis Membrane Tubing 12-14 kDa MWCO. For purification, the dialyzed product was directly loaded onto an AEX column (Cytiva 2x5 mL, HiTrap^{™} Q HP) on an AKTA explorer (GE Healthcare). After loading the product, the column was first washed with buffer A (20 mM Tris, 100 mM NaCl, pH 7.5). Retained protein was eluted with a linear gradient to buffer B (20 mM Tris buffer, 1 M NaCl, pH 7.5). The elute was directly loaded onto a desalting column (Cytiva, HiPrep^{™} 26/10 desalting column) using PBS pH 7.5 as a mobile phase on an AKTA explorer (GE Healthcare). Mass spectrometry showed the correct product (observed mass 14929 Da) corresponding to **218-NHO2-PF52.**

### Preparation of modified antibodies

### General procedure for analytical RP-UPLC of cytokines, monoclonal antibodies and antibody-cytokine conjugates

Priorto RP-UPLC analysis, protein samples were diluted to approximately 0.1 mg/mL using PBS pH 7.4. RP-UPLC analysis was performed on a Waters Acquity UPLC-SQD. The sample (5 µL) was injected with 0.4 mL/min onto Bioresolve RP mAb 2.1×150 mm 2.7 µm (Waters) with a column temperature of 70 °C. A linear gradient was applied in 9 minutes from 30 to 54% acetonitrile in 0.1% TFA and water.

### General procedure for analytical SE-HPLC of cytokines, monoclonal antibodies and antibody-cytokine conjugates

HPLC-SEC analysis was performed on an Agilent 1100 series (Hewlett Packard) using an Xbridge BEH200A (3.5 µM, 7.8x300 mm, PN 186007640 Waters) column. The sample was diluted to 1 mg/mL in PBS and measured with 0.86 mL/min isocratic method (0.1 M sodium phosphate buffer pH 6.9 (NaHPO4/Na2PO4) containing 10% isopropanol) for 16 minutes.

### Example 20. Transient expression of mPD-L1 in CHO

mPD-L1 is an antibody targeting murine PD-L1 as described in US2016/0340429 A1, consisting of a LC sequence being identified by SEQ ID NO: 10, and a HC sequence being identified by SEQ ID NO: 11. mPD-L1 was transiently expressed in CHO KI cells by Evitria (Zurich, Switzerland) at 500 mL scale. The antibody was purified using two HiTrap MabSelect Sure 5 mL columns connected in series. After loading of the supernatant the column was washed with TBS pH 7.5 for 10 CV. The IgG was eluted with 0.1 M sodium acetate pH 3.0 and neutralized with 2.5 M Tris-HCl pH 7.2. After three times dialysis to 20 mM Histidine-HCl, 150 mM NaCl, pH 7.5 the IgG was concentrated to 19.3 mg/mL using a Vivaspin Turbo 15 ultrafiltration unit (Sartorius) and obtained in a final yield of 87 mg.

### Example 21. Enzymatic remodeling of trastuzumab to tras(6-N₃-GalNAc)₂ (tras-v1a)

Trastuzumab (Herceptin), obtained from the pharmacy, was reconstituted and buffer exchanged to 20 mM Histidine-HCl, 150 mM NaCl, pH 7.5 using a HiPrep^{™} 26/10 Desalting Column (Cytiva) on an AKTA Purifier-10 (Cytiva). Trastuzumab (31.3 mL, 1128 mg, 36.0 mg/mL in 20 mM Histidine-HCl, 150 mM NaCl, pH 7.5) was incubated with EndoSH (1% w/w), as described in PCT/EP2017/052792, His-TnGaINAcT, as described in PCT/EP2016/059194 (3% w/w), alkaline phosphatase (commercially available from Roche, 0.01% w/w) and UDP-6-N3-GalNAc (10 eq compared to IgG), prepared according to PCT/EP2016/059194, in 20 mM Histidine-HCl pH 7.5 150 mM NaCl pH 7.5 and 6 mM MnCl₂ for 16 hours at 30 °C. Next, the functionalized IgG was purified using a protein A column (Captiva PriMab, CV = 50 mL). After loading of the reaction mixture the column was washed with TBS + 0.2% Triton for 10 CV followed by TBS pH 7.5 for 10 CV. The IgG was eluted with 0.1 M sodium acetate pH 3.0 and neutralized with 2.5 M Tris-HCl pH 7.2. After three times dialysis to TBS pH 7.5, the IgG was concentrated to approximately 25 mg/mL using a Vivaspin Turbo 15 ultrafiltration unit (Sartorius). Mass spectral analysis of a sample after IdeS treatment showed one major Fc/2 products (observed mass 24366 Da, approximately 90% of total Fc/2) corresponding to the 6-N₃-GalNAc-GlcNAc(Fuc)-substituted Fc/2 fragment, and one minor Fc/2 products (observed mass 24220 Da, approximately 10% of total Fc/2) corresponding to the 6-N₃-GalNAc-GlcNAc-substituted Fc/2 fragment.

### Example 22. Intramolecular cross-linking of trast-v1a with trivalent linker 145 to give trast-v1a-145

To a solution of ***trast-v1a*** (1698 µL, 35 mg, 20.61 mg/mL in TBS pH 7.5) was added TBS pH 7.5 (1802 pL), propylene glycol (3427 µL) and trivalent linker 145 (23.3 µL, 40 mM solution in DMF, 4 equiv. compared to IgG). The reaction was incubated overnight at RT followed by purification on a Superdex200 Increase 16/600 column (Cytiva) on an AKTA Purifier-10 (Cytiva) using PBS pH 7.4 as mobile phase. Mass spectral analysis of the IdeS-digested sample showed one major product (calculated mass 49799 Da, observed mass 49799Da), corresponding to intramolecularly cross-linked trastuzumab derivative ***trast-v1a-145**.*

### Example 23. Enzymatic remodeling ofmPD-L1 to mPD-L1(₆-N₃-GalNAc)₂ (mPD-L1-v1a)

mPD-L1 (4528 µL, 87 mg, 19.3 mg/mL in 20 mM Histidine-HCl, 150 mM NaCl, pH 7.5) was incubated with EndoSH (1% w/w), as described in PCT/EP2017/052792, His-TnGaINAcT, as described in PCT/EP2016/059194 (4% w/w), alkaline phosphatase (commercially available from Roche, 0.01% w/w) and UDP-6-N3-GalNAc (25 eq compared to IgG), prepared according to PCT/EP2016/059194, in 20 mM Histidine-HCl pH 7.5 150 mM NaCl pH 7.5 and 6 mM MnCl₂ for 16 hours at 30 °C. Next, the functionalized IgG was purified using two HiTrap MabSelect Sure 5 mL columns connected in series. After loading of the reaction mixture the column was washed with TBS + 0.2% Triton for 10 CV followed by TBS pH 7.5 for 10 CV. The IgG was eluted with 0.1 M sodium acetate pH 3.0 and neutralized with 2.5 M Tris-HCl pH 7.2. After three times dialysis to PBS pH 7.4, the IgG was concentrated to approximately 25 mg/mL using a Vivaspin Turbo 15 ultrafiltration unit (Sartorius). Mass spectral analysis of a sample after IdeS treatment showed one major Fc/2 products (observed mass 24368 Da, approximately 90% of total Fc/2) corresponding to the 6-N₃-GalNAc-GlcNAc(Fuc)-substituted Fc/2 fragment.

### Preparation of antibody-cytokine conjugates

### General procedure for purification of antibody-cytokine conjugates

Unless stated otherwise, antibody-cytokine conjugates were purified according to the following procedure. The reaction was diluted with 2 volumes of 20 mM Tris-HCl pH 8.0 to reduce the salt concentration to 50 mM NaCl. Next, the sample was loaded onto a 5 mL HiTrap Q HP column (Cytiva) using 20 mM Tris-HCl pH 8.0, 50 mM NaCl as mobile phase. After loading the column was washed with ≥20 column volumes (CV) of 20 mM Tris-HCl pH 8.0, 50 mM NaCl, 0.2% Triton-X100 followed by ≥20CV of 20 mM Tris-HCl pH 8.0, 50 mM NaCl. Next the product was eluted using a linear gradient of 20CV to 20 mM Tris-HCl pH 8.0, 500 mM NaCl. Fractions containing the desired product were collected, pooled and concentrated to ~5 mL using Vivaspin Turbo 15 ultrafiltration units (Sartorius). Next, the sample was purified on a Superdex200 Increase 16/600 column (Cytiva) using PBS pH 7.4 as mobile phase. The final antibody-cytokine conjugate was snap-frozen and stored at -80 °C until further use.

### Example 24. Conjugation of mPD-L1-v1a to 218-NHO1-PF51to obtain conjugate mPD-L1-v1a-218-NHO1-PF51 (2:2)

To a solution of **mPD-L1-v1a** (659 µL, 21 mg, 31.9 mg/mL in PBS pH 7.4) was added 218-**NHO1-PF51** (1352 µL, 7.8 mg/mL in PBS pH 7.4, 5 equiv. compared to IgG). The reaction was incubated overnight at 37 °C followed by purification according to the standard procedure. SDS-PAGE analysis confirmed formation of **mPD-L1-v1a-218-NHO1-PF51 (2:2)** (Figure 19).

### Example 25. Conjugation of mPD-L1-v1a to 218-NHO2-PF52 to obtain conjugate mPD-L1-v1a-218-NHO2-PF52 (2:2)

To a solution of **mPD**-**L1-v1a** (785 µL, 25 mg, 31.9 mg/mL in PBS pH 7.4) was added **218-NHO2-PF52** (2077 µL, 6.0 mg/mL in PBS pH 7.4, 5 equiv. compared to IgG). The reaction was incubated overnight at 37 °C followed by purification according to the standard procedure. SDS-PAGE analysis confirmed formation of **mPD-L1-v1a-218-NHO2-PF52 (2:2)** (Figure 19). In addition, mass spectral analysis of the IdeS digested sample showed one major product (observed mass 39295 Da), corresponding to conjugated Fc/2-fragment.

### Example 26. Conjugation of tras-v1a-145 to NHO1-PF57 to obtain conjugate tras-v1a-145-NHO1-PF57 (2:1)

To a solution of **trast-v1a-145** (1600 µL, 16 mg, 10 mg/mL in PBS pH 7.4) was added **NHO1-PF57** (727 µL, 6.20 mg/mL in PBS pH 7.4, 1.6 equiv. compared to IgG). The reaction was incubated overnight at room temperature. The reaction was diluted 20-fold using 20 mM Tris-HCl pH 8.0 and loaded onto a 1 mL HiTrap Q HP column (Cytiva) using 20 mM Tris-HCl pH 8.0, 10 mM NaCl as mobile phase. Next the product was eluted using a linear gradient of 50 CV to 20 mM Tris-HCl pH 8.0, 1 M NaCl. Fractions containing the desired 2:1 format were collected and concentrated to 1 mL using centrifugal filters (Amicon Ultra-0.5 mL MWCO 10 kDa, Merck Millipore). Next, the sample was purified on a Superdex200 Increase 10/300 GL column (Cytiva) on an AKTA Purifier-10 (Cytiva) using PBS pH 7.4 as mobile phase. Mass spectral analysis of the intact sample showed one major product (observed mass 173099 Da), corresponding to conjugate **tras-v1a-145-NHO1-PF57 (2:1).** RP-UPLC analysis of the intact sample confirmed formation of the conjugate **tras-v1a-145-NHO1-PF57 (2:1)** (Figure 20).

### Example 27. Conjugation of tras-v1a-145 to NHO1-PF58 to obtain conjugate tras-v1a-145-NHO1-PF58 (2:1)

To a solution of **trast-v1a-145** (86 µL, 0.7 mg, 8.1 mg/mL in PBS pH 7.4) was added **NHO1-PF58** (42 µL, 7.94 mg/mL in PBS pH 7.4, 3 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO1-PF58 (2:1)** (Figure 20).

### Example 28. Conjugation of tras-v1a-145 to NHO1-PF59 to obtain conjugate tras-v1a-145-NHO1-PF59 (2:1)

To a solution of **trast-v1a-145** (85 µL, 0.9 mg, 10 mg/mL in PBS pH 7.4) was added **NHO1-PF59** (140 µL, 2.16 mg/mL in PBS pH 7.4, 2 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO1-PF59 (2:1)** (Figure 20).

### Example 29. Conjugation of tras-v1a-145 to NHO2-PF57 to obtain conjugate tras-v1a-145-NHO2-PF57 (2:1)

To a solution of **trast-v1a-145** (95 µL, 1.0 mg, 10.6 mg/mL in PBS pH 7.4) was added **NHO2-PF57** (69 µL, 7.49 mg/mL in PBS pH 7.4, 3 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO2-PF57 (2:1)** (Figure 21).

### Example 30. Conjugation of tras-v1a-145 to NHO3-PF57 to obtain conjugate tras-v1a-145-NHO3-PF57 (2:1)

To a solution of **trast-v1a-145** (95 µL, 1.0 mg, 10.6 mg/mL in PBS pH 7.4) was added **NHO3-PF57** (86 µL, 5.96 mg/mL in PBS pH 7.4, 3 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO3-PF57 (2:1)** (Figure 21).

### Example 31. Conjugation of tras-v1a-145 to NHO4-PF57 to obtain conjugate tras-v1a-145-NHO4-PF57 (2:1)

To a solution of **trast-v1a-145** (95 µL, 1.0 mg, 10.6 mg/mL in PBS pH 7.4) was added **NHO4-PF57** (73 µL, 7.06 mg/mL in PBS pH 7.4, 3 equiv. compared to IgG). The reaction was incubated overnight at room temperature. Next, the sample was purified on a Superdex200 Increase 10/300 GL column (Cytiva) on an AKTA Purifier-10 (Cytiva) using PBS pH 7.4 as mobile phase. RP-UPLC analysis of the intact sample confirmed formation of the conjugate **tras-v1a-145-NHO4-PF57 (2:1)** (Figure 21).

### Stability of antibody-cytokine conjugates

### Example 32: Stability study for antibody-cytokine conjugates prepared with IL-15 variants with variable N-terminal sequences

Antibody-cytokine conjugates were diluted to 1 mg/mL in PBS pH 7.4 and incubated at 37°C. At the indicated time points, samples were analyzed by RP-UPLC to determine the stability (Table 2). The variant with N-terminal ST-sequence **tras-v1a-145-NHO1-PF59 (2:1)** showed improved stability compared to the variants with N-terminal SYR- and S-sequence.

**Table 2. Stability of antibody-cytokine conjugates as determined by RP-UPLC. Stability is plotted as percentage of the cytokine-to-antibody ratio compared to T0.**

| | day 0 | day 3 | day 7 | day 10 | day 17 | day 28 |
|---|---|---|---|---|---|---|
| **tras-v1a-145-NHO1-PF57 (2:1)** | 100% | 100% | 93% | 89% | 79% | 70% |
| **tras-v1a-145-NHO1-PF58 (2:1)** | 100% | 94% | 89% | 85% | 77% | 72% |
| **tras-v1a-145-NHO1-PF59 (2:1)** | 100% | 97% | 94% | 92% | 85% | 81% |

### Example 33: Stability study for antibody-cytokine conjugates prepared using different hydroxylamines

Antibody-cytokine conjugates were diluted to 1 mg/mL in PBS pH 7.4 and incubated at 37°C. At the indicated time points, samples were analyzed by RP-UPLC to determine the stability (Table 3). The variants prepared with hydroxylamines **HO2, HO3** and **HO4** showed improved stability over the variant prepared using **HO1.**

**Table 3. Stability of antibody-cytokine conjugates as determined by RP-UPLC. Stability is plotted as percentage of the cytokine-to-antibody ratio compared to T0.**

| | day 0 | day 10 | day 14 | day 21 | day 28 |
|---|---|---|---|---|---|
| **tras-v1a-145-NHO1-PF57 (2:1)** | 100% | 94% | 89% | 89% | 85% |
| **tras-v1a-145-NHO2-PF57 (2:1)** | 100% | 97% | 93% | 92% | 89% |
| **tras-v1a-145-NHO3-PF57 (2:1)** | 100% | 97% | 93% | 93% | 89% |
| **tras-v1a-145-NHO4-PF57 (2:1)** | 100% | 97% | 93% | 93% | 89% |

### Example 34: Stability study for antibody-cytokine conjugates prepared with IL-15 variants with variable N-terminal IL-15 sequence and using different hydroxylamines

Antibody-cytokine conjugates were diluted to 1 mg/mL in PBS pH 7.4 and incubated at 37°C. At the indicated time points, samples were analyzed by RP-UPLC to determine the stability (Table 4). The variants prepared with hydroxylamines **HO2** and N-terminal showed improved stability over the variant prepared using **HO1.**

**Table 4. Stability of antibody-cytokine conjugates as determined by RP-UPLC. Stability is plotted as percentage of the cytokine-to-antibody ratio compared to T0.**

| | day 1 | day 3 | day 7 | day 10 | day 14 | day 21 | day 28 |
|---|---|---|---|---|---|---|---|
| **mPD-L1-v1a-218-NHO1-PF51 (2:2)** | 98% | 92% | 87% | 81% | 76% | 62% | 55% |
| **mPD-L1-v1A-218-NHO2-PF52 (2:2)** | 99% | 97% | 95% | 94% | 92% | 88% | 85% |

### In vivo evaluation

### Example 35. In vivo efficacy in CT26 syngeneic colon cancer model

Female BALB/c mice (5- to 8-week-old at study initiation, obtained from Vital River Laboratory Animal Technology Co., Ltd., China) were inoculated subcutaneously it the right rear flank region with 5 × 10⁵ CT26 tumor cells in 0.1 ml of PBS for tumor development. When the tumor volume was in the range of 70 to 120 mm³, groups of eight mice were injected *s.c.* with either vehicle (PBS pH 7.4) or the indicated antibody-cytokine conjugate (6 mg/kg). In all cases a dose was administered on day 0 and 7. Tumor volume and body weight was measured 2-3 times per week after randomization. **mPD-L1-v1a-218-NHO2-PF52 (2:2)** showed improved anti-tumor efficacy compared to the variant with lower stability **mPD-L1-v1a-218-NHO1-PF51 (2:2)** (Figure 22).

## Claims

1. A process for preparing an isoxazoline compound by strain-promoted alkyne nitrone cycloaddition (SPANC), comprising reacting a nitrone compound with a (hetero)cycloalkyne compound to obtain the isoxazoline compound, wherein the nitrone compound has structure **(1):** 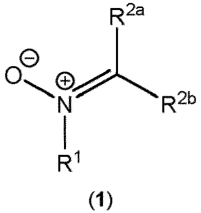 wherein
- R¹ is L¹⁰XR⁴, wherein:
- L¹⁰ is a linker of structure (C(R³)₂)_{z}, wherein z is 2 or 3, and each R³ is individually selected from H and C₁₋₄ alkyl, or two occurrences of R³ may be joined together to form an oxo group, a C₃₋₆ (hetero)cycloalkyl group;
- R⁴ is selected from H and C₁₋₄ alkyl;
- XisS, OorNH,
- R^{2a} is selected from H and C₁₋₆ (cyclo)alkyl;
- R^{2b} is selected from the group consisting of C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups, or wherein R^{2b} is L(D)ᵣ, wherein D is a payload, r is an integer in the range of 1 - 10, and L is a linker covalently connecting D with the nitrone group, or R^{2b} is L⁶B, wherein B is a biomolecule and L⁶ is a linker covalently connecting B with the nitrone group;
- wherein when R^{2a} and R^{2b} are different, the configuration of the C=N double bond in the nitrone group may be ***E*** or ***Z*,**
or a salt thereof.

2. The process according to claim 1, wherein:
- R^{2b} is L(D)ᵣ and the (hetero)cycloalkyne compound is according to structure Q-L⁶-B, or
- R^{2b} is L⁶B and the (hetero)cycloalkyne compound is according to structure Q-L(D)ᵣ, wherein
- B, L⁶, L, D and r are as defined in claim 1;
- Q is a (hetero)cycloalkyne group.

3. The process according to any one of the preceding claims, wherein:
- R^{2a} is H and XR⁴ is OH; and/or
- the (hetero)cycloalkyne compound comprises a click probe Q comprising a (hetero)cycloalkyne moiety, preferably wherein the click probe Q is selected from the group consisting of (Q21) - (Q38), (Q38a) - (Q38d) and (Q44) - (Q56): 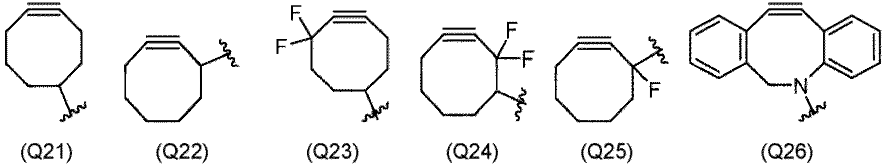 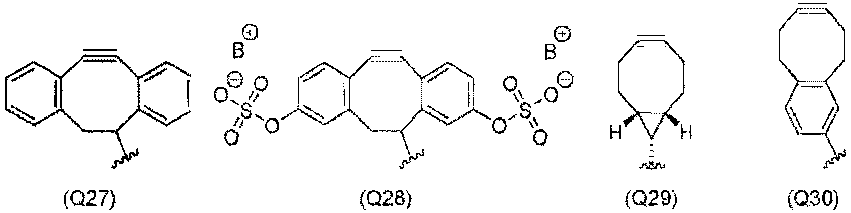 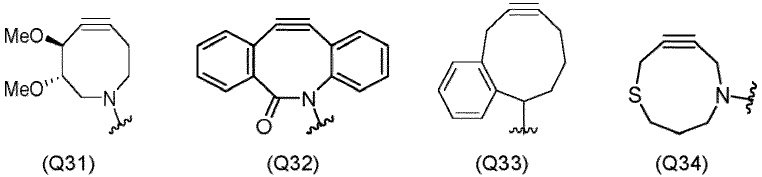 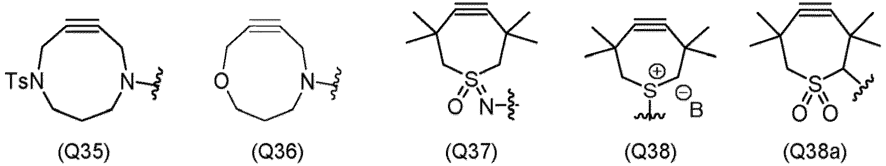 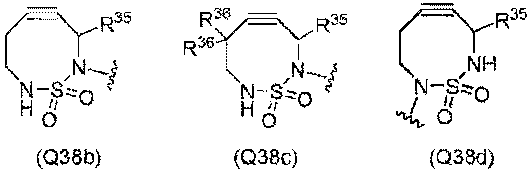 wherein
- B is an anion; and
- R³⁶ is a halogen selected from fluor, chlorine, bromine and iodine;
- R³⁵ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, Si(R¹⁴)₂, S and NR¹⁴ wherein R¹⁴ is independently selected from the group consisting of hydrogen and C₁ - C₄ alkyl groups.

4. A bioconjugate having structure **(3), (4), (5)** or **(6),** or a salt thereof: 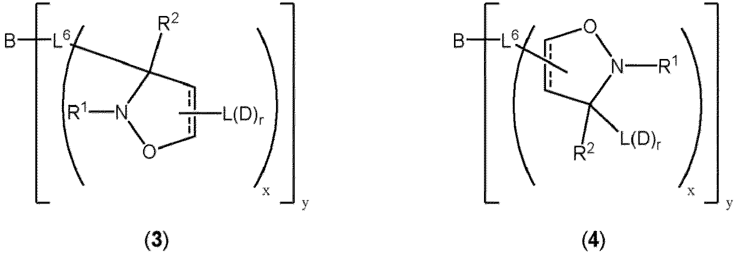 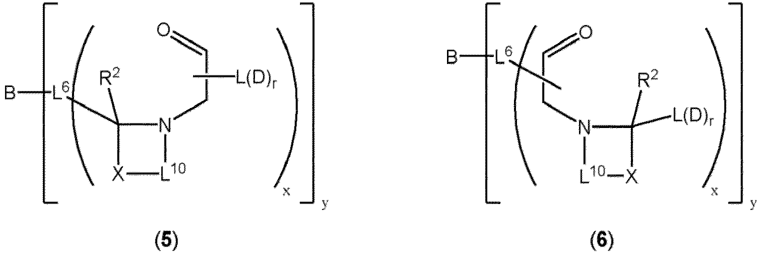 wherein:
- B is a biomolecule;
- D is a payload;
- L⁶ is a linker covalently connecting B with the isoxazoline group;
- L is a linker covalently connecting the isoxazoline group with (D)ᵣ;
- r is an integer in the range of 1 - 10;
- x is an integer in the range of 1 - 4;
- y is an integer in the range of 1 - 10;
- is L¹⁰XR⁴;
- L¹⁰ is a linker of structure (C(R³)₂)_{z}, wherein z is 2 or 3, and each R³ is individually selected from H and C₁₋₄ alkyl, wherein two occurrences of R³ may be joined together to form a C₃₋₆ (hetero)cycloalkyl group;
- R⁴ is selected from H and C₁₋₄ alkyl;
- XisS, OorNH,
- R² is selected from H and C₁₋₄ alkyl.

5. The bioconjugate according to claim 4, wherein one or more, preferably all, of the following applies:
- R² is H;
- z is 2;
- B is a protein, preferably an antibody;
- r is an integer in the range of 1 - 4, preferably r = 1 or 2;
- x = 1 or 2, preferably x = 1;
- y is an integer in the range of 1 - 4, preferably y = 2 or 4;
- D is a payload selected from an active substance.

6. The bioconjugate according to claim 4 or 5, having structure **(3a), (4a), (5a)** or **(6a),** or a salt thereof: 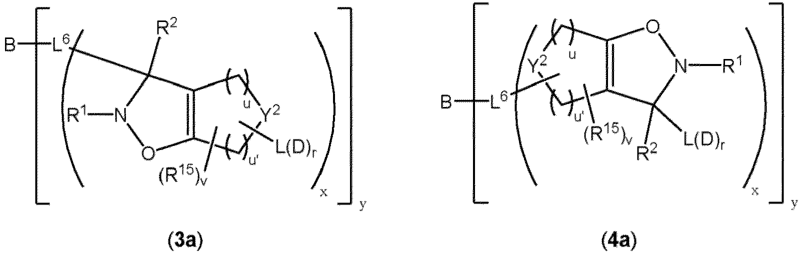 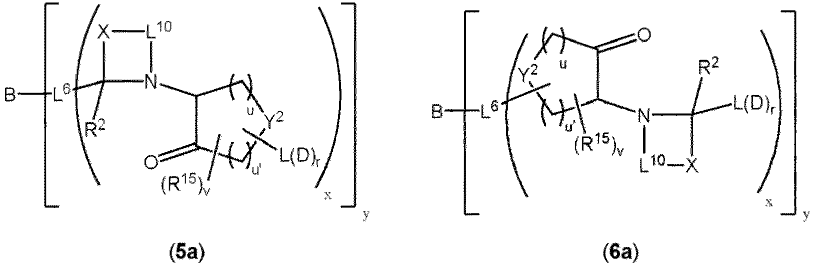 wherein:
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y² is C(R³¹)₂, O, S, S⁽⁺⁾R³¹, S(O)R³¹, S(O)=NR³¹ or NR³¹, wherein S⁽⁺⁾ is a cationic sulfur atom counterbalanced by B⁽⁻⁾, wherein B⁽⁻⁾ is an anion, and wherein each R³¹ individually is R¹⁵ or a connection with B or D, connected via L;
- u is 0, 1, 2, 3, 4 or 5;
- u' is 0, 1, 2, 3, 4 or 5, wherein u + u' = 4, 5, 6, 7 or 8;
- v is an integer in the range 8 - 16;
preferably wherein the bioconjugate has a structure selected from **(3b)** - **(6b), (3c)** - **(6c)** and **(3d)** - **(6d),** or a salt thereof: 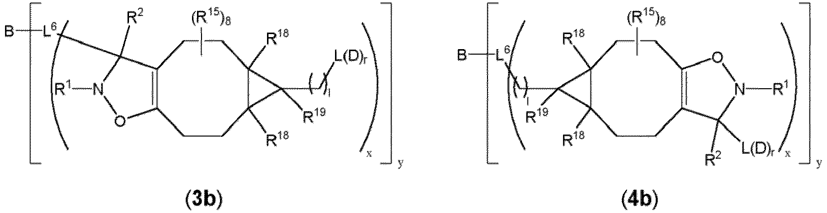 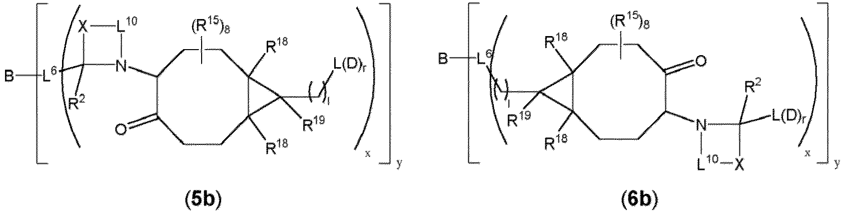 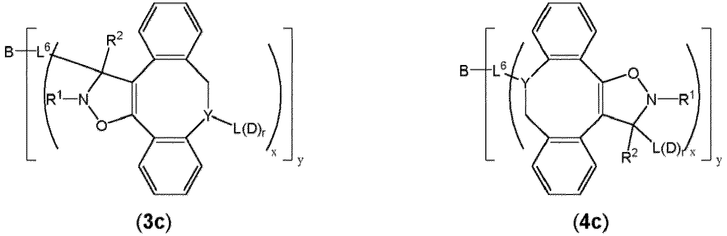 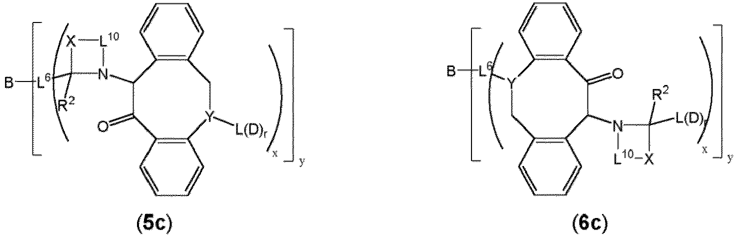 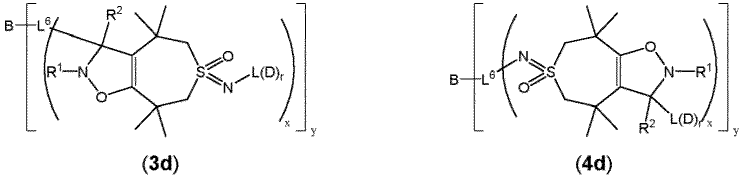 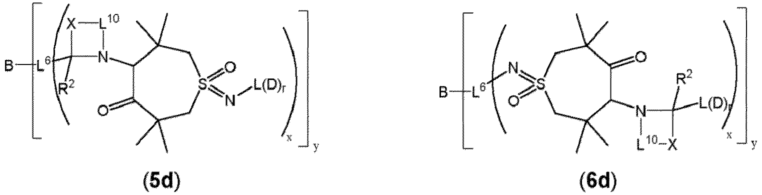 wherein:
- R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of B or D connected via a spacer moiety; and
- I is an integer in the range 0 to 10;
- Y isN or CR¹⁵;
or the regioismers of structures **(3c)** - **(6c)** wherein the positions of Y-L(D)ᵣ and CH₂ within the 8-membered ring are reversed.

7. A nitrone compound of structure **(1):** 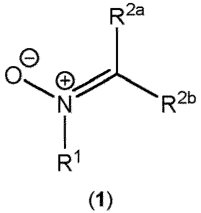 wherein
- is L¹⁰XR⁴, wherein:
- L¹⁰ is a linker of structure (C(R³)₂)_{z}, wherein z is 2 or 3, and each R³ is individually selected from H and C₁₋₄ alkyl, or two occurrences of R³ may be joined together to form an oxo group, a C₃₋₆ (hetero)cycloalkyl group;
- R⁴ is selected from H and C₁₋₄ alkyl;
- XisS, OorNH,
- R^{2a} is selected from H and C₁₋₆ (cyclo)alkyl;
- R^{2b} is selected from the group consisting of C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups, or wherein R^{2b} is L(D)ᵣ, wherein D is a payload, r is an integer in the range of 1 - 10, and L is a linker covalently connecting D with the nitrone group, or R^{2b} is L⁶B, wherein B is a biomolecule and L⁶ is a linker covalently connecting B with the nitrone group;
- wherein when R^{2a} and R^{2b} are different, the configuration of the C=N double bond in the nitrone group may be *E* or *Z,*
or a salt thereof.

8. The nitrone compound according to claim 7, wherein one or more, preferably all, of the following apply:
- R^{2a} is H;
- XR⁴ is OH;
- R^{2b} is L(D)ᵣ or L⁶B.

9. Use of the nitrone compound according to claim 7 or 8 for preparing an isoxazoline compound by strain-promoted alkyne or alkene nitrone cycloaddition (SPANC), preferably wherein R^{2b} is L(D)ᵣ or L⁶B and the isoxazoline compound is a bioconjugate.

10. The use according to claim 9, which is for improving the stability of the bioconjugate and/or for reducing non-induced release of a payload D from the bioconjugate.

11. The bioconjugate according to any one of claims 4 - 6 for use in medical treatment.

12. The bioconjugate according to any one of claims 4 - 6 for use in the treatment of cancer.

13. A pharmaceutical composition comprising the bioconjugate according to any one of claims 4 - 6 and a pharmaceutically acceptable carrier.

14. A method for targeting a tumour cell expressing a specific extracellular receptor, comprising contacting the bioconjugate according to any one of claims 4 - 6 with cells that may possibly express the extracellular receptor, wherein the antibody specifically targets the extracellular receptor, preferably wherein the extracellular receptor is selected from the group consisting of 5T4, ADAM-9, AMHRII, ASCT2, ASLG659, ASPHD1, av-integrin, Axl, B7-H3, B7-H4, BAFF-R, BCMA, BMPR1B, brevican, c-KIT, c-Met, C4.4a, CA-IX, cadherin-6, CanAg, CD123, CD13, CD133, CD138/syndecan-1, CD166, CD19, CD20, CD203c, CD205, CD21, CD22, CD228, CD25, CD30, CD324, CD33, CD37, CD38, CD45, CD46, CD48a, CD56, CD70, CD71, CD72, CD74, CD79a, CD79b, CEACAM5, claudin-18.2, claudin-6, CLEC12A, CLL-1, Cripto, CRIPTO, CS1, CXCR5, DLK-1, DLL3, DPEP3, E16, EGFR, ENPP3, EpCAM, EphA2, EphB2R, ETBR, FAP, FcRH1, FcRH2, FcRH5, FGFR2, fibronectin, FLT3, folate receptor alpha (FOL-R1), Gal-3BP, GD3, GDNF-Ra1, GEDA, GFRA1, Globo H, gpNMB, GPR172A, GPR19, GPR54, guanyl cyclase C, HER2, HER3, HLA-DOB, IGF-1R, IL13R, IL20Rα, Lewis Y, LGR5, LIV-1, LRRC15, LY64, Ly6E, Ly6G6D, LY6K, MDP, MFI2, MICA/B, MOSPD2, MPF, MSG783, MUC1, MUC16, NaPi2b, NCA, nectin-4, Notch3, P-cadherin, P2X5, PD-L1, PMEL17, PRLR, PSCA, PSMA, PTK7, RET, RNF43, RON, ROR1, ROR2, SLITRK6, SSTR2, STEAP1, STEAP2, TAG72, TENB2, TF, TIM-1, TM4SF, TMEFF, TMEM118, TMEM46, transferrin, TROP-2, TrpM4, TWEAKR, receptor tyrosine kinases (RTK), tenascin, or the biomolecule is an antibody is specific to an extracellular protein resulting from a viral infection and/or for a tumour-associated carbohydrate antigen (TACA).

15. A method for the treatment of cancer, comprising administering to a subject in need thereof the bioconjugate according to any one of claims 4-6, wherein the cancer cells specifically express an extracellular receptor, preferably wherein the extracellular receptor is selected from the group consisting of 5T4, ADAM-9, AMHRII, ASCT2, ASLG659, ASPHD1, av-integrin, Axl, B7-H3, B7-H4, BAFF-R, BCMA, BMPR1B, brevican, c-KIT, c-Met, C4.4a, CA-IX, cadherin-6, CanAg, CD123, CD13, CD133, CD138/syndecan-1, CD166, CD19, CD20, CD203c, CD205, CD21, CD22, CD228, CD25, CD30, CD324, CD33, CD37, CD38, CD45, CD46, CD48a, CD56, CD70, CD71, CD72, CD74, CD79a, CD79b, CEACAM5, claudin-18.2, claudin-6, CLEC12A, CLL-1, Cripto, CRIPTO, CS1, CXCR5, DLK-1, DLL3, DPEP3, E16, EGFR, ENPP3, EpCAM, EphA2, EphB2R, ETBR, FAP, FcRH1, FcRH2, FcRH5, FGFR2, fibronectin, FLT3, folate receptor alpha (FOL-R1), Gal-3BP, GD3, GDNF-Ra1, GEDA, GFRA1, Globo H, gpNMB, GPR172A, GPR19, GPR54, guanyl cyclase C, HER2, HER3, HLA-DOB, IGF-1R, IL13R, IL20Rα, Lewis Y, LGR5, LIV-1, LRRC15, LY64, Ly6E, Ly6G6D, LY6K, MDP, MFI2, MICA/B, MOSPD2, MPF, MSG783, MUC1, MUC16, NaPi2b, NCA, nectin-4, Notch3, P-cadherin, P2X5, PD-L1, PMEL17, PRLR, PSCA, PSMA, PTK7, RET, RNF43, RON, ROR1, ROR2, SLITRK6, SSTR2, STEAP1, STEAP2, TAG72, TENB2, TF, TIM-1, TM4SF, TMEFF, TMEM118, TMEM46, transferrin, TROP-2, TrpM4, TWEAKR, receptor tyrosine kinases (RTK), tenascin, or the biomolecule is an antibody is specific to an extracellular protein resulting from a viral infection and/or for a tumour-associated carbohydrate antigen (TACA).
